# EUROPEAN PATENT APPLICATION

(11) **EP 4 289 427 A1**
(43) Date of publication of application: **13.12.2023**
(21) Application number: 22305848.8
(22) Date of filing: 10.06.2022
(51) Int. Cl.: A61K 31/4353, A61K 31/4706, A61P 35/00, A61P 35/02, C07D 491/04

(54) **DIHYDRO[1,8]NAPHTHYRIDIN-7-ONE AND PYRIDO[3,2-B][1,4]OXAZIN-3-ONE FOR USE IN TREATING CANCER, AND METASTASES IN PARTICULAR.**

(71) Applicant: Anagenesis Biotechnologies, 67400 Illkirch-Graffenstaden (FR)
(72) Inventor: MORICE, Christophe, Widensolen (FR); RIGUET, Eric, Bruguières (FR)
(74) Representative: Cabinet Becker et Associés

(57) **Abstract**

The invention provides compounds for treating, ameliorating, delaying, curing and/or preventing cancer. In particular, the invention relates to compounds of general formula (I) or a pharmaceutically acceptable salt thereof, and their use for treating cancer and/or for reducing or preventing the appearance of metastases in a patient afflicted with a cancer. The invention also relates to new compounds of general formula (II) or a pharmaceutical acceptable salt thereof, and their use as a drug, in particular for treating cancer and/or for reducing or preventing the appearance of metastases in a patient afflicted with a cancer.

## Description

### FIELD

The invention relates to the field of compounds for treating cancer and/or for reducing or preventing the appearance of metastases in a patient afflicted with a cancer. The invention in particular relates to the use of compounds with improved characteristics enhancing clinical applicability as further defined herein.

### BACKGROUND OF THE INVENTION

Cancer and inflammatory diseases are among the most frequent illnesses nowadays. In particular, cancer is one of the most important causes of death in industrialized countries, and the number of people diagnosed with cancer continues to grow at the current time.

Several treatment modes have been developed so far, however chemotherapy remains the only one that can be used against circulating tumors, such as lymphomas and leukemias, and metastases.

It is shown in the art that cancer in humans can be linked to the activity of non-viral, endogenous oncogenes, and that a substantial portion of these oncogenes code for protein tyrosine kinases.

Tyrosine kinases are a class of enzymes that catalyze the transfer of the terminal phosphate of adenosine triphosphate (ATP) to tyrosine residues in protein substrates. Tyrosine kinases are believed, by way of substrate phosphorylation, to play critical roles in signal transduction for a number of cell functions. In fact, tyrosine kinases have been shown to be important contributing factors in cell proliferation, carcinogenesis, and cell differentiation. Tyrosine kinases can be categorized as either receptor tyrosine kinases (RTK) or non-receptor tyrosine kinases (nRTK).

Receptor tyrosine kinases (RTK) such as EGFR, HER2, KIT and KDR are for example well characterized proteins with a clearly established function in cancer.

Unlike the receptor tyrosine kinases (RTKs), the second subgroup of tyrosine kinases, the non-receptor tyrosine kinases are cytosolic enzymes. Non-receptor tyrosine kinases regulate cell growth, proliferation, differentiation, adhesion, migration and apoptosis, and they are critical components in the regulation of the immune system.

Src family kinase (SFK) is a family of non-receptor tyrosine kinases, including c-Src, LYN, FYN, LCK, HCK, FGR, BLK, YES and YRK. SFK is linked to multiple *in vivo* signal transduction pathways and is activated by growth factors, cytokines and immune cell receptors, G-protein coupled receptors, and integrins and other cell adhesion molecules, and then activating the corresponding signal transduction pathway, causing a variety of physiological effects of the cell. The activity of SFK mainly includes the regulation of cell morphology, cell motility, cell proliferation and survival. Abnormal activation and expression of these kinases lead to the development and progression of a wide range of diseases, such as a large number of solid tumors, a variety of hematological malignancies and some neuronal pathologies.

Several tyrosine kinase inhibitors have been identified to possess effective antitumor activity and have been approved or are in clinical trials. These include gefitinib (Iressa^{®}), sunitinib (Sutent^{®}; SU11248), erlotinib (Tarceva^{®}; OSI-1774), lapatinib (GW-572016), canertinib (CI 1033), semaxinib (SU5416), vatalanib (PTK787/ZK222584), sorafenib (BAY 43-9006), imatinib mesylate (Gleevec^{®}; STI571), and leflunomide (SU101).

Despite the variety of available therapies for treating cancer and advancements within them, an important need exists, for novel and effective treatment for cancer. In particular, there is a need for novel effective tyrosine kinase inhibitors useful for treating cancer.

### SUMMARY

The present invention provides new compounds for treating cancer and/or for reducing or preventing the appearance of metastases in a patient afflicted with a cancer.

The present invention relates to these compounds, a pharmaceutical composition comprising such a compound, and their uses as a drug, in particular for treating, ameliorating, delaying, curing and/or preventing cancer. The present invention further relates to the use of a compound according to the present invention for the manufacture of a medicament, in particular for treating, ameliorating, delaying, curing and/or preventing cancer. In addition, it relates to a method of treating, ameliorating, delaying, curing and/or preventing cancer in a subject in need thereof, comprising administering a therapeutic amount of a compound according to the present invention. More particularly, the cancer is selected in the group consisting of angiogenic and/or metastatic cancers such as bladder, brain, breast, colon, kidney, liver, lung, melanoma, ovary, muscle (Granular Cell Tumor, Leiomyosarcoma, Myosarcoma such as Rhabdomyosarcoma (RMS), Smooth Muscle Tumor and Alveolar Soft-Part Sarcoma), pancreas, prostate, rectal, sarcoma, stomach, thyroid, and uterus cancers.

In a first aspect, the invention relates to a compound of general formula (I), or a pharmaceutically acceptable salt and/or solvate thereof, for use for treating cancer and/or for reducing or preventing the appearance of metastases in a patient afflicted with a cancer, wherein formula (I) is 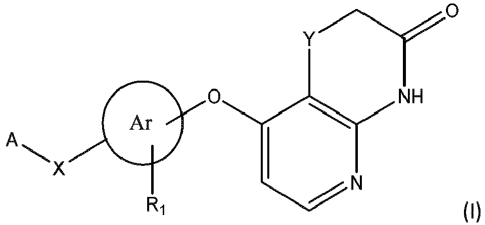 wherein,
X is
   1) -X₁C(O)X₂-; X₁ and X₂ being, independently of each other, absent, -CH₂-or-NH-; and Ar is a phenyl, naphthyl, or heteroaryl comprising 5 or 6 cyclic atoms;
      or
   2) -X₁C(O)-; X₁ being absent, -CH₂- or -NH-; and Ar is: 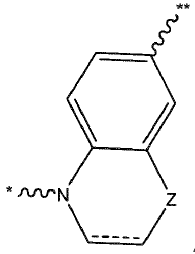 , with Z being -O-, -S-, -CH₂-, -NR_{z}- or absent; R_{z} being H , (C₁-C₆)alkyl group, -CO-(C₁-C₆)alkyl group, -SO-(C₁-C₆)alkyl group or - SO₂-(C₁-C₆)alkyl group; and with 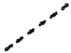 indicating an optional double bond; or
   3) -NH-; and Ar is 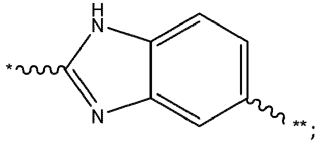 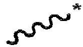 indicating the bound between Ar and X; 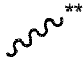 indicating the bound between Ar and the oxygen atom;
      and wherein,
      R₁ is selected in the group consisting of a (C₁-C₆)alkyl group, (C₁-C₆)heteroalkyl group, (C₁-C₆)haloalkyl group, or a halogen;
      Y is an oxygen atom or -CH₂-; and
      A is a group selected from (C₁-C₆)alkyl, (C₃-C₁₀)cycloalkyl, aryl or heterocycle, said group being optionally substituted with A₁, A₂, A₃ and/or A₄, wherein
         A₁ is -CF₃; halo; -NHC(O)CH₂NR_{A}R_{B}; -NHSO₂R_{c}; -NHR_{E}; -OR_{F}; -C(O)R_{G}; -C(O)NHR_{H}; or A₁ is a group G1 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
            said group G1 being optionally substituted with at least one, such as one or two, substituent S1 independently selected from a halo; -CN; -NR_{A}R_{B}; -R_{D}NR_{A}R_{B}; or a group G2 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
               said group G2 being optionally substituted with at least one, such as one or two, substituent S2 independently selected from a halo, -CN, -NR_{A}R_{B}, -R_{D}NR_{A}R_{B}, (C₁-C₆)alkyl group, (C₃-C₁₀)cycloalkyl group, aryl group, heterocycle group, (C₁-C₆)alkylaryl, (C₁-C₆)alkyl-heterocycle, and (C₁-C₆)alkoxyl group;
            R_{A} and R_{B} being independently an hydrogen atom or a (C₁-C₆)alkyl group;
            R_{C}, R_{D}, R_{E}, R_{F}, R_{G} and R_{H} being independently a group G3 selected from a phenyl, (C₁-C₆)alkyl, heterocycle, (C₁-C₆)alkyl-phenyl, and (C₁-C₆)alkyl-heterocycle, said group G3 being optionally substituted with at least one, such as one or two, substituent S3 independently selected from -CN, halo, -CF₃ and a (C₁-C₆)alkyl;
            and
         A₂, A₃ and A₄ are independently selected from -CF₃, halo, (C₁-C₆)alkyl, and (C₁-C₃)alkoxyl.

In a second aspect, the invention relates to a compound of general formula (II), or a pharmaceutically acceptable salt and/or solvate thereof: 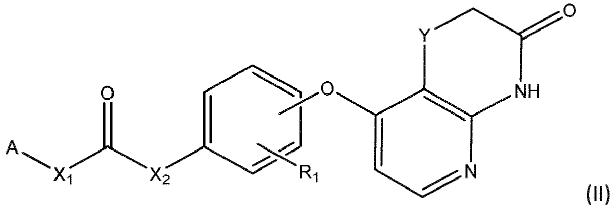 wherein,
X₁ and X₂ are, independently of each other, absent, -CH₂- or -NH-;
R₁ is selected in the group consisting of a (C₁-C₆)alkyl group, (C₁-C₆)heteroalkyl group, (C₁-C₆)haloalkyl group, or a halogen;
Y is an oxygen atom or -CH₂-; and
A is an aryl, preferably a phenyl, or a (C₃-C₁₀)cycloalkyl, preferably a cyclopropyl, and is optionally substituted with A₁, A₂, A₃ and/or A₄, wherein
   A₁ is -CF₃; halo; -NHC(O)CH₂NR_{A}R_{B}; -NHSO₂R_{C}; -NHR_{E}; -OR_{F}; -C(O)R_{G}; -C(O)NHR_{H}; or A₁ is a group G1 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
      said group G1 being optionally substituted with at least one, such as one or two, substituent S1 independently selected from a halo; -CN; -NR_{A}R_{B}; -R_{D}NR_{A}R_{B}; or a group G2 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
         said group G2 being optionally substituted with at least one, such as one or two, substituent S2 independently selected from a halo, -CN, -NR_{A}R_{B}, -R_{D}NR_{A}R_{B}, (C₁-C₆)alkyl group, (C₃-C₁₀)cycloalkyl group, aryl group, heterocycle group, (C₁-C₆)alkylaryl, (C₁-C₆)alkyl-heterocycle, and (C₁-C₆)alkoxyl group;
      R_{A} and R_{B} being independently an hydrogen atom or a (C₁-C₆)alkyl group;
      R_{C}, R_{D}, R_{E}, R_{F}, R_{G} and R_{H} being independently a group G3 selected from a phenyl, (C₁-C₆)alkyl, heterocycle, (C₁-C₆)alkyl-phenyl, and (C₁-C₆)alkyl-heterocycle, said group G3 being optionally substituted with at least one, such as one or two, substituent S3 independently selected from -CN, halo, -CF₃ and a (C₁-C₆)alkyl;
      and
   A₂, A₃ and A₄ are independently selected from -CF₃, halo, (C₁-C₆)alkyl, and (C₁-C₃)alkoxyl.

The invention further relates to a compound of general formula (II) according to the invention, or a pharmaceutically acceptable salt and/or solvate thereof, for use as a drug.

The invention further relates to a pharmaceutical composition comprising a compound of general formula (II) according to the invention, or a pharmaceutically acceptable salt and/or solvate thereof, and at least one pharmaceutically acceptable excipient.

The invention further relates to a compound of general formula (II) according to the invention or a pharmaceutical composition as defined above, for use for treating cancer and/or for reducing or preventing the appearance of metastases in a patient afflicted with a cancer, preferably said cancer being angiogenic and/or metastatic such as bladder, brain, breast, colon, kidney, liver, lung, melanoma, ovary, muscle, pancreas, prostate, rectal, sarcoma, stomach, thyroid, or uterus cancers.

### BRIEF DESCRIPTION OF THE FIGURES

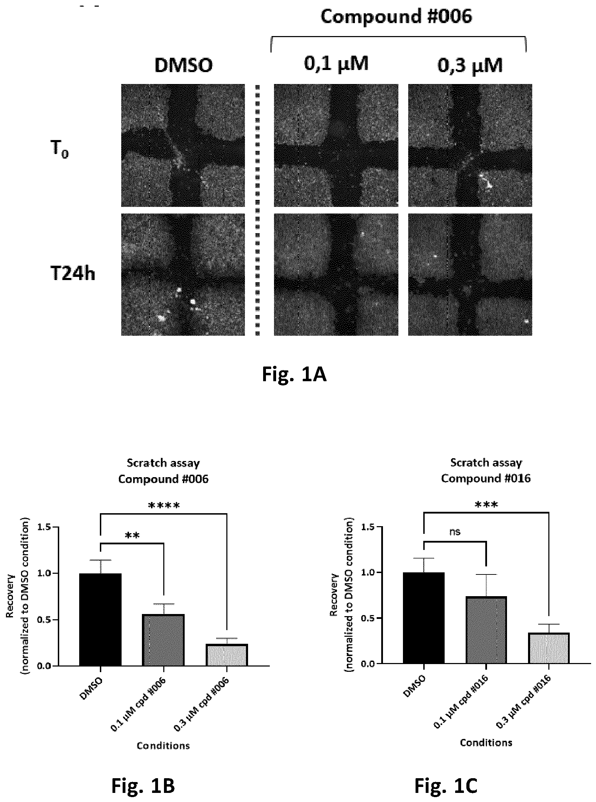

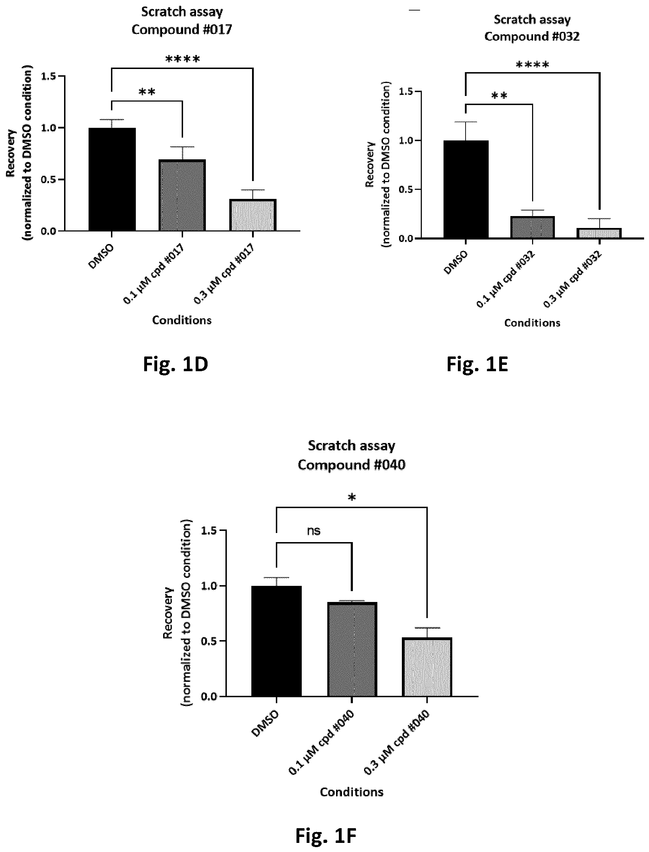

**Figure 1** shows results obtained in the scratch (wound healing) assay on MDA-MB-231 cells. (A) Pictures of the cellular layer at time zero and twenty-four hours later, following treatment with DMSO vehicle or 0.3 µM or 0.1 µM of compound #006. Quantifications of migration inhibition in the scratch assay for compound #006 (B), compound #016 (C), compound #017 (D), compound #032 (E) and compound #040 (F). Compiled data are expressed as mean ± standard error. For statistical comparison, the Kruskal-Wallis test was used. The level of significance is indicated as follows: ^{∗} : p<0.05, ^{∗∗} : p<0.01, ^{∗∗∗} : p<0.001, ^{∗∗∗∗}: p<0.0001.

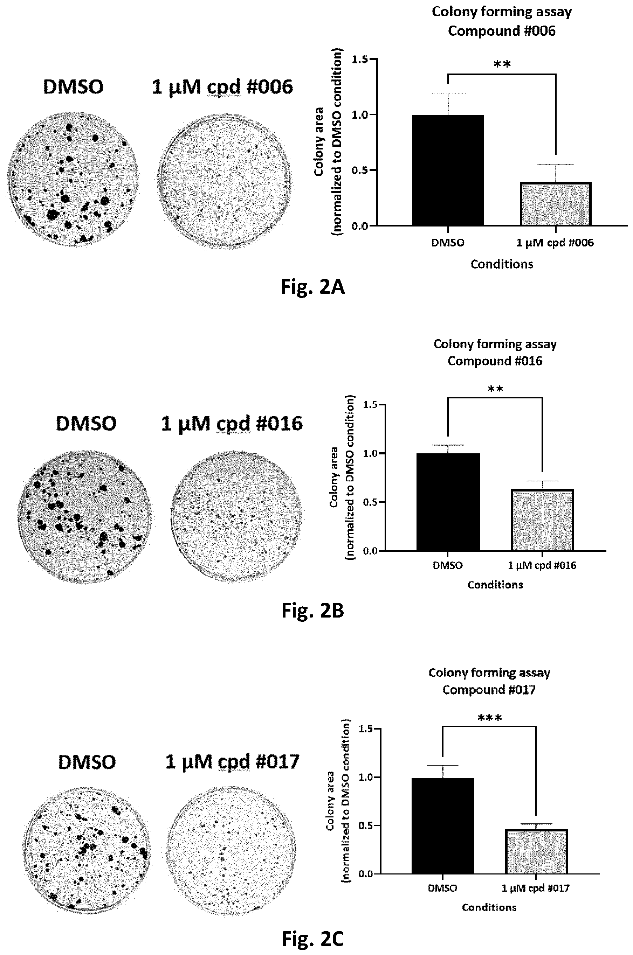

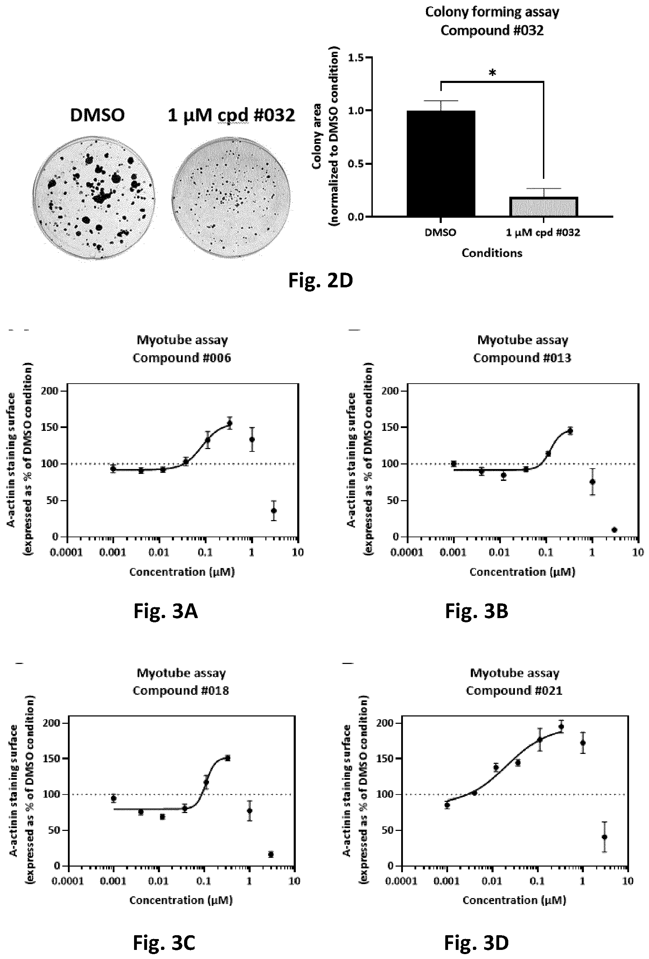

**Figure 2** shows the inhibition of clonogenicity of 4T1 cells by compound of the present invention. Pictures of the wells and corresponding colony area quantification for compound #006 (A), compound #016 (B), compound #017 (C) and compound #032 (D). Compiled data are expressed as mean ± standard error. For statistical comparison, the Mann-Whitney test was used. The level of significance is indicated as follows: ^{∗} : p<0.05, ^{∗∗} : p<0.01, ^{∗∗∗} : p<0.001.

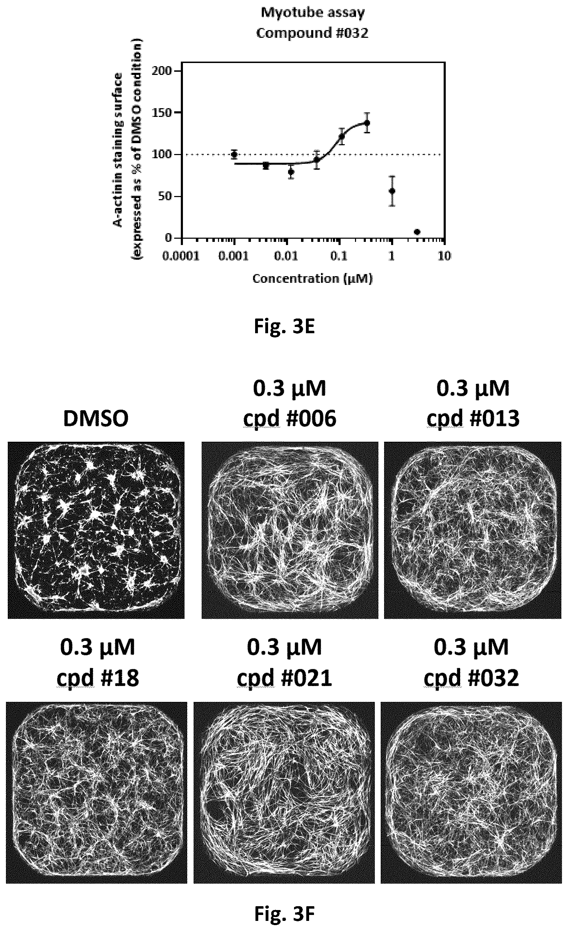

**Figure 3** shows the induction of differentiation of RD cells by compounds of the present invention. Quantifications of alpha-actinin staining surface (i.e. myotubes) for compounds #006 (A), compound #013 (B), compound #018 (C), compound #021 (D) and compound #032 (E). Each point of the dose-response curves represents the mean ± standard error of technical duplicates. Representative pictures of DMSO-treated wells and of the concentration of each compound inducing the maximum effect are shown in (F).

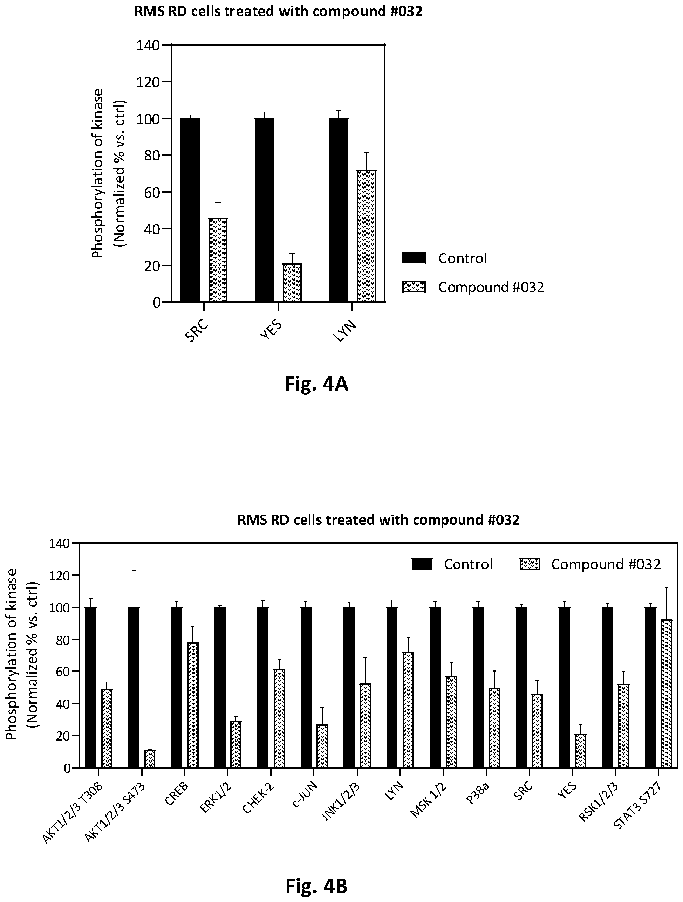

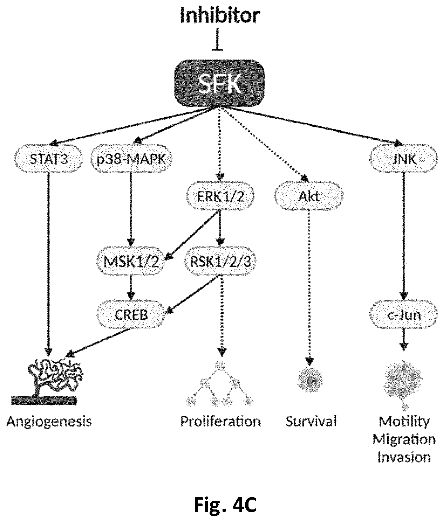

**Figure 4** shows the inhibition of the Src kinase family (SFK) by compound #032 in the rhabdomyosarcoma RD cells (n = 2 independent experiments) and its subsequent inhibition of the SFK pathways (A), as illustrated by the decrease of phosphorylation of their key downstream proteins (B). The inhibition of SFK pathways decreases the cancer-associated mechanisms (C).

### DETAILED DESCRIPTION OF THE INVENTION

### Definitions

According to the present invention, the terms below have the following meanings:
The term "Cₓ-C_{y}" in which x and y are integers, as used in the present disclosure, means that the corresponding hydrocarbon chain comprises from x to y carbon atoms. If, for example, the term C₁-C₆ is used, it means that the corresponding hydrocarbon chain may comprise from 1 to 6 carbon atoms, especially 1, 2, 3, 4, 5, or 6 carbon atoms. If, for example, the term C₁-C₄ is used, it means that the corresponding hydrocarbon chain may comprise from 1 to 4 carbon atoms, especially 1, 2, 3 or 4 carbon atoms. If, for example, the term C₁-C₃ is used, it means that the corresponding hydrocarbon chain may comprise from 1 to 3 carbon atoms, especially 1, 2, or 3 carbon atoms. C₀-C₃ means that the corresponding hydrocarbon chain may comprise from 0 to 3 carbon atoms, especially 0, 1, 2 or 3 carbon atoms. In particular, in the context of C₀, the hydrocarbon chain is absent.

The term "alkyl" refers to a saturated, linear or branched aliphatic group. The term "(C₁-C₆)alkyl" more specifically means methyl, ethyl, propyl, isopropyl, butyl (n-butyl, i-butyl, sec-butyl and tert-butyl), pentyl, or hexyl. The term "(C₁-C₄)alkyl" more specifically means methyl, ethyl, propyl, isopropyl, or butyl (n-butyl, i-butyl, sec-butyl and tert-butyl). The term "(C₁-C₃)alkyl" more specifically means methyl, ethyl, isopropyl or propyl. In a particular aspect, the "alkyl" is a methyl. By "Me", it refers to a methyl, and by "Et", it refers to an ethyl.

The term "alkenyl" refers to an unsaturated, linear or branched aliphatic group, having at least one carbon-carbon double bond. The term "(C₂-C₆)alkenyl" refers to an alkenyl having 2 to 6 carbon atoms. The term alkenyl (or (C₂-C₆)alkenyl) includes for instance ethenyl, propenyl, butenyl, pentenyl, or hexenyl.

The term "alkynyl" refers to an unsaturated, linear or branched aliphatic group, having at least one carbon-carbon triple bond. The term "(C₂-C₆)alkynyl" refers to an alkynyl having 2 to 6 carbon atoms. The term alkynyl (or (C₂-C₆)alkynyl) includes for instance ethynyl, propynyl, butynyl, pentynyl, or hexynyl.

The term "haloalkyl" refers to an alkyl as defined herein substituted with at least one halogen, for example one, two or three halogens. A " (C₁-C₆)haloalkyl" refers to a haloalkyl having 1 to 6 carbon atoms and at least one halogen such as one fluorine, chlorine, bromine, or iodine atom. The haloalkyl includes a fluoromethyl (-CH₂F), a difluoromethyl (-CHF₂), or a trifluoromethyl (-CF₃).

The term « heteroalkyl » refers to an alkyl as defined herein, wherein the aliphatic carbon chain comprises at one or both of its two ends (in particular, the end attached to the remainder of the molecule), and/or is interrupted by at least one heteroatom such as O, N or S. Examples of heteroalkyl are in particular alkoxy (-O-alkyl), alkylthio (-S-alkyl), and alkylamino (-NH(alkyl) or -N(alkyl)₂). A « (C₁-C₆)heteroalkyl » refers to a heteroalkyl having 1 to 6 carbon atoms and at least one heteroatom such as O, N or S. Examples of heteroalkyl (or (C₁-C₆)heteroalkyl) include, but are not limited to, methoxy, ethoxy, propyloxy, isopropyloxy, butyloxy, isobutyloxy, tert-butyloxy, pentyloxy, hexyloxy, methylthio, ethylthio, propylthio, isopropylthio, butylthio, isobutylthio, tert-butylthio, pentylthio, hexylthio, methylamino, ethylamino, propylamino, isopropylamino, butylamino, isobutylamino, tert-butylamino, pentylamino, or hexylamino.

The term "alkoxy" or "alkyloxy" corresponds to the alkyl group as above defined bonded to the molecule by an -O- (ether) bond. (C₁-C₆)alkoxy includes methoxy, ethoxy, propyloxy, isopropyloxy, butyloxy, pentyloxy, or hexyloxy. (C₁-C₄)alkoxy includes methoxy, ethoxy, propyloxy, isopropyloxy or butyloxy. (C₁-C₃)alkoxy includes methoxy, ethoxy, propyloxy or isopropyloxy. In a particular aspect, the "alkoxy" or "alkyloxy" is a methoxy.

The term "cycloalkyl" corresponds to a saturated or unsaturated mono-, bi- or tri-cyclic alkyl group comprising between 3 and 20 cyclic atoms of carbons. It also includes fused, bridged, or spiro-connected cycloalkyl groups. The term "cycloalkyl" includes for instance cyclopropyl, cyclobutyl, cyclopentyl, and cyclohexyl. The term "spirocycloalkyl" includes for instance a spirocyclopentyl. In a particular aspect, the term "cycloalkyl" corresponds to a saturated monocycloalkyl group comprising between 3 and 10 atoms of carbons. In a particular aspect, the cycloalkyl group is cyclopropyl or cyclohexyl.

The term "heterocycloalkyl" corresponds to a saturated or unsaturated cycloalkyl group as above defined further comprising at least one heteroatom such as nitrogen, oxygen, or sulfur atom, preferably at least one nitrogen atom or oxygen atom. An heterocycloalkyl is thus a saturated or unsaturated mono-, bi- or tri-cyclic group comprising between 5 and 20 cyclic atoms, preferably 5 and 10 cyclic atoms, and comprising at least one heteroatom such as nitrogen, oxygen or sulfur atom. It also includes fused, bridged, or spiro-connected heterocycloalkyl groups. Representative heterocycloalkyl groups include, but are not limited to aziridinyl, azepanyl, diazepanyl, dioxolanyl, benzo [1,3] dioxolyl, azetidinyl, oxetanyl, pyrazolinyl, pyranyl, thiomorpholinyl, pyrazolidinyl, piperidyl, piperazinyl, 1,4-dioxanyl, imidazolinyl, phthalimidyl, pyrrolinyl, pyrrolidinyl, piperidinyl, imidazolidinyl, morpholinyl, 1,4-dithianyl, pyrrolidinyl, pyrimidinyl, oxozolinyl, oxazolidinyl, isoxazolinyl, isoxazolidinyl, thiooxetanyl, thiopyranyl, thiomorpholinyl, thiazolinyl, thiazolidinyl, isothiazolinyl, isothiazolidinyl, dihydropyranyl, dihydrofuranyl, dihydrothiopyranyl, dihydrothiophenyl, dihydropiperidinyl, tetrahydropiperidinyl, tetrahydrothiopyranyl, tetrahydropyranyl, tetrahydrofuranyl, and tetrahydrothiophenyl. In a particular aspect, the heterocycloalkyl group is for instance azetidinyl, pyrrolidinyl, piperidinyl, piperazinyl or pyrrolidinyl. In another particular aspect, the heterocycloalkyl group is morpholinyl.

"Cycloalkyl" and "heterocycloalkyl" also include cycloalkenyl and heterocycloalkenyl which correspond respectively to a cycloalkyl having at least one carbon-carbon double bond and a heterocycloalkyl having at least one carbon-carbon double bond such as cyclohexenyl, and dihydropyranyl.

The term "aryl" corresponds to a mono- or bi-cyclic aromatic hydrocarbons having from 6 to 12 carbon atoms. For instance, the term "aryl" includes phenyl, biphenyl, naphthyl, or anthracenyl. In a particular aspect, the aryl is a phenyl or a naphthyl, preferably a phenyl.

The term "heteroaryl" as used herein corresponds to an aromatic, mono- or poly-cyclic group comprising between 5 and 14 cyclic atoms, preferably 5 and 10 cyclic atoms, including at least one heteroatom such as nitrogen, oxygen or sulfur atom. As used herein, the term "heteroaryl" further includes the "fused arylheteroaryl", "fused arylheterocycloalkyl" and "fused heteroarylcycloalkyl". The terms "fused arylheteroaryl" may for instance include non-exhaustively quinolinyl, indole, or benzoxazole. The terms "fused arylheterocycloalkyl" and "fused heteroarylcycloalkyl" correspond to a bicyclic group in which an aryl as above defined or a heteroaryl is respectively bounded to the heterocycloalkyl or the cycloalkyl as above defined by at least two carbons. In other terms, the aryl or the heteroaryl shares a carbon bond with the heterocycloalkyl or the cycloalkyl. Examples of such mono- and poly-cyclic heteroaryl group, fused arylheterocycloalkyl and fused arylcycloalkyl may be: pyridinyl, thiazolyl, thiophenyl, furanyl, pyrrolyl, pyrazolyl, imidazolyl, triazolyl, tetrazolyl, thienopyridinyl, dihydropyridinyl, benzofuranyl, thianaphthalenyl, indolyl, indolinyl, quinolinyl, isoquinolinyl, benzimidazolyl, tetrahydroquinolinyl, tetrahydroisoquinolinyl, triazinyl, thianthrenyl, isobenzofuranyl, chromenyl, xanthenyl, phenoxanthinyl, isothiazolyl, isoxazolyl, pyrazinyl, pyridazinyl, indolizinyl, isoindolyl, indazolyl, purinyl, quinolizinyl, phtalazinyl, naphthyridinyl, quinoxalinyl, quinazolinyl, cinnolinyl, pteridinyl, carbazolyl, β-carbolinyl, phenanthridinyl, acridinyl, pyrimidinyl, phenanthrolinyl, phenazinyl, phenothiazinyl, furazanyl, phenoxazinyl, isochromanyl, chromanyl, imidazolidinyl, imidazolinyl, pyrazolidinyl, pyrazolinyl, dihydropyrazolyl-3-one indolinyl, isoindolinyl, oxazolidinyl, benzotriazolyl, benzoisoxazolyl, oxindolyl, benzoxazolyl, benzothiazolyl, benzothiphenyl, benzoxazolinyl, benzoxazinyl, benzothienyl, benzothiazolyl, benzodiazepinyl, benzazepinyl, benzoxazepinyl, isatinyl, dihydropyridyl, pyrimidinyl, s-triazinyl, oxazolyl, or thiofuranyl. In a particular aspect, the heteroaryl is for instance pyridinyl, pyrimidyl, 1,2,4-triazinyl or benzoxazolyl. In a particular aspect, the heteroaryl is for instance tetrazolyl or 1,2,3-trizolyl. In a particular aspect, the heteroaryl is for instance thiophenyl.

The term "fused" when applied to the description of two rings corresponds to a bicyclic group in which the first ring is respectively bounded to the second ring by at least two carbons.

The term "heterocycle group" as used herein refers to both heteroaryl and heterocycloalkyl groups as defined herein.

The term "halogen" corresponds to a fluorine, chlorine, bromine, or iodine atom, preferably a fluorine, a chlorine or a bromide.

The expression "substituted by at least" or "substituted by" means that the group is substituted by one or several substituents of the list. For instance, the expression "a (C₁-C₆)alkyl substituted by at least one halogen" or "a (C₁-C₆)alkyl substituted by a halogen" may include a fluoromethyl (-CH₂F), a difluoromethyl (-CHF₂), or a trifluoromethyl (-CF₃).

The expression "optionally substituted" means that the group is not substituted or substituted by one or several substituents of the list.

By "-CO-" or "-C(O)-", it refers to an oxo group. By "-SO-" or "-S(O)-", it refers to a sulfinyl group. By "-SO₂-" or "-S(O₂)-", it refers to a sulfonyl group.

The "stereoisomers" are isomeric compounds that have the same molecular formula and sequence of bonded atoms, but differ in the 3D-dimensional orientations of their atoms in space. The stereoisomers include enantiomers, diastereoisomers, Cis-trans and E-Z isomers, conformers, and anomers. In a preferred embodiment of the invention, the stereoisomers include diastereoisomers and enantiomers.

The "tautomers" are isomeric compounds that differ only in the position of the protons and the electrons.

The "pharmaceutically salts" include inorganic as well as organic acids salts. Representative examples of suitable inorganic acids include hydrochloric, hydrobromic, hydroiodic, phosphoric, and the like. Representative examples of suitable organic acids include formic, acetic, trichloroacetic, trifluoroacetic, propionic, benzoic, cinnamic, citric, fumaric, maleic, lactic, tartaric, methanesulfonic and the like. Further examples of pharmaceutically inorganic or organic acid addition salts include the pharmaceutically salts listed in J. Pharm. Sci. 1977, 66, 2, and in Handbook of Pharmaceutical Salts: Properties, Selection, and Use edited by P. Heinrich Stahl and Camille G. Wermuth 2002. In a preferred embodiment, the salt is selected from the group consisting of maleate, chlorhydrate, bromhydrate, and methanesulfonate. The "pharmaceutically salts" also include inorganic as well as organic base salts. Representative examples of suitable inorganic bases include sodium or potassium salt, an alkaline earth metal salt, such as a calcium or magnesium salt, or an ammonium salt. Representative examples of suitable salts with an organic base includes for instance a salt with methylamine, dimethylamine, trimethylamine, piperidine, morpholine or tris-(2-hydroxyethyl)amine. In a preferred embodiment, the salt is a chlorhydrate.

As used herein, the terms "treatment", "treat" or "treating" refer to any act intended to ameliorate the health status of patients such as therapy, prevention, prophylaxis and retardation of a disease. In certain aspects, such terms refer to the amelioration or eradication of the disease, or symptoms associated with it. In other aspects, this term refers to minimizing the spread or worsening of the disease, resulting from the administration of one or more therapeutic agents to a subject with such a disease.

As used herein, the terms "subject", "individual" or "patient" are interchangeable and refer to an animal, preferably to a mammal, even more preferably to a human, including adult and child. However, the term "subject" can also refer to non-human animals, in particular mammals such as dogs, cats, horses, cows, pigs, sheep and non-human primates, among others.

The terms "quantity," "amount," and "dose" are used interchangeably herein and may refer to an absolute quantification of a molecule.

As used herein, the terms "active principle", "active ingredient" and "active pharmaceutical ingredient" are equivalent and refers to a component of a pharmaceutical composition having a therapeutic effect.

As used herein, the term "therapeutic effect" refers to an effect induced by an active ingredient, or a pharmaceutical composition according to the invention, capable to prevent or to delay the appearance or development of a disease or disorder, or to cure or to attenuate the effects of a disease or disorder.

As used herein, the terms "effective amount", "active amount" and "therapeutic amount", refer to a quantity of an active ingredient or of a pharmaceutical composition which prevents, removes or reduces the deleterious effects of the disease. It is obvious that the quantity to be administered can be adapted by the man skilled in the art according to the subject to be treated, to the nature of the disease, etc. In particular, doses and regimen of administration may be function of the nature, of the stage and of the severity of the disease to be treated, as well as of the weight, the age and the global health of the subject to be treated, as well as of the judgment of the doctor.

As used herein, the term "pharmaceutically acceptable excipient" refers to any ingredient except active ingredients which are present in a pharmaceutical composition. Its addition may be aimed to confer a particular consistency or other physical or gustative properties to the final product. A pharmaceutically acceptable excipient must be devoid of any interaction, in particular chemical, with the active ingredients.

A compound as defined in this first aspect is referred to in the current application as "a compound according to the invention" or as "a compound of the invention". Said terms are used interchangeably in the context of this application.

In the context of the invention, the ranges of values expressed by "from X to XX" or "between X and XX" comprise the upper and lower limits.

### Compounds for use according to the invention

In a first aspect, the invention provides a compound for use in treating, ameliorating, delaying, curing and/or preventing cancer, or a pharmaceutically acceptable salt thereof, wherein said compound is represented by the general formula (I): 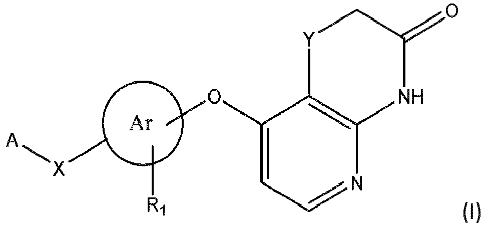 wherein,
X is
   1) -X₁C(O)X₂-; X₁ and X₂ being, independently of each other, absent, -CH₂- or -NH-; and Ar is a phenyl, naphthyl, or a heteroaryl comprising 5 or 6 cyclic atoms;
      or
   2) -X₁C(O)-; X₁ being absent, -CH₂- or -NH-; and Ar is: 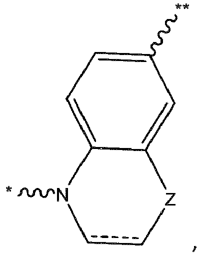 with Z being -O-, -S-, -CH₂-, -NR_{z}- or absent; R_{Z} being H , (C₁-C₆)alkyl group, -CO-(C₁-C₆)alkyl group, -SO-(C₁-C₆)alkyl group or -SO₂-(C₁-C₆)alkyl group; and with 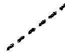 indicating an optional double bond; or
   3) -NH-; and Ar is 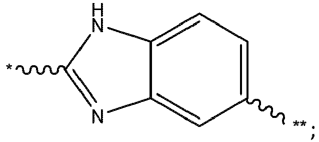 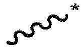 indicating the bound between Ar and X; 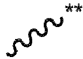 indicating the bound between Ar and the oxygen atom;
   and wherein,
   R₁ is selected in the group consisting of a (C₁-C₆)alkyl group, (C₁-C₆)heteroalkyl group, (C₁-C₆)haloalkyl group, or a halogen such as chlorine and fluorine ;
   Y is an oxygen atom or -CH₂-; and
   A is a group selected from (C₁-C₆)alkyl, (C₃-C₁₀)cycloalkyl, aryl or heterocycle, said group being optionally substituted with A₁, A₂, A₃ and/or A₄, wherein
      A₁ is -CF₃; halo; -NHC(O)CH₂NR_{A}R_{B}; -NHSO₂R_{C}; -NHR_{E}; -OR_{F}; -C(O)R_{G}; -C(O)NHR_{H}; or A₁ is a group G1 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
         said group G1 being optionally substituted with at least one, such as one or two, substituent S1 independently selected from a halo; -CN; -NR_{A}R_{B}; -R_{D}NR_{A}R_{B}; or a group G2 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
            said group G2 being optionally substituted with at least one, such as one or two, substituent S2 independently selected from a halo, -CN, -NR_{A}R_{B}, -R_{D}NR_{A}R_{B}, (C₁-C₆)alkyl group, (C₃-C₁₀)cycloalkyl group, aryl group, heterocycle group, (C₁-C₆)alkylaryl, (C₁-C₆)alkyl-heterocycle, and (C₁-C₆)alkoxyl group;
         R_{A} and R_{B} being independently an hydrogen atom or a (C₁-C₆)alkyl group;
         R_{C}, R_{D}, R_{E}, R_{F}, R_{G} and R_{H} being independently a group G3 selected from a phenyl, (C₁-C₆)alkyl, heterocycle, (C₁-C₆)alkyl-phenyl, and (C₁-C₆)alkyl-heterocycle, said group G3 being optionally substituted with at least one, such as one or two, substituent S3 independently selected from -CN, halo, -CF₃ and a (C₁-C₆)alkyl;
      and
      A₂, A₃ and A₄ are independently selected from -CF₃, halo, (C₁-C₆)alkyl, and (C₁-C₃)alkoxyl.

In this context of the invention, R₁ is a halogen such as chlorine and fluorine, a (C₁-C₆)alkyl group, (C₁-C₆)heteroalkyl group, or (C₁-C₆)haloalkyl group, said groups being optionally substituted. In a specific embodiment, R₁ is a (C₁-C₆)alkyl group, (C₁-C₆)heteroalkyl group comprising an oxygen or a sulfur atom, (C₁-C₆)haloalkyl group such as -CF₃, or a halogen such as chlorine and fluorine. Preferably, R₁ is a (C₁-C₆)alkyl group, -SMe, -SEt, -OMe, -OEt, -CF₃, or a halogen such as chlorine and fluorine. In a more specific embodiment, R₁ is a methyl, halogen such as chlorine and fluorine, -SMe or -CF₃.

In the context of the invention, Y is an oxygen atom or -CH₂-. In a specific embodiment, Y is an oxygen atom. In another specific embodiment, Y is -CH₂-.

In the context of the invention, A is a group selected from (C₁-C₆)alkyl, (C₃-C₁₀)cycloalkyl, aryl or heterocycle, said group being optionally substituted with A₁, A₂, A₃ and/or A₄, wherein
A₁ is -CF₃; halo; -NHC(O)CH₂NR_{A}R_{B}; -NHSO₂R_{c}; -NHR_{E}; -OR_{F}; -C(O)R_{G}; -C(O)NHR_{H}; or A₁ is a group G1 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
   said group G1 being optionally substituted with at least one, such as one or two, substituent S1 independently selected from a halo; -CN; -NR_{A}R_{B}; -R_{D}NR_{A}R_{B}; or a group G2 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
      said group G2 being optionally substituted with at least one, such as one or two, substituent S2 independently selected from a halo, -CN, -NR_{A}R_{B}, -R_{D}NR_{A}R_{B}, (C₁-C₆)alkyl group, (C₃-C₁₀)cycloalkyl group, aryl group, heterocycle group, (C₁-C₆)alkylaryl, (C₁-C₆)alkyl-heterocycle, and (C₁-C₆)alkoxyl group;
   R_{A} and R_{B} being independently an hydrogen atom or a (C₁-C₆)alkyl group;
   R_{C}, R_{D}, R_{E}, R_{F}, R_{G} and R_{H} being independently a group G3 selected from a phenyl, (C₁-C₆)alkyl, heterocycle, (C₁-C₆)alkyl-phenyl, and (C₁-C₆)alkyl-heterocycle, said group G3 being optionally substituted with at least one, such as one or two, substituent S3 independently selected from -CN, halo, -CF₃ and a (C₁-C₆)alkyl ;
and
A₂, A₃ and A₄ are independently selected from -CF₃, halo, (C₁-C₆)alkyl group, and (C₁-C₃)alkoxyl group, said groups being optionally substituted.

In a specific embodiment, in the definition of A, the aryl is a phenyl or a naphthyl, preferably a phenyl.

In a specific embodiment, in the definition of A, the heterocycle group is a heteroaryl or heterocycloalkyl group comprising 5 to 10 cyclic atoms and preferably comprising 1, 2 or 3 heteroatoms, more preferably 1 or 2 heteroatoms. Preferably, the heterocycle group is a heteroaryl or heterocycloalkyl group comprising 5 to 8 cyclic atoms and preferably comprising 1 or 2 heteroatoms. Preferably, the heterocycle group is a heteroaryl or heterocycloalkyl group comprising 5 to 8 cyclic atoms and preferably comprising one or two heteroatoms including at least one nitrogen atom. In particular, in the definition of A, the heterocycle group is preferably selected in the group consisting of furanyl, thiophenyl, pyrrolyl, pyrrolinyl, pyrrolidinyl, pyrazolyl, imidazolyl, isoxazolyl, oxazolyl, tetrahydropyridinyl, pyridinyl, piperidinyl, pyridazinyl, pyrimidinyl, pyrazinyl, piperazinyl, indolinyl, quinolinyl, cinnolinyl, phtalazinyl, isoquinolinyl, quinazolinyl, quinoxalinyl, morpholinyl, hydroxypyridinyl, thiazolyl, and 1,2-dihydropyrazolyl-3-one. Preferably, the heterocycle group is preferably selected in the group consisting of furanyl, thiophenyl, pyrrolyl, pyrrolinyl, pyrrolidinyl, pyrazolyl, imidazolyl, isoxazolyl, oxazolyl, tetrahydropyridinyl, pyridinyl, , pyridazinyl, pyrimidinyl, pyrazinyl, hydroxypyridinyl, thiazolyl, and 1,2-dihydropyrazolyl-3-one.

In a specific embodiment, in the definition of A, the (C₁-C₆)alkyl-aryl is a methyl-aryl or an ethyl-aryl, preferably a methyl-phenyl or an ethyl-phenyl.

In a specific embodiment, in the definition of A, the (C₁-C₆)alkyl-heterocycle is a methyl-heterocycle or an ethyl-heterocycle, preferably a methyl-morpholinyl, methyl-piperazinyl, methyl-pyrrolidinyl, ethyl-morpholinyl, ethyl-piperazinyl, or ethyl-pyrrolidinyl.

In a specific embodiment, A is preferably a group selected from (C₃-C₁₀)cycloalkyl, aryl and heterocycle, said group being optionally substituted with A₁, A₂, A₃ and/or A₄. Preferably, A is a group selected from (C₃-C₆)cycloalkyl, phenyl, naphthyl, and a heterocycle comprising from 5 to 10 cyclic atoms and preferably one or two heteroatoms; said group being optionally substituted with A₁, A₂, A₃ and/or A₄. More preferably A is selected from the group consisting of cyclopropyl, phenyl, furanyl, thiophenyl, pyrrolyl, pyrrolinyl, pyrrolidinyl, pyrazolyl, imidazolyl, isoxazolyl, oxazolyl, tetrahydropyridinyl, pyridinyl, piperidinyl, pyridazinyl, pyrimidinyl, pyrazinyl, piperazinyl, indolinyl, quinolinyl, cinnolinyl, phtalazinyl, isoquinolinyl, quinazolinyl, quinoxalinyl, morpholinyl, hydroxypyridinyl, thiazolyl, and 1,2-dihydropyrazolyl-3-one; said group being optionally substituted with A₁, A₂, A₃ and/or A₄. More preferably, A is selected from the group consisting of cyclopropyl, phenyl, pyrazolyl, imidazolyl, isoxazolyl, oxazolyl, pyridinyl, piperidinyl, pyridazinyl, pyrimidinyl, pyrazinyl, piperazinyl, indolinyl, hydroxypyridinyl, thiazolyl, and 1,2-dihydropyrazolyl-3-one; said group being optionally substituted with A₁, A₂, A₃ and/or A₄. More preferably, A is selected from the group consisting of cyclopropyl, phenyl, pyrazolyl, isoxazolyl, oxazolyl, pyridinyl, indolinyl, hydroxypyridinyl, thiazolyl, and 1,2-dihydropyrazolyl-3-one; said group being optionally substituted with A₁, A₂, A₃ and/or A₄. In a particularly preferred embodiment, A is selected from the group consisting of cyclopropyl, phenyl, and pyrazolyl; said group being optionally substituted with A₁ or A₁ and A₂.

In a specific embodiment, A₄ is absent.

In a specific embodiment, A₃ is absent or is (C₁-C₆)alkyl.

In a specific embodiment A₂ is absent or is selected from -CF₃, Cl, F, (C₁-C₆)alkyl, and (C₁-C₃)alkoxyl. Preferably, A₂ is selected from -CF₃, Cl, F, (C₁-C₆)alkyl, methoxyl and ethoxyl. More preferably, A₂ is selected from -CF₃, Cl, F, methyl, t-butyl, methoxyl and ethoxyl.

In a specific embodiment, A₃ and A₄ are absent, and A₂ is selected from -CF₃, halo, (C₁-C₆)alkyl, and (C₁-C₃)alkoxyl. Preferably A₃ and A₄ are absent, and A₂ is selected from -CF₃, Cl, F, (C₁-C₆)alkyl, methoxyl and ethoxyl. More preferably, Preferably A₃ and A₄ are absent, and A₂ is selected from -CF₃, Cl, F, methyl, t-butyl, methoxyl and ethoxyl.

In another specific embodiment, A₄ is absent, A₃ is (C₁-C₆)alkyl preferably methyl or t-butyl, and A₂ is selected from -CF₃, halo, (C₁-C₆)alkyl, and (C₁-C₃)alkoxyl. Preferably A₄ is absent, A₃ is (C₁-C₆)alkyl preferably methyl or t-butyl, and A₂ is selected from -CF₃, Cl, F, (C₁-C₆)alkyl, methoxyl and ethoxyl. More preferably, Preferably A₄ is absent, A₃ is (C₁-C₆)alkyl preferably methyl or t-butyl, and A₂ is selected from -CF₃, Cl, F, methyl, t-butyl, methoxyl and ethoxyl.

In another specific embodiment, A₂, A₃ and A₄ are absent.

In a specific embodiment, A₁ is -CF₃; halo such as fluorine or chlorine; or a group G1 selected from a (C₁-C₆)alkyl, (C₃-C₁₀)cycloalkyl, aryl, heterocycle, (C₁-C₆)alkyl-aryl, (C₁-C₆)alkyl-heterocycle,
said group G1 being optionally substituted with at least one, such as one or two, substituent S1 independently selected from a halo; -CN; -NR_{A}R_{B}; or a group G2 selected from a (C₁-C₆)alkyl, (C₃-C₁₀)cycloalkyl, aryl, heterocycle, (C₁-C₆)alkyl-aryl, and (C₁-C₆)alkyl-heterocycle,
   said group G2 being optionally substituted with at least one, such as one or two, substituent S2 independently selected from a halo, -CN, -NR_{A}R_{B}, (C₁-C₆)alkyl group, (C₃-C₁₀)cycloalkyl group, aryl group, heterocycle group, (C₁-C₆)alkyl-aryl, (C₁-C₆)alkyl-heterocycle, and (C₁-C₆)alkoxyl group;
R_{A} and R_{B} being independently an hydrogen atom or a (C₁-C₆)alkyl group.

In another specific embodiment, A₁ is -CF₃; halo such as fluorine or chlorine; -NHC(O)CH₂NR_{A}R_{B}; - NHSO₂R_{C}; -NHR_{E}; -OR_{F}; -C(O)R_{G}; or -C(O)NHR_{H}; wherein:
R_{A} and R_{B} are, independently of each other, an hydrogen atom or a (C₁-C₆)alkyl group; and
R_{C}, R_{E}, R_{F}, R_{G} and R_{H} are, independently of each other, a group G3 selected from a phenyl, (C₁-C₆)alkyl, heterocycle, (C₁-C₆)alkyl-phenyl, and (C₁-C₆)alkyl-heterocycle, said group G3 being optionally substituted with at least one, such as one or two, substituent S3 independently selected from -CN, halo, -CF₃ and a (C₁-C₆)alkyl.

In this embodiment, R_{A}, R_{B} and R_{C}, are preferably, independently of each other, an hydrogen atom, a methyl or an ethyl. In this embodiment, R_{F}, R_{G} and R_{H} are preferably, independently of each other, a group G3 selected from a (C₁-C₆)alkyl, a phenyl, (C₁-C₆)alkyl-phenyl, heterocycle, and (C₁-C₆)alkyl-heterocycle; the heterocycle being a nitrogen-heterocycle comprising 5 or 6 cyclic atoms, preferably a pyrrolidinyl or piperazinyl. The group G3 is preferably unsubstituted or substituted with at least one, such as one or two, substituent S3 independently selected from -CN, halo, -CF₃ and a (C₁-C₆)alkyl, preferably -CN, F, Cl or methyl.

In a specific embodiment, in the definitions of A₁, the aryl is a phenyl or a naphthyl, preferably a phenyl.

In a specific embodiment, in the definitions of A₁, the heterocycle group is a heteroaryl or heterocycloalkyl group comprising 5 to 10 cyclic atoms and preferably comprising 1, 2 or 3 heteroatoms, more preferably 1 or 2 heteroatoms. Preferably, the heterocycle group is a heteroaryl or heterocycloalkyl group comprising 5 to 8 cyclic atoms and preferably comprising 1 or 2 heteroatoms. Preferably, the heterocycle group is a heteroaryl or heterocycloalkyl group comprising 5 to 8 cyclic atoms and preferably comprising one or two heteroatoms including at least one nitrogen atom. In particular, in the definitions of A₁, the heterocycle group is preferably selected in the group consisting of furanyl, thiophenyl, pyrrolyl, pyrrolinyl, pyrrolidinyl, pyrazolyl, imidazolyl, isoxazolyl, oxazolyl, tetrahydropyridinyl, pyridinyl, piperidinyl, pyridazinyl, pyrimidinyl, pyrazinyl, piperazinyl, indolinyl, quinolinyl, cinnolinyl, phtalazinyl, isoquinolinyl, quinazolinyl, quinoxalinyl, morpholinyl, hydroxypyridinyl, thiazolyl, and 1,2-dihydropyrazolyl-3-one. Preferably, the heterocycle group is preferably selected in the group consisting of furanyl, thiophenyl, pyrrolyl, pyrrolinyl, pyrrolidinyl, pyrazolyl, imidazolyl, isoxazolyl, oxazolyl, tetrahydropyridinyl, pyridinyl, , pyridazinyl, pyrimidinyl, pyrazinyl, hydroxypyridinyl, thiazolyl, and 1,2-dihydropyrazolyl-3-one.

In a specific embodiment, in the definitions of A₁, the (C₁-C₆)alkyl-aryl is a methyl-aryl or an ethyl-aryl, preferably a methyl-phenyl or an ethyl-phenyl.

In a specific embodiment, in the definitions of A₁, the (C₁-C₆)alkyl-heterocycle is a methyl-heterocycle or an ethyl-heterocycle, preferably a methyl-morpholinyl, methyl-piperazinyl, methyl-pyrrolidinyl, ethyl-morpholinyl, ethyl-piperazinyl, or ethyl-pyrrolidinyl.

In a specific embodiment, A is a moiety selected from the group consisting of the moieties listed in the Table 1 below, the wavy line being the bond between A and the X.

In a specific embodiment, in the formula (I), X is -X₁C(O)X₂-; with X₁ and X₂ being, independently of each other, absent, -CH₂- or -NH-; and Ar being a phenyl, naphthyl or a heteroaryl comprising 5 or 6 cyclic atoms. Accordingly, the compounds for use according to the invention can be of the following formula (I-1) or (II) as described below.

In another aspect of the invention, in the compound of formula (I), X is -X₁C(O)-, with X₁ being absent, -CH₂- or -NH-; and Ar being: 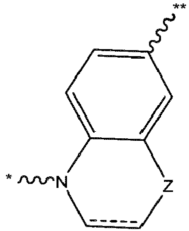 , with Z being -O-, -S-, -CH₂-, -NR_{z}- or absent; R_{z} being H , (C₁-C₆)alkyl group, -CO-(C₁-C₆)alkyl group, -SO-(C₁-C₆)alkyl group or -SO₂-(C₁-C₆)alkyl group;, and with 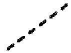 indicating an optional double bond; 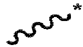 indicating the bound between Ar and X; and 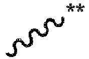 indicating the bound between Ar and the oxygen atom. Accordingly, the compounds for use according to the invention can be of the following formula (I-2) as described below.

In another specific aspect of the invention, in the compound of formula (I), X is-NH-, and Ar is 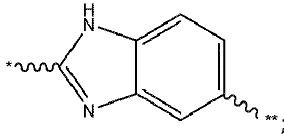 with 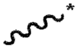 indicating the bound between Ar and X; and 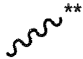 indicating the bound between Ar and the oxygen atom. Accordingly, the compounds for use according to the invention can be of the following formula (I-3) as described below.

### Compounds of general formula (I-1) and (II) for use according to the invention

In a specific embodiment, in the formula (I), X is -X₁C(O)X₂-; with X₁ and X₂ being, independently of each other, absent, -CH₂- or -NH-; and Ar being a phenyl, naphthyl or a heteroaryl comprising 5 or 6 cyclic atoms. Accordingly, the compounds for use according to the invention can be of the following formula (I-1): 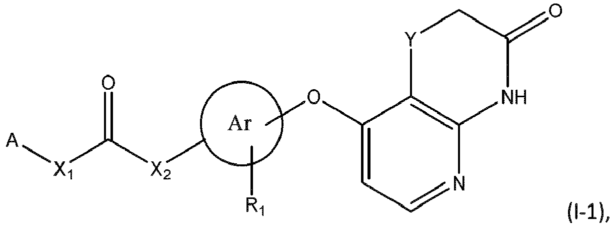 wherein:
X₁ and X₂ are, independently of each other, absent, -CH₂- or -NH-;
Ar is a phenyl, naphthyl or heteroaryl comprising 5 or 6 cyclic atoms; and
R₁, Y and A are as defined above.

Preferably, in the compound of formula (I-1), Ar is a phenyl, naphthyl, or a nitrogen-heteroaryl comprising 5 or 6 cyclic atoms; more preferably Ar is a phenyl, naphthyl, pyridinyl, pyridazinyl, pyrimidinyl or pyrazinyl. In particular, Ar is a phenyl, naphthyl or pyridinyl, preferably a phenyl or pyridinyl, more preferably a phenyl.

Preferably, in the compound of formula (I-1) or (II), Ar is a phenyl substituted with R₁; R₁ being selected in the group consisting of a halogen such as chlorine and fluorine, (C₁-C₆)alkyl group, (C₁-C₆)heteroalkyl group, (C₁-C₆)haloalkyl group, said group being optionally substituted. In this context, the moiety AX₁C(O)X₂- is preferably in *meta* or *para* position, more preferably *para,* on the phenyl Ar.

In a preferred embodiment, the compound for use according to the invention is of the following general formula (II): 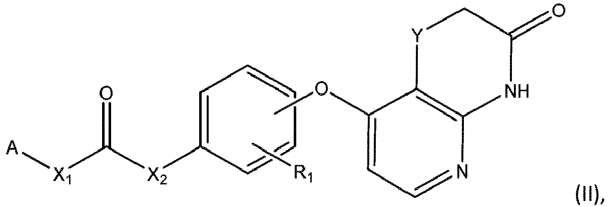 wherein A, R₁ and Y are as defined above; and X₁ and X₂ are, independently of each other, absent, -CH₂- or -NH-.

In particular, the compound for use according to the invention is of the following general formula (II-1) or (II-2): 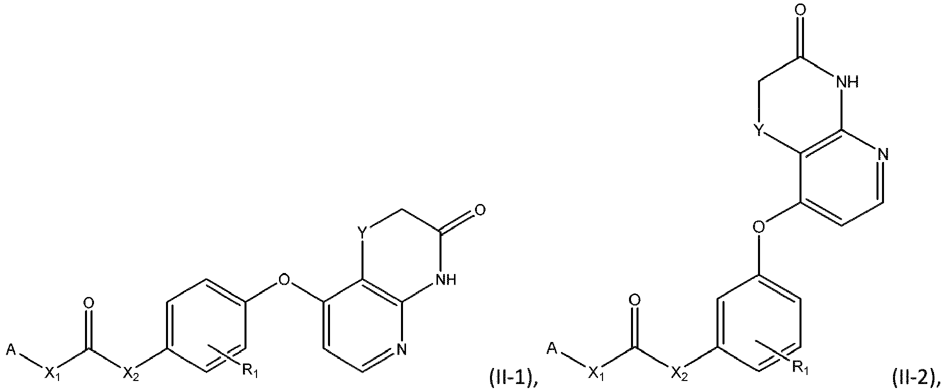 wherein A, R₁ and Y are as defined above; and X₁ and X₂ are, independently of each other, absent, -CH₂- or -NH-.

Preferably, in the compound of formula (I-1) or (II), X₁ is -NH- or -CH₂-; and X₂ is -NH- or absent. More preferably, X₁ and X₂ are both -NH-.

In a specific embodiment, in the compound of general formula (I-1) or (II), R₁ is a (C₁-C₆)alkyl group, (C₁-C₆)heteroalkyl group comprising an oxygen or a sulfur atom, (C₁-C₆)haloalkyl group such as -CF₃, or a halogen such as chlorine and fluorine. Preferably, R₁ is a (C₁-C₆)alkyl group, -SMe, -SEt, -OMe, -OEt, -CF₃, or a halogen such as chlorine and fluorine. In a more specific embodiment, R₁ is a methyl, halogen such as chlorine and fluorine, -SMe or -CF₃.

In a specific embodiment, in the compound of general formula (I-1) or (II), R₁ can be in *ortho, meta* or *para* position on the phenyl Ar. Preferably, R₁ is in *ortho* or *meta* position. In a particular embodiment, the moiety AX₁C(O)X₂- is preferably in *para* position and R₁ is in *meta* position.

### Compounds of general formula (I-2) for use according to the invention

In another aspect of the invention, in the compound of formula (I), X is -X₁C(O)-, with X₁ being absent, - CH₂- or -NH-; and Ar being: 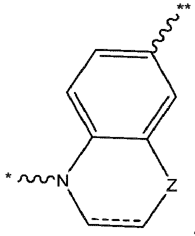 , with Z being -O-, -S-, -CH₂-, -NR_{z}- or absent; R_{z} being H , (C₁-C₆)alkyl group, -CO-(C₁-C₆)alkyl group, -SO-(C₁-C₆)alkyl group or -SO₂-(C₁-C₆)alkyl group; and with 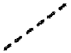 indicating an optional double bond; 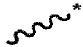 indicating the bound between Ar and X; and 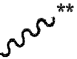 indicating the bound between Ar and the oxygen atom.

Accordingly, the compounds for use according to the invention can be of the following formula (I-2): 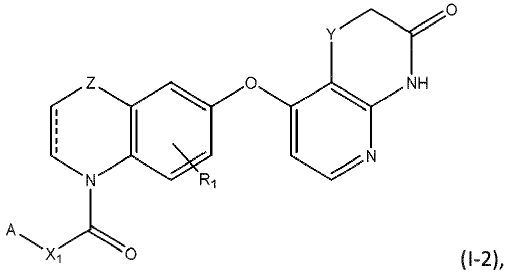 wherein: 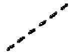 indicates an optional double bond;
Z is -O-, -S-, -CH₂-, -NR_{z}- or absent; R_{z} being H , (C₁-C₆)alkyl group, -CO-(C₁-C₆)alkyl group, -SO-(C₁-C₆)alkyl group or -SO₂-(C₁-C₆)alkyl group;
X₁ is absent, -CH₂- or -NH-, preferably -NH-; and
Y and A are as defined above.

In a specific embodiment, Z is preferably -O-, -S-, -N(CH₃)-, -N(CO-CH₃)-, -N(SO-CH₃)-, -N(SO₂-CH₃)-, or absent. Z is preferably -O-, -S- or absent; more preferably, Z is -O- or -S-.

In a specific embodiment, 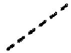 indicates a double bond. In another specific embodiment, 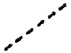 is absent.

In a specific embodiment, in the compound of formula (I-2), R₁ can be absent or a (C₁-C₆)alkyl group, (C₁-C₆)heteroalkyl group, (C₁-C₆)haloalkyl group, or a halogen such as chlorine and fluorine. In particular, R₁ can be absent or a (C₁-C₆)alkyl group, (C₁-C₆)heteroalkyl group comprising an oxygen or a sulfur atom, (C₁-C₆)haloalkyl group such as -CF₃, or a halogen such as chlorine and fluorine. Preferably, R₁ is absent or is a (C₁-C₆)alkyl group, -SMe, -SEt, -OMe, -OEt, -CF₃, or a halogen such as chlorine and fluorine. In a more specific embodiment, R₁ is absent, or a methyl, halogen such as chlorine and fluorine, -SMe or -CF₃. More preferably, R₁ is absent.

In a specific embodiment, in the compound of formula (I-2), X is -X₁C(O)-, with X₁ being -NH- or -CH₂-, more preferably -NH-.

In the context of the invention, in the compound of formula (I-2), A is as defined above.

In a specific embodiment, in the compound of formula (I-2), A is preferably a group selected from (C₃-C₁₀)cycloalkyl, aryl and heterocycle comprising 5 or 6 cyclic atoms, said group being optionally substituted with A₁ and/or A₂. Preferably, A is a group selected from (C₃-C₆)cycloalkyl, phenyl, naphthyl, and a heterocycle comprising 5 or 6 cyclic atoms and preferably one or two heteroatoms; said group being optionally substituted with A₁ and/or A₂. More preferably A is selected from the group consisting of cyclopropyl, phenyl, furanyl, thiophenyl, pyrrolyl, pyrrolinyl, pyrrolidinyl, pyrazolyl, imidazolyl, isoxazolyl, oxazolyl, tetrahydropyridinyl, pyridinyl, piperidinyl, pyridazinyl, pyrimidinyl, pyrazinyl, piperazinyl, morpholinyl, hydroxypyridinyl, thiazolyl, and 1,2-dihydropyrazolyl-3-one; said group being optionally substituted with A₁ and/or A₂. More preferably, A is selected from the group consisting of cyclopropyl, phenyl or pyridinyl; said group being optionally substituted with A₁ and/or A₂. More preferably, A is a phenyl optionally substituted with A₁ and/or A₂. In these definitions, A₁ and A₂ are as defined above.

In a specific embodiment, in the compound of formula (I-2), A₁ is -CF₃; halo such as fluorine or chlorine; or a group G1 selected from a (C₁-C₆)alkyl, (C₃-C₁₀)cycloalkyl, aryl, heterocycle, (C₁-C₆)alkyl-aryl, (C₁-C₆)alkyl-heterocycle. In particular, A₁ is -CF₃; halo such as fluorine or chlorine; or a methyl. In these definitions, the group G1 is optionally substituted with at least one, such as one or two, substituent S1 independently selected from a halo; -CN; -NR_{A}R_{B}; or a group G2 selected from a (C₁-C₆)alkyl, (C₃-C₁₀)cycloalkyl, aryl, heterocycle, (C₁-C₆)alkyl-aryl, and (C₁-C₆)alkyl-heterocycle,
said group G2 being optionally substituted with at least one, such as one or two, substituent S2 independently selected from a halo, -CN, -NR_{A}R_{B}, (C₁-C₆)alkyl group, (C₃-C₁₀)cycloalkyl group, aryl group, heterocycle group, (C₁-C₆)alkyl-aryl, (C₁-C₆)alkyl-heterocycle, and (C₁-C₆)alkoxyl group;
R_{A} and R_{B} being independently an hydrogen atom or a (C₁-C₆)alkyl group.

### Compounds of general formula (I-3) for use according to the invention

In another specific aspect of the invention, in the compound of formula (I), X is-NH-, and Ar is 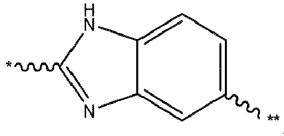 ; with 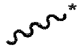 indicating the bound between Ar and X; and 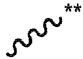 indicating the bound between Ar and the oxygen atom.

Accordingly, the compounds for use according to the invention can be of the following formula (I-3) 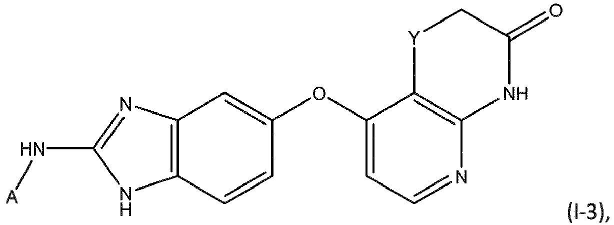 wherein Y and A are as defined above.

In a specific embodiment, in the compound of formula (I-3), A is preferably a group selected from (C₃-C₁₀)cycloalkyl, aryl and heterocycle comprising 5 or 6 cyclic atoms, said group being optionally substituted with A₁ and/or A₂. Preferably, A is a group selected from (C₃-C₆)cycloalkyl, phenyl, naphthyl, and a heterocycle comprising 5 or 6 cyclic atoms and preferably at least one, such as one or two, heteroatoms; said group being optionally substituted with A₁ and/or A₂. More preferably A is selected from the group consisting of cyclopropyl, phenyl, furanyl, thiophenyl, pyrrolyl, pyrrolinyl, pyrrolidinyl, pyrazolyl, imidazolyl, isoxazolyl, oxazolyl, tetrahydropyridinyl, pyridinyl, piperidinyl, pyridazinyl, pyrimidinyl, pyrazinyl, piperazinyl, morpholinyl, hydroxypyridinyl, thiazolyl, and 1,2-dihydropyrazolyl-3-one; said group being optionally substituted with A₁ and/or A₂. More preferably, A is selected from the group consisting of cyclopropyl, phenyl or pyridinyl; said group being optionally substituted with A₁ and/or A₂. More preferably, A is a phenyl optionally substituted with A₁ and/or A₂. In these definitions, A₁ and A₂ are as defined above.

In a specific embodiment, in the compound of formula (I-3), A₁ is -CF₃; halo such as fluorine or chlorine; or a group G1 selected from a (C₁-C₆)alkyl, (C₃-C₁₀)cycloalkyl, aryl, heterocycle, (C₁-C₆)alkyl-aryl, (C₁-C₆)alkyl-heterocycle. In particular, A₁ is -CF₃; halo such as fluorine or chlorine; or a methyl. In these definitions, the group G1 is optionally substituted with at least one, such as one or two, substituent S1 independently selected from a halo; -CN; -NR_{A}R_{B}; or a group G2 selected from a (C₁-C₆)alkyl, (C₃-C₁₀)cycloalkyl, aryl, heterocycle, (C₁-C₆)alkyl-aryl, and (C₁-C₆)alkyl-heterocycle,
said group G2 being optionally substituted with at least one, such as one or two, substituent S2 independently selected from a halo, -CN, -NR_{A}R_{B}, (C₁-C₆)alkyl group, (C₃-C₁₀)cycloalkyl group, aryl group, heterocycle group, (C₁-C₆)alkyl-aryl, (C₁-C₆)alkyl-heterocycle, and (C₁-C₆)alkoxyl group;
R_{A} and R_{B} being independently an hydrogen atom or a (C₁-C₆)alkyl group.

In the context of the invention, the compound of formula (I) for use according to the invention is preferably selected from the list consisting of the compounds listed in Table 2 below, or a pharmaceutically acceptable salt and/or solvate thereof. Preferably, the compound of formula (I) for use according to the invention is preferably selected from the list consisting of compounds 001, 002, 003, 004, 005, 006, 007, 008, 009, 010, 011, 012, 013, 014, 015, 016, 017, 018, 019, 020, 021, 022, 023, 024, 026, 027, 028, 029, 030, 031, 032, 033, 034, 035, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, and 087, as defined in Table 2, or a pharmaceutically acceptable salt and/or solvate thereof. More preferably, the compound of formula (I) for use according to the invention is preferably selected from the list consisting of compounds 001, 002, 003, 004, 005, 006, 007, 008, 009, 010, 011, 012, 013, 014, 015, 016, 017, 018, 019, 020, 021, 022, 023, 024, 026, 027, 028, 029, 030, 031, 032, 033, 034, 035, 037, 038, 039, 040, 041, 042, 043, 044, 045, 046, 047, 048, 049, 050, 051, 052, 053, 054, 058, 059, 060, 061, 062, 063, 064, 065, 066, 067, 068, 069, 070, 071, 072, 073, 074, 075, 076, 077, 078, 079, 080, 081, 082, 083, 084, 085, 086, and 087, as defined in Table 2, or a pharmaceutically acceptable salt and/or solvate thereof.

In the context of the invention, the compounds are for use for treating cancer and/or for reducing or preventing the appearance of metastases in a patient afflicted with a cancer, the cancer being preferably selected from the group consisting of angiogenic and/or metastatic cancers such as bladder, brain, breast, colon, kidney, liver, lung, melanoma, ovary, muscle, pancreas, prostate, rectal, sarcoma, stomach, thyroid, and uterus cancers.

In the context of the invention, muscle cancer comprises Granular Cell Tumor, Leiomyosarcoma, Myosarcoma such as Rhabdomyosarcoma (RMS), Smooth Muscle Tumor and Alveolar Soft-Part Sarcoma.

In a specific embodiment of the invention, the cancer is a muscle cancer, in particular a muscle cancer selected from Granular Cell Tumor, Leiomyosarcoma, Myosarcoma such as Rhabdomyosarcoma (RMS), Smooth Muscle Tumor and Alveolar Soft-Part Sarcoma. In a preferred embodiment of the invention, the cancer is Rhabdomyosarcoma (RMS).

### New compounds according to the invention

The invention also relates to a compound of general formula (II), or a pharmaceutically acceptable salt and/or solvate thereof: 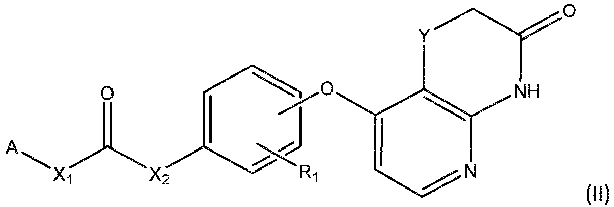 wherein,
X₁ and X₂ are, independently of each other, absent, -CH₂- or -NH-;
R₁ is selected in the group consisting of a (C₁-C₆)alkyl group, (C₁-C₆)heteroalkyl group, (C₁-C₆)haloalkyl group, or a halogen;
Y is an oxygen atom or -CH₂-; and
A is an aryl, preferably a phenyl, or a (C₃-C₁₀)cycloalkyl, preferably a cyclopropyl, and is optionally substituted with A₁, A₂, A₃ and/or A₄, wherein
   A₁ is -CF₃; halo; -NHC(O)CH₂NR_{A}R_{B}; -NHSO₂R_{C}; -NHR_{E}; -OR_{F}; -C(O)R_{G}; -C(O)NHR_{H}; or A₁ is a group G1 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
      said group G1 being optionally substituted with at least one, such as one or two, substituent S1 independently selected from a halo; -CN; -NR_{A}R_{B}; -R_{D}NR_{A}R_{B}; or a group G2 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
         said group G2 being optionally substituted with at least one, such as one or two, substituent S2 independently selected from a halo, -CN, -NR_{A}R_{B}, -R_{D}NR_{A}R_{B}, (C₁-C₆)alkyl group, (C₃-C₁₀)cycloalkyl group, aryl group, heterocycle group, (C₁-C₆)alkylaryl, (C₁-C₆)alkyl-heterocycle, and (C₁-C₆)alkoxyl group;
      R_{A} and R_{B} being independently an hydrogen atom or a (C₁-C₆)alkyl group;
      R_{C}, R_{D}, R_{E}, R_{F}, R_{G} and R_{H} being independently a group G3 selected from a phenyl, (C₁-C₆)alkyl, heterocycle, (C₁-C₆)alkyl-phenyl, and (C₁-C₆)alkyl-heterocycle, said group G3 being optionally substituted with at least one, such as one or two, substituent S3 independently selected from -CN, halo, -CF₃ and a (C₁-C₆)alkyl ;
   and
   A₂, A₃ and A₄ are independently selected from -CF₃, halo, (C₁-C₆)alkyl, and (C₁-C₃)alkoxyl.

In this specification, such a compound according to the invention is named "new compound" or "new compound of formula (II)" or "new compound according to the invention".

In a specific embodiment, the new compound according to the invention is preferably of the following general formula (II-1) or (II-2): 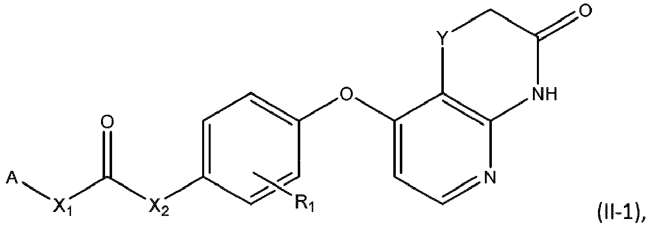 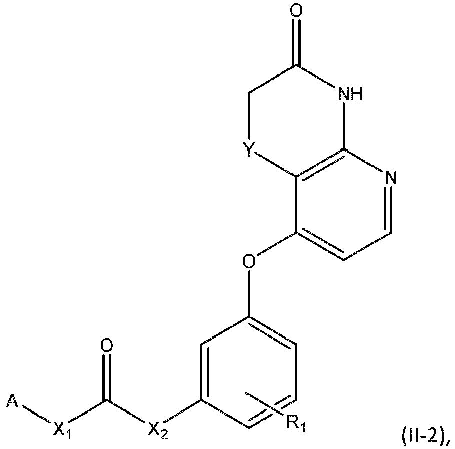 wherein A, X₁, X₂, R₁ and Y are as defined above.

In this context of the invention, R₁ is a halogen such as chlorine and fluorine, a (C₁-C₆)alkyl group, (C₁-C₆)heteroalkyl group, (C₁-C₆)haloalkyl group, said groups being optionally substituted. In a specific embodiment, R₁ is a (C₁-C₆)alkyl group, (C₁-C₆)heteroalkyl group comprising an oxygen or a sulfur atom, (C₁-C₆)haloalkyl group such as -CF₃, or a halogen such as chlorine and fluorine. Preferably, R₁ is a (C₁-C₆)alkyl group, -SMe, -SEt, -OMe, -OEt, -CF₃, or a halogen such as chlorine and fluorine. In a more specific embodiment, R₁ is a methyl, halogen such as chlorine and fluorine, -SMe or -CF₃.

In this context of the invention, Y is an oxygen atom or -CH₂-. In a specific embodiment, Y is an oxygen atom. In another specific embodiment, Y is -CH₂-.

In this context of the invention, in the compound of formula (II), in particular (II-1) and (II-2), X₁ and X₂ are, independently of each other, absent, -CH₂- or -NH-. Preferably, X₁ is -NH- or -CH₂-; and X₂ is -NH- or absent. More preferably, X₁ and X₂ are both -NH-.

In this context of the invention, A is an aryl, preferably a phenyl, or a (C₃-C₁₀)cycloalkyl, preferably a cyclopropyl, and is optionally substituted with A₁, A₂, A₃ and/or A₄, wherein
A₁ is -CF₃; halo; -NHC(O)CH₂NR_{A}R_{B}; -NHSO₂R_{c}; -NHR_{E}; -OR_{F}; -C(O)R_{G}; -C(O)NHR_{H}; or A₁ is a group G1 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
   said group G1 being optionally substituted with at least one, such as one or two, substituent S1 independently selected from a halo; -CN; -NR_{A}R_{B}; -R_{D}NR_{A}R_{B}; or a group G2 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
      said group G2 being optionally substituted with at least one, such as one or two, substituent S2 independently selected from a halo, -CN, -NR_{A}R_{B}, -R_{D}NR_{A}R_{B}, (C₁-C₆)alkyl group, (C₃-C₁₀)cycloalkyl group, aryl group, heterocycle group, (C₁-C₆)alkylaryl, (C₁-C₆)alkyl-heterocycle, and (C₁-C₆)alkoxyl group;
   R_{A} and R_{B} being independently an hydrogen atom or a (C₁-C₆)alkyl group;
   R_{C}, R_{D}, R_{E}, R_{F}, R_{G} and R_{H} being independently a group G3 selected from a phenyl, (C₁-C₆)alkyl, heterocycle, (C₁-C₆)alkyl-phenyl, and (C₁-C₆)alkyl-heterocycle, said group G3 being optionally substituted with at least one, such as one or two, substituent S3 independently selected from -CN, halo, -CF₃ and a (C₁-C₆)alkyl ;
and
A₂, A₃ and A₄ are independently selected from -CF₃, halo, (C₁-C₆)alkyl, and (C₁-C₃)alkoxyl.

In a specific embodiment, in the definition of A, the aryl is a phenyl or a naphthyl, preferably a phenyl.

In a specific embodiment, in the definition of A, the cycloalkyl is preferably a (C₃-C₆)cycloalkyl, more preferably a cyclopropyl.

In a specific embodiment, in the new compound of formula (II), in particular (II-1) and (II-2), A₄ is absent.

In a specific embodiment, in the new compound of formula (II), in particular (II-1) and (II-2), A₃ is absent or is (C₁-C₆)alkyl.

In a specific embodiment, in the new compound of formula (II), in particular (II-1) and (II-2), A₂ is absent or is selected from -CF₃, Cl, F, (C₁-C₆)alkyl, and (C₁-C₃)alkoxyl. Preferably, A₂ is selected from -CF₃, Cl, F, (C₁-C₆)alkyl, methoxyl and ethoxyl. More preferably, A₂ is selected from -CF₃, Cl, F, methyl, t-butyl, methoxyl and ethoxyl.

In a specific embodiment, in the new compound of formula (II), in particular (II-1) and (II-2), A₃ and A₄ are absent, and A₂ is selected from -CF₃, halo, (C₁-C₆)alkyl, and (C₁-C₃)alkoxyl. Preferably A₃ and A₄ are absent, and A₂ is selected from -CF₃, Cl, F, (C₁-C₆)alkyl, methoxyl and ethoxyl. More preferably, Preferably A₃ and A₄ are absent, and A₂ is selected from -CF₃, Cl, F, methyl, t-butyl, methoxyl and ethoxyl.

In another specific embodiment, in the new compound of formula (II), in particular (II-1) and (II-2), A₄ is absent, A₃ is (C₁-C₆)alkyl preferably methyl or t-butyl, and A₂ is selected from -CF₃, halo, (C₁-C₆)alkyl, and (C₁-C₃)alkoxyl. Preferably A₄ is absent, A₃ is (C₁-C₆)alkyl preferably methyl or t-butyl, and A₂ is selected from -CF₃, Cl, F, (C₁-C₆)alkyl, methoxyl and ethoxyl. More preferably, Preferably A₄ is absent, A₃ is (C₁-C₆)alkyl preferably methyl or t-butyl, and A₂ is selected from -CF₃, Cl, F, methyl, t-butyl, methoxyl and ethoxyl.

In another specific embodiment, in the new compound of formula (II), in particular (II-1) and (II-2), A₂, A₃ and A₄ are absent.

In a specific embodiment, in the new compound of formula (II), in particular (II-1) and (II-2), A₁ is -CF₃; halo such as fluorine or chlorine; or a group G1 selected from a (C₁-C₆)alkyl, (C₃-C₁₀)cycloalkyl, aryl, heterocycle, (C₁-C₆)alkyl-aryl, (C₁-C₆)alkyl-heterocycle,
said group G1 being optionally substituted with at least one, such as one or two, substituent S1 independently selected from a halo; -CN; -NR_{A}R_{B}; or a group G2 selected from a (C₁-C₆)alkyl, (C₃-C₁₀)cycloalkyl, aryl, heterocycle, (C₁-C₆)alkyl-aryl, and (C₁-C₆)alkyl-heterocycle,
   said group G2 being optionally substituted with at least one, such as one or two, substituent S2 independently selected from a halo, -CN, -NR_{A}R_{B}, (C₁-C₆)alkyl group, (C₃-C₁₀)cycloalkyl group, aryl group, heterocycle group, (C₁-C₆)alkyl-aryl, (C₁-C₆)alkyl-heterocycle, and (C₁-C₆)alkoxyl group;
R_{A} and R_{B} being independently an hydrogen atom or a (C₁-C₆)alkyl group.

In another specific embodiment, in the new compound of formula (II), in particular (II-1) and (II-2), A₁ is -CF₃; halo such as fluorine or chlorine; -NHC(O)CH₂NR_{A}R_{B}; -NHSO₂R_{C}; -NHR_{E}; -OR_{F}; -C(O)R_{G}; or -C(O)NHR_{H}; wherein:
R_{A} and R_{B} are, independently of each other, an hydrogen atom or a (C₁-C₆)alkyl group; and
R_{C}, R_{E}, R_{F}, R_{G} and R_{H} are, independently of each other, a group G3 selected from a phenyl, (C₁-C₆)alkyl, heterocycle, (C₁-C₆)alkyl-phenyl, and (C₁-C₆)alkyl-heterocycle, said group G3 being optionally substituted with at least one, such as one or two, substituent S3 independently selected from -CN, halo, -CF₃ and a (C₁-C₆)alkyl.

In this embodiment, R_{A}, R_{B} and R_{C}, are preferably, independently of each other, an hydrogen atom, a methyl or an ethyl. In this embodiment, R_{F}, R_{G} and R_{H} are preferably, independently of each other, a group G3 selected from a (C₁-C₆)alkyl, a phenyl, (C₁-C₆)alkyl-phenyl, heterocycle, and (C₁-C₆)alkyl-heterocycle; the heterocycle being a nitrogen-heterocycle comprising 5 or 6 cyclic atoms, preferably a pyrrolidinyl or piperazinyl. The group G3 is preferably unsubstituted or substituted with at least one, such as one or two, substituent S3 independently selected from -CN, halo, -CF₃ and a (C₁-C₆)alkyl, preferably -CN, F, Cl or methyl.

In a specific embodiment, in the definitions of A₁, the aryl is a phenyl or a naphthyl, preferably a phenyl.

In a specific embodiment, in the definitions of A₁ (including G1, S1, G2, S2, G3 and S3), the heterocycle group is a heteroaryl or heterocycloalkyl group comprising 5 to 10 cyclic atoms and preferably comprising 1, 2 or 3 heteroatoms, more preferably 1 or 2 heteroatoms. Preferably, the heterocycle group is a heteroaryl or heterocycloalkyl group comprising 5 to 8 cyclic atoms and preferably comprising 1 or 2 heteroatoms. Preferably, the heterocycle group is a heteroaryl or heterocycloalkyl group comprising 5 to 8 cyclic atoms and preferably comprising one or two heteroatoms including at least one nitrogen atom. In particular, in the definitions of Ai, the heterocycle group is preferably selected in the group consisting of furanyl, thiophenyl, pyrrolyl, pyrrolinyl, pyrrolidinyl, pyrazolyl, imidazolyl, isoxazolyl, oxazolyl, tetrahydropyridinyl, pyridinyl, piperidinyl, pyridazinyl, pyrimidinyl, pyrazinyl, piperazinyl, indolinyl, quinolinyl, cinnolinyl, phtalazinyl, isoquinolinyl, quinazolinyl, quinoxalinyl, morpholinyl, hydroxypyridinyl, thiazolyl, and 1,2-dihydropyrazolyl-3-one. Preferably, the heterocycle group is preferably selected in the group consisting of furanyl, thiophenyl, pyrrolyl, pyrrolinyl, pyrrolidinyl, pyrazolyl, imidazolyl, isoxazolyl, oxazolyl, tetrahydropyridinyl, pyridinyl, , pyridazinyl, pyrimidinyl, pyrazinyl, hydroxypyridinyl, thiazolyl, and 1,2-dihydropyrazolyl-3-one.

In a specific embodiment, in the definitions of A₁ (including G1, S1, G2, S2, G3 and S3),, the (C₁-C₆)alkylaryl is a methyl-aryl or an ethyl-aryl, preferably a methyl-phenyl or an ethyl-phenyl.

In a specific embodiment, in the definitions of A₁ (including G1, S1, G2, S2, G3 and S3),, the (C₁-C₆)alkyl-heterocycle is a methyl-heterocycle or an ethyl-heterocycle, preferably a methyl-morpholinyl, methylpiperazinyl, methyl-pyrrolidinyl, ethyl-morpholinyl, ethyl-piperazinyl, or ethyl-pyrrolidinyl.

In a specific embodiment, in the new compound of formula (II), in particular (II-1) and (II-2), A is a moiety selected from the group consisting of the moieties listed in the Table 3 below, the wavy line being the bond between A and the X.

In the context of the invention, the new compound of formula (II) is preferably selected from the list consisting of the compounds listed in Table 4, or a pharmaceutically acceptable salt and/or solvate thereof. Preferably, the compound of formula (II) according to the invention is preferably selected from the list consisting of compounds 058, 059, 062, 063, 066, 067, 069, 070, 072, 073, 074, 075, 076, 078, 079, 081, 082, 083, and 084, as defined in Table 4, or a pharmaceutically acceptable salt and/or solvate thereof.

In a particular aspect, the invention also relates to
- a compound of general formula (II) or a pharmaceutically acceptable salt thereof or a pharmaceutical or veterinary composition comprising such a compound, in particular a compound represented by a structure selected in Table 4, or a pharmaceutically acceptable salt thereof, for use as a medicament, preferably for use in treating, ameliorating, delaying, curing and/or preventing cancer and/or for reducing or preventing the appearance of metastases in a patient afflicted with a cancer,
- the use of a compound of general formula (II) or a pharmaceutically acceptable salt thereof or a pharmaceutical or veterinary composition comprising such a compound, in particular a compound represented by a structure selected in Table 4, or a pharmaceutically acceptable salt thereof, for the manufacture of a medicament, in particular for treating, ameliorating, delaying, curing and/or preventing cancer and/or for reducing or preventing the appearance of metastases in a patient afflicted with a cancer; or
- a method for treating, ameliorating, delaying, curing and/or preventing cancer and/or for reducing or preventing the appearance of metastases in a patient afflicted with a cancer, in a subject in need thereof comprising administering an effective amount of a compound of general formula (II) or a pharmaceutical or veterinary composition comprising such a compound, in particular a compound represented by a structure selected in Table 2 or 4, or a pharmaceutically acceptable salt thereof, thereby treating, ameliorating, delaying, curing and/or preventing cancer and/or reducing or preventing the appearance of metastases in a patient afflicted with a cancer.

In the context of the invention, the cancer is preferably selected from the group consisting of angiogenic and/or metastatic cancers such as bladder, brain, breast, colon, kidney, liver, lung, melanoma, ovary, muscle, pancreas, prostate, rectal, sarcoma, stomach, thyroid, and uterus cancers.

In the context of the invention, muscle cancer comprises Granular Cell Tumor, Leiomyosarcoma, Myosarcoma such as Rhabdomyosarcoma (RMS), Smooth Muscle Tumor and Alveolar Soft-Part Sarcoma.

In a specific embodiment of the invention, the cancer is a muscle cancer, in particular a muscle cancer selected from Granular Cell Tumor, Leiomyosarcoma, Myosarcoma such as Rhabdomyosarcoma (RMS), Smooth Muscle Tumor and Alveolar Soft-Part Sarcoma. In a preferred embodiment of the invention, the cancer is Rhabdomyosarcoma (RMS).

### Pharmaceutical or veterinary compositions and the uses thereof

As illustrated by examples, the inventors have demonstrated the therapeutic interest of the new compounds of the invention. Accordingly, the present invention relates to a pharmaceutical or veterinary composition comprising any new compound according to the present invention, or a pharmaceutically acceptable salt thereof. Preferably, the pharmaceutical or veterinary composition further comprises a pharmaceutically or veterinary acceptable carrier or excipient. The present invention relates to the use of any new compound according to the invention as a drug or as a medicine. The invention further relates to a method for treating a disease in a subject, wherein a therapeutically effective amount of any new compound according to the invention, is administered to said subject in need thereof. The invention also relates to the use of any new compound according to the invention, for the manufacture of a medicine. The invention also relates to a pharmaceutical composition comprising any new compound according to the invention for use as a drug. The terms "any new compound" mean herein any compounds as defined in the section "new compound according to the invention" herein above. The terms "any compound according to the invention" mean herein any compounds of general formula (I) or (II) as defined in the present specification, in particular in the sections "Compounds for use according to the invention" and "New compounds according to the invention" herein above.

More particularly, the present invention provides compounds for treating cancer and/or for reducing or preventing the appearance of metastases in a patient afflicted with a cancer.

Accordingly, the present invention relates to any compound according to the present invention or a pharmaceutically acceptable salt thereof, or a pharmaceutical or veterinary composition comprising it, for use for treating cancer and/or for reducing or preventing the appearance of metastases in a patient afflicted with a cancer. The present invention relates to the use of any compound according to the present invention or a pharmaceutically acceptable salt thereof, or a pharmaceutical or veterinary composition comprising it, for treating cancer and/or for reducing or preventing the appearance of metastases in a patient afflicted with a cancer. The invention further relates to a method for treating cancer and/or for reducing or preventing the appearance of metastases in a patient afflicted with a cancer in a subject, wherein a therapeutically effective amount of any compound according to the present invention or a pharmaceutically acceptable salt thereof, or a pharmaceutical or veterinary composition comprising it is administered to said subject in need thereof. The invention also relates to the use of any compound according to the present invention or a pharmaceutically acceptable salt thereof, or a pharmaceutical or veterinary composition comprising it for the manufacture of a medicine for use for treating cancer and/or for reducing or preventing the appearance of metastases in a patient afflicted with a cancer.

The terms "cancer", "cancerous", or "malignant" refer to or describe the physiological condition in mammals that is typically characterized by unregulated cell growth. Examples of cancer include, for example, leukemia, lymphoma, blastoma, carcinoma and sarcoma.

Various cancers are also encompassed by the scope of the invention, including, but not limited to, the following: carcinoma including that of the bladder (including accelerated and metastatic bladder cancer), brain, breast, colon (including colorectal cancer), kidney, liver, lung (including small and non-small cell lung cancer and lung adenocarcinoma), ovary, muscle, prostate, testis, genitourinary tract, lymphatic system, rectum, larynx, pancreas (including exocrine pancreatic carcinoma), esophagus, stomach, gall bladder, cervix, thyroid, uterus, and skin (including squamous cell carcinoma); hematopoietic tumors of lymphoid lineage including leukemia, acute lymphocytic leukemia, acute lymphoblastic leukemia, B-cell lymphoma, T-cell lymphoma (including cutaneous or peripheral T-cell lymphoma), Hodgkins lymphoma, non-Hodgkins lymphoma, hairy cell lymphoma, histiocytic lymphoma, and Burketts lymphoma; hematopoietic tumors of myeloid lineage including acute and chronic myelogenous leukemias, myelodysplastic syndrome, myeloid leukemia, and promyelocytic leukemia; tumors of the central and peripheral nervous system including astrocytoma, neuroblastoma, glioma, and schwannomas; tumors of mesenchymal origin including fibrosarcoma, rhabdomyosarcoma, and osteosarcoma; other tumors including melanoma, xenoderma pigmentosum, keratoactanthoma, seminoma, thyroid follicular cancer, and teratocarcinoma; melanoma, unresectable stage III or IV malignant melanoma, squamous cell carcinoma, small-cell lung cancer, non-small cell lung cancer, glioma, gastrointestinal cancer, renal cancer, ovarian cancer, liver cancer, colorectal cancer, endometrial cancer, kidney cancer, prostate cancer, thyroid cancer, neuroblastoma, pancreatic cancer, glioblastoma multiforme, cervical cancer, stomach cancer, bladder cancer, hepatocarcinoma, breast cancer, colon carcinoma, and head and neck cancer, retinoblastoma, gastric cancer, germ cell tumor, bone cancer, bone tumors, adult malignant fibrous histiocytoma of bone; childhood malignant fibrous histiocytoma of bone, sarcoma, pediatric sarcoma; myelodysplastic syndromes; neuroblastoma; testicular germ cell tumor, intraocular melanoma, myelodysplastic syndromes; myelodysplastic/myeloproliferative diseases, synovial sarcoma.

In a particular aspect, the cancer is selected among angiogenic and/or metastatic cancers, for instance selected in the group consisting of bladder, brain, breast, colon, kidney, liver, lung, melanoma, ovary, muscle (e.g., Granular Cell Tumor, Leiomyosarcoma, Myosarcoma such as Rhabdomyosarcoma (RMS), Smooth Muscle Tumor and Alveolar Soft-Part Sarcoma), pancreas, prostate, rectal, sarcoma, stomach, thyroid, and uterus cancers.

In a preferred embodiment of the present invention, the cancer is a solid tumor. For instance, the cancer may be sarcoma and osteosarcoma such as Kaposi sarcome, AIDS-related Kaposi sarcoma, melanoma, in particular uveal melanoma, and cancers of the head and neck, kidney, ovary, pancreas, prostate, thyroid, lung, esophagus, breast, in particular triple negative breast cancer (TNBC), bladder, colorectum, liver and biliary tract, uterine, appendix, and cervix, testicular cancer, gastrointestinal cancers and endometrial and peritoneal cancers. Preferably, the cancer may be sarcoma, melanoma, in particular uveal melanoma, and cancers of the head and neck, kidney, ovary, pancreas, prostate, thyroid, lung, esophagus, breast in particular (TNBC), bladder, colorectum, liver, cervix, and endometrial and peritoneal cancers.

In a particular aspect, the cancer can be selected from the group consisting of leukemia, lymphoma, sarcoma, melanoma, and cancers of the head and neck, kidney, ovary, pancreas, prostate, thyroid, lung, esophagus, breast, bladder, brain, colorectum, liver, and cervix.

In another aspect, the cancer can be selected from the group consisting of lung cancer, in particular non-small cell lung cancer, leukemia, in particular acute myeloid leukemia, chronic lymphocytic leukemia, lymphoma, in particular peripheral T-cell lymphoma, chronic myelogenous leukemia, squamous cell carcinoma of the head and neck, advanced melanoma with BRAF mutation, colorectal cancer, gastrointestinal stromal tumor, breast cancer, in particular HER2⁺ breast cancer, thyroid cancer, in particular advanced medullary thyroid cancer, kidney cancer, in particular renal cell carcinoma, prostate cancer, glioma, pancreatic cancer, in particular pancreatic neuroendocrine cancer, multiple myeloma, and liver cancer, in particular hepatocellular carcinoma.

In a particular aspect, the cancer presents KRAS mutation. In this aspect, the cancer can be selected from the group consisting of lung cancer, colorectal cancer and pancreatic cancer, these cancers are frequently driven by KRAS mutations. For instance, KRAS drives 32% of lung cancers, 40% of colorectal cancers, and 85% to 90% of pancreatic cancer cases. G12C, G12D and G12R are some of the most common KRAS mutations.

In the context of the invention the cancer is preferably selected from the group consisting of angiogenic and/or metastatic cancers such as bladder, brain, breast, colon, kidney, liver, lung, melanoma, ovary, muscle, pancreas, prostate, rectal, sarcoma, stomach, thyroid, and uterus cancers.

In the context of the invention, muscle cancer comprises Granular Cell Tumor, Leiomyosarcoma, Myosarcoma such as Rhabdomyosarcoma (RMS), Smooth Muscle Tumor and Alveolar Soft-Part Sarcoma.

In a specific embodiment of the invention, the cancer is a muscle cancer, in particular a muscle cancer selected from Granular Cell Tumor, Leiomyosarcoma, Myosarcoma such as Rhabdomyosarcoma (RMS), Smooth Muscle Tumor and Alveolar Soft-Part Sarcoma. In a preferred embodiment of the invention, the cancer is Rhabdomyosarcoma (RMS).

The pharmaceutical or veterinary composition comprises a compound of the present invention and optionally a pharmaceutically acceptable excipient or carrier.

The compound according to the invention or the pharmaceutical composition according to the invention may be administered by any conventional route of administration. In particular, the compound or the pharmaceutical composition of the invention can be administered by a topical, enteral, oral, parenteral, intranasal, intravenous, intra-arterial, intramuscular, or subcutaneous administration and the like.

Preferably, the compound according to the invention or the pharmaceutical composition according to the invention is administered by enteral or parenteral route of administration. When administered parenterally, the compound according to the invention or the pharmaceutical composition according to the invention is preferably administered by intravenous route of administration. When administered enterally, the compound according to the invention or the pharmaceutical composition according to the invention is preferably administered by oral route of administration.

The pharmaceutical composition comprising the molecule is formulated in accordance with standard pharmaceutical practice (Lippincott Williams & Wilkins, 2000 and Encyclopedia of Pharmaceutical Technology, eds. J. Swarbrick and J. C. Boylan, 1988-1999, Marcel Dekker, New York) known by a person skilled in the art.

For oral administration, the composition can be formulated into conventional oral dosage forms such as tablets, capsules, powders, granules and liquid preparations such as syrups, elixirs, and concentrated drops. Nontoxic solid carriers or diluents may be used which include, for example, pharmaceutical grades of mannitol, lactose, starch, magnesium stearate, sodium saccharine, talcum, cellulose, glucose, sucrose, magnesium, carbonate, polysorbate (e.g., 20, 40, 65, 80, 100, 120), and the like. For compressed tablets, binders, which are agents which impart cohesive qualities to powdered materials, are also necessary. For example, starch, gelatin, sugars such as lactose or dextrose, and natural or synthetic gums can be used as binders. Disintegrants are also necessary in the tablets to facilitate break-up of the tablet. Disintegrants include starches, clays, celluloses, algins, gums and crosslinked polymers. Moreover, lubricants and glidants are also included in the tablets to prevent adhesion to the tablet material to surfaces in the manufacturing process and to improve the flow characteristics of the powder material during manufacture. Colloidal silicon dioxide is most commonly used as a glidant and compounds such as talc or stearic acids are most commonly used as lubricants. Detergents such as dimethyl sulfoxide, dimethyl acetamide and dimethylformamide could be added.

For transdermal administration, the composition can be formulated into ointment, cream or gel form and appropriate penetrants or detergents could be used to facilitate permeation, such as dimethyl sulfoxide, dimethyl acetamide and dimethylformamide.

For transmucosal administration, nasal sprays, rectal or vaginal suppositories can be used. The active compound can be incorporated into any of the known suppository bases by methods known in the art. Examples of such bases include cocoa butter, polyethylene glycols (carbowaxes), polyethylene sorbitan monostearate, and mixtures of these with other compatible materials to modify the melting point or dissolution rate.

Pharmaceutical compositions according to the invention may be formulated to release the active drug substantially immediately upon administration or at any predetermined time or time period after administration.

The amount of compound according to the invention or of pharmaceutical composition according to the invention to be administered has to be determined by standard procedure well known by those of ordinary skills in the art. Physiological data of the patient (e.g. age, size, and weight) and the routes of administration have to be taken into account to determine the appropriate dosage, so as a therapeutically effective amount will be administered to the patient.

In a preferred embodiment, the total compound dose for each administration of the compound according to the invention or of the pharmaceutical composition according to the invention is comprised between 0.00001 and 1 g.

The form of the pharmaceutical compositions, the route of administration and the dose of administration of the compound according to the invention, or the pharmaceutical composition according to the invention can be adjusted by the man skilled in the art according to the type and severity of the disease, and to the patient, in particular its age, weight, sex, and general physical condition.

The pharmaceutical composition of the invention can further comprise at least an additional active ingredient.

The present invention also relates to the combined use of a compound of the present invention with at least an additional active ingredient or with an appropriate dietary regimen.

The additional therapeutic agent can be selected in the non-exhaustive list comprising alkylating agents, angiogenesis inhibitors, antibodies, in particular anti-tumor targeting antibodies, antimetabolites, antimitotics, antiproliferatives, antivirals, aurora kinase inhibitors, apoptosis promoters (for example, Bcl-2 family inhibitors), activators of death receptor pathway, Bcr-Abl kinase inhibitors, BiTE (Bi-Specific T cell Engager) antibodies, antibody drug conjugates, biologic response modifiers, Bruton's tyrosine kinase (BTK) inhibitors, cyclin-dependent kinase inhibitors, cell cycle inhibitors, cyclooxygenase-2 inhibitors, DVDs, leukemia viral oncogene homolog (ErbB2) receptor inhibitors, growth factor inhibitors, heat shock protein (HSP)-90 inhibitors, histone deacetylase (HDAC) inhibitors, hormonal therapies, immunologicals, inhibitors of inhibitors of apoptosis proteins (lAPs), intercalating antibiotics, kinase inhibitors, kinesin inhibitors, Jak2 inhibitors, mammalian target of rapamycin inhibitors, microRNAs, mitogen-activated extracellular signal-regulated kinase inhibitors, multivalent binding proteins, non-steroidal antiinflammatory drugs (NSAIDs), poly ADP (adenosine diphosphate)-ribose polymerase (PARP) inhibitors, platinum chemotherapeutics, polo-like kinase (Plk) inhibitors, phosphoinositide-3 kinase (PI3K) inhibitors, proteasome inhibitors, purine analogs, pyrimidine analogs, receptor tyrosine kinase inhibitors, retinoids/deltoids plant alkaloids, small inhibitory ribonucleic acids (siRNAs), topoisomerase inhibitors, ubiquitin ligase inhibitors, hypomethylating agents, checkpoints inhibitors, peptide vaccine and the like, epitopes or neoepitopes from tumor antigens, as well as combinations of one or more of these agents.

For instance, the additional therapeutic agent can be selected in the group consisting of chemotherapy, radiotherapy, targeted therapy, anti-tumor targeting antibodies, antiangiogenic agents, hypomethylating agents, cancer vaccines, epitopes or neoepitopes from tumor antigens, myeloid checkpoints inhibitors, other immunotherapies, and HDAC inhibitors.

In a particular aspect, the active ingredient can be an immunomodulator such as an immune checkpoint inhibitor (ICI). In certain embodiments, the immunomodulator is an activator of a costimulatory molecule. In one aspect, the agonist of the costimulatory molecule is selected from an agonist (e.g., an agonistic antibody or antigen-binding fragment thereof, or a soluble fusion) of OX40, CD2, CD27, CDS, ICAM-1, LFA-1 (CD11a/CD18), ICOS (CD278), 4-1 BB (CD137), GITR, CD30, CD40, BAFFR, HVEM, CD7, LIGHT, NKG2C, SLAMF7, NKp80, CD160, B7-H3 or CD83 ligand. In certain aspect, the immunomodulator is an inhibitor of an immune checkpoint molecule. In one embodiment, the immunomodulator is an inhibitor of PD-1, PD-L1, PD-L2, CTLA-4, TIM-3, LAG-3, NKG2D, NKG2L, KIR, VISTA, BTLA, TIGIT, LAIR1, CD160, 2B4 and/or TGFRbeta. In one embodiment, the inhibitor of an immune checkpoint molecule inhibits PD-1, PD-L1, LAG-3, TIM-3 or CTLA-4, or any combination thereof.

For instance, the additional therapeutic agent is selected from the group consisting of chemotherapeutic agents, radiotherapy agents, immunotherapeutic agents, cell therapy agents (such as CAR-T cells), antibiotics and probiotics.

Said immunotherapeutic agent can also be an antibody targeting tumoral antigen, particularly selected from the group consisting of anti-Her2, anti-EGFR, anti-CD20, anti-CD19 and anti-CD52. Said antibodies can be cytotoxic antibodies targeting tumor cells or antibodies inducing cytolytic activity of immune cells (such as NK cells, T cells or macrophages) against tumor cells. A non-exhaustive list of such antibodies includes rituximab, pertuzumab, alemtuzumab, atezolizumab, bevacizumab, cetuximab, herceptin, panitumumab, necitumumab, dinutuximab, ramucirumab, olaratumab, ipilimumab, cemiplimab, tremelimumab, CS1001, relatlimab, naxitamab, margetuximab, BAT8001, KN035, isatuximab, andecaliximab, bemarituzumab, trastuzumab, anti-PD1 antibody, anti-PDL-1, anti-CD47 antibody, and anti-SIRPa antibody.

Further aspects and advantages of the present invention will be described in the following examples, which should be regarded as illustrative and not limiting.

### TABLES

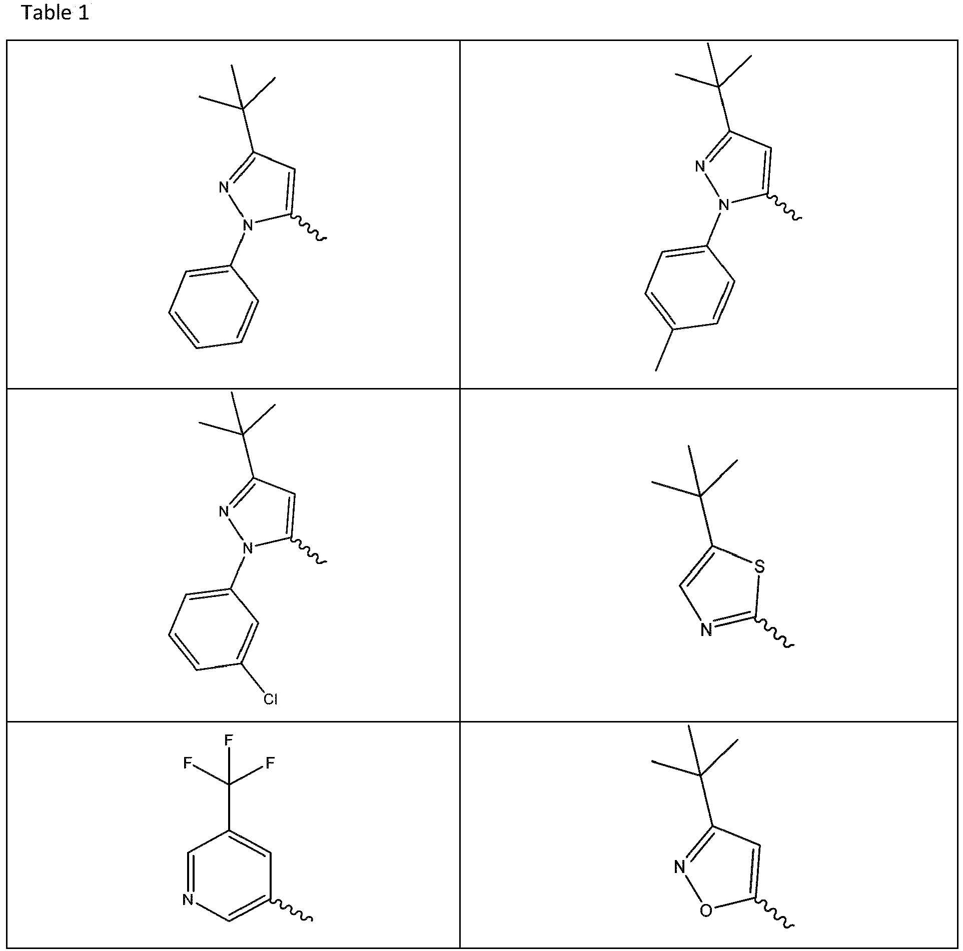

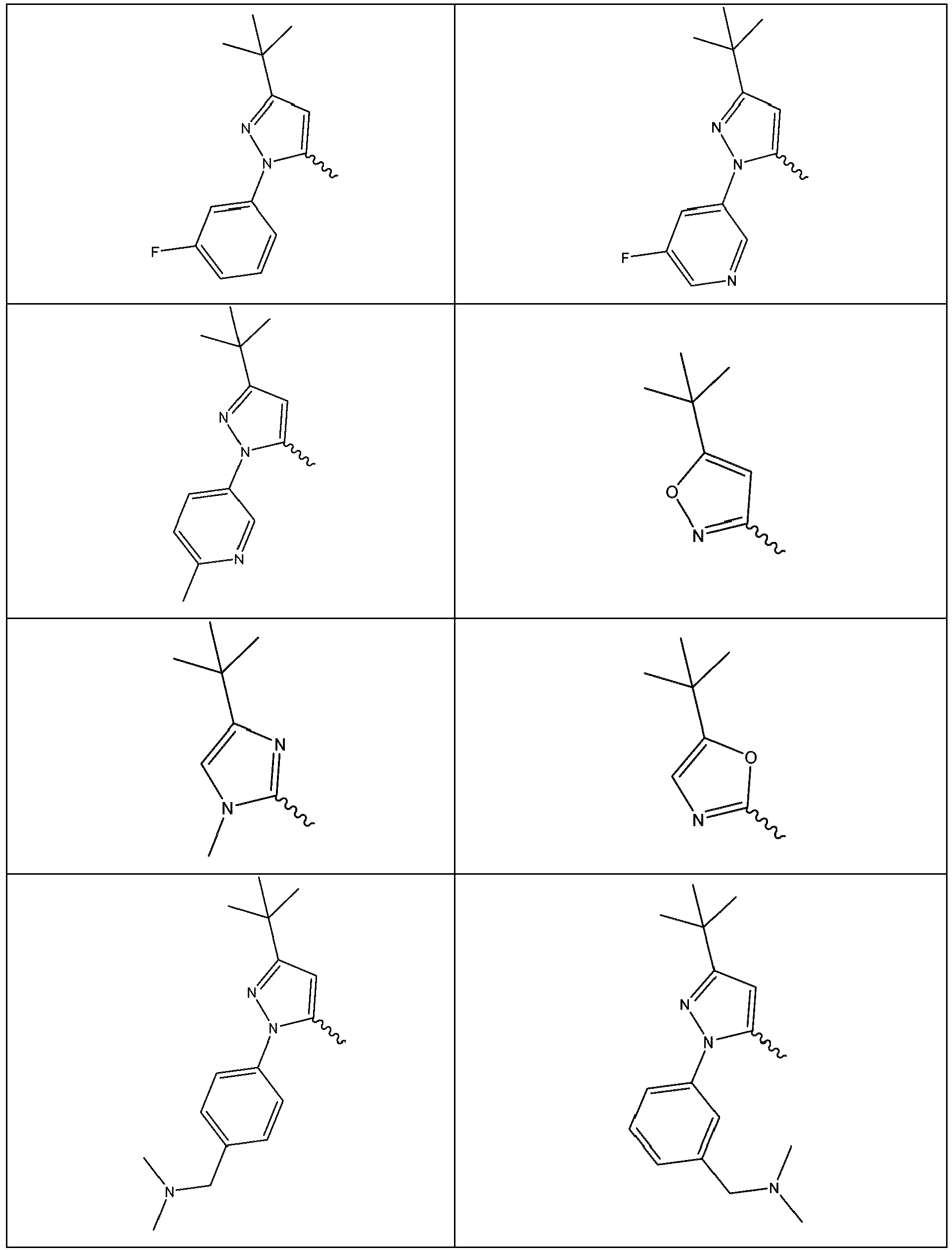

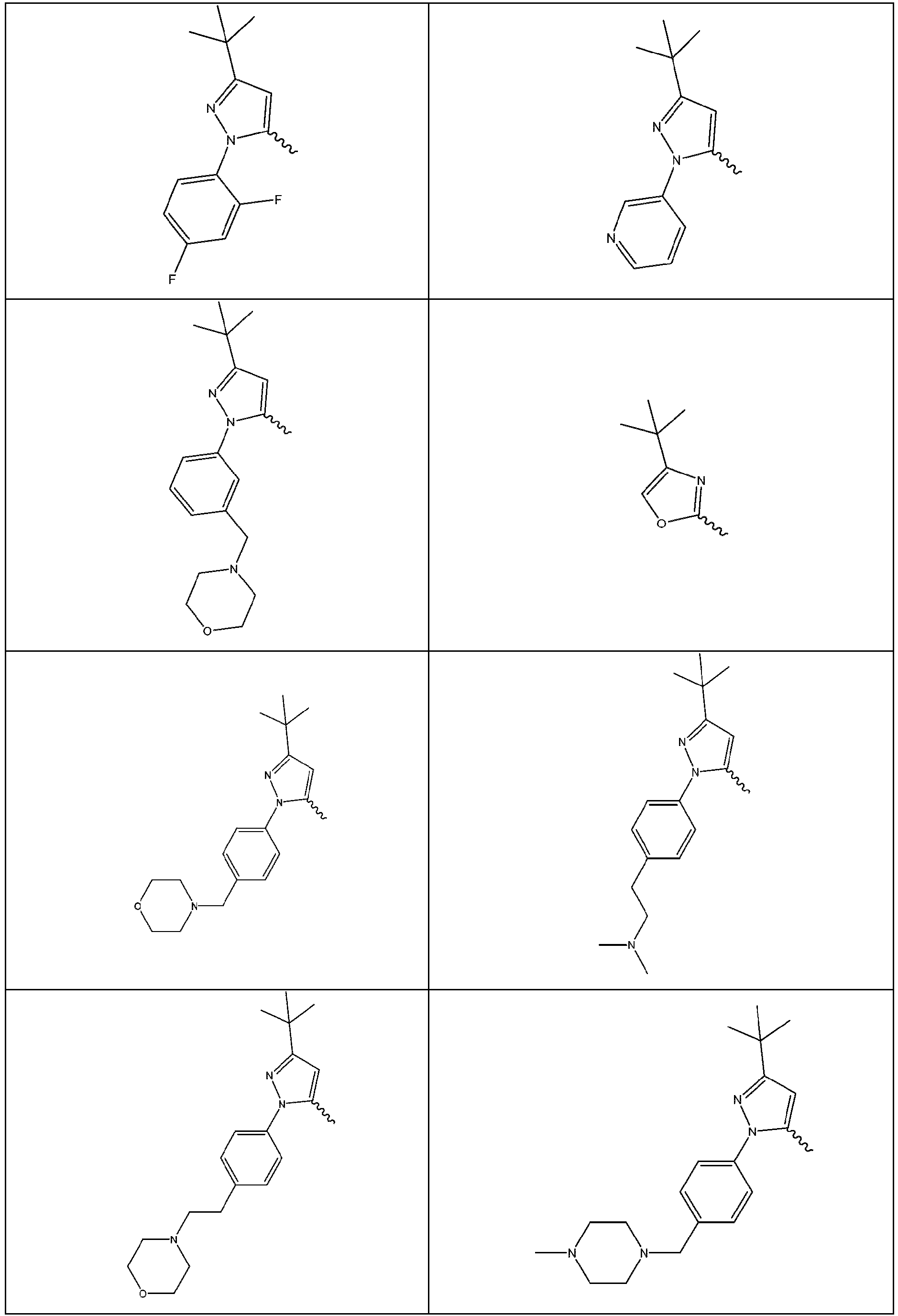

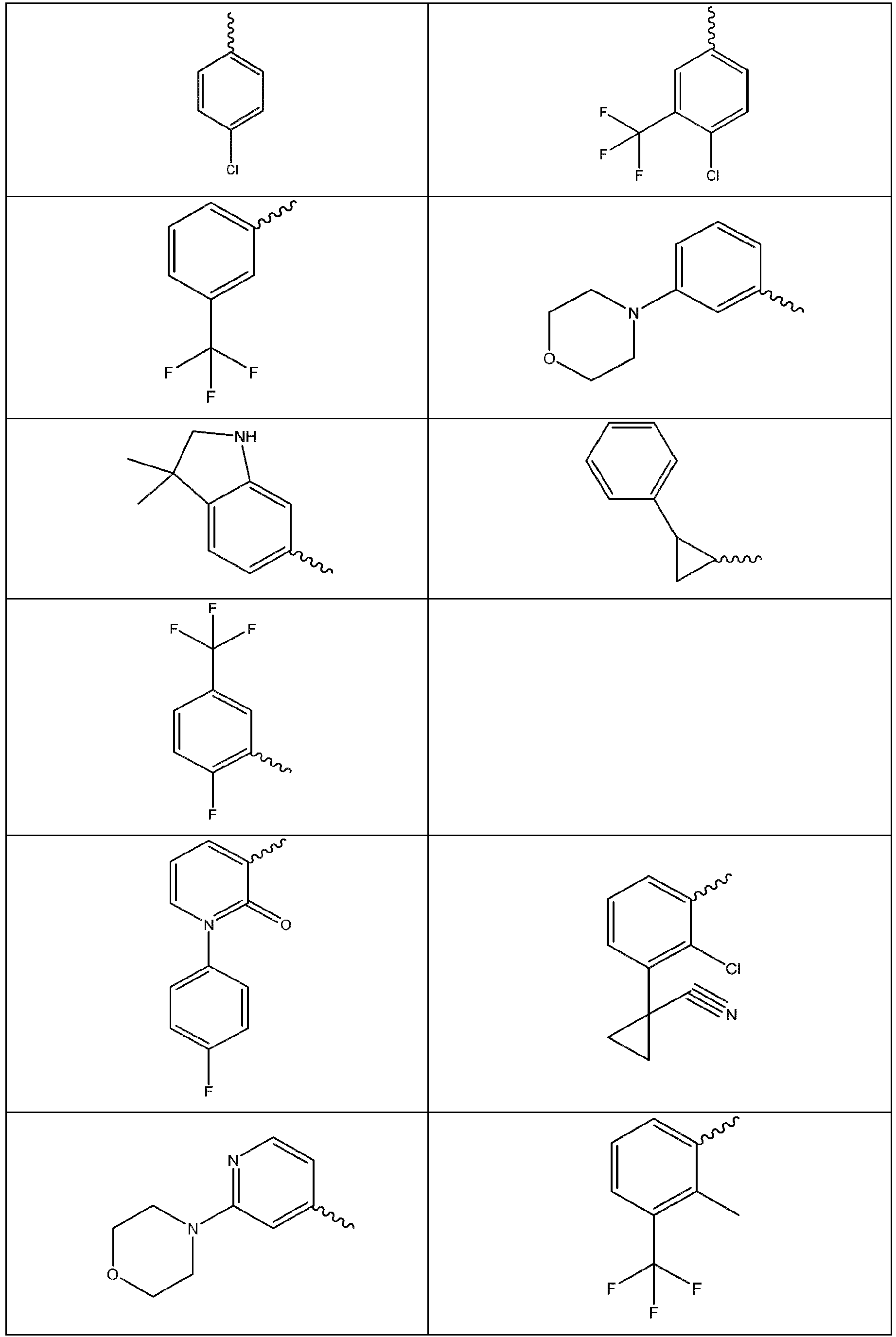

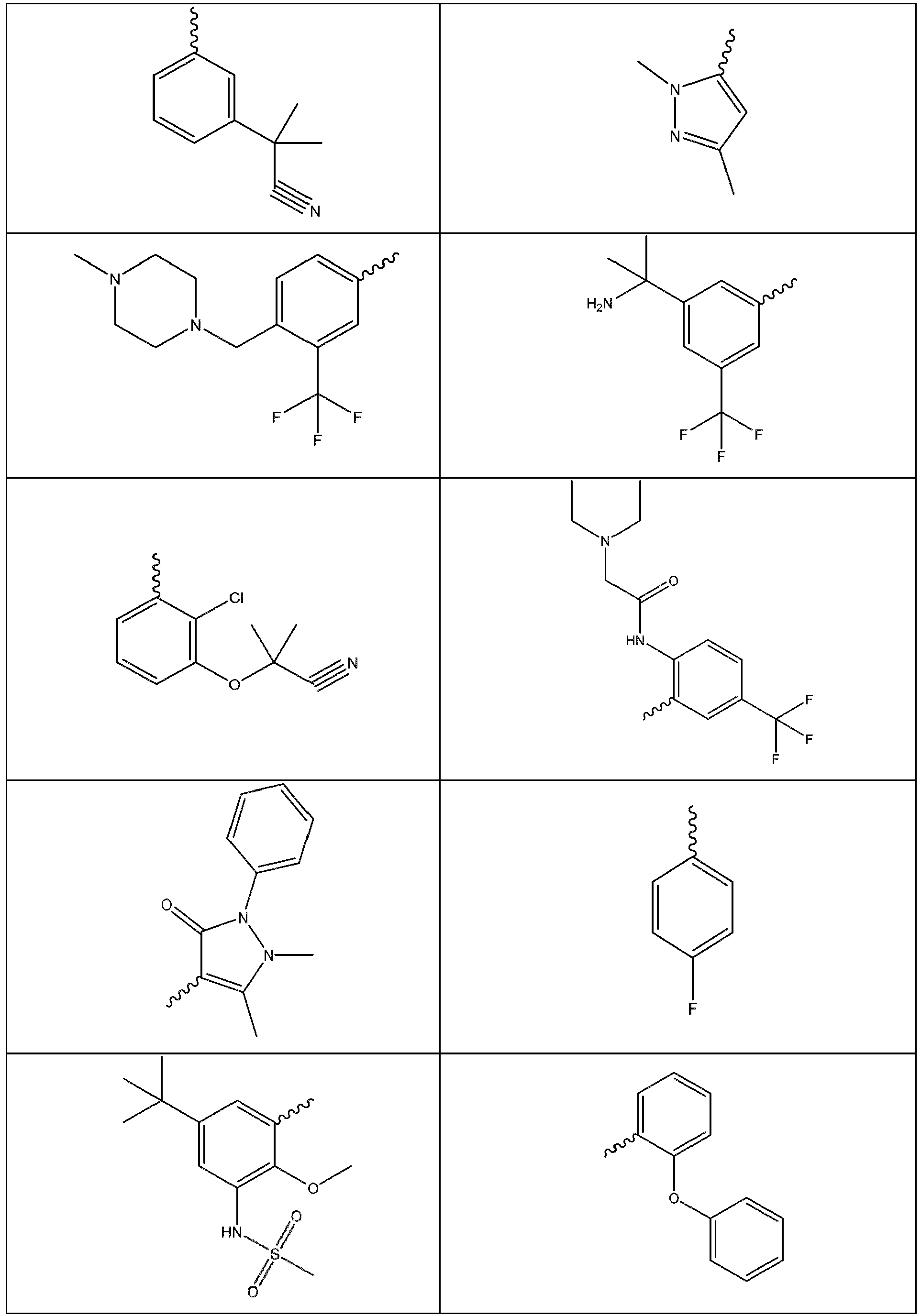

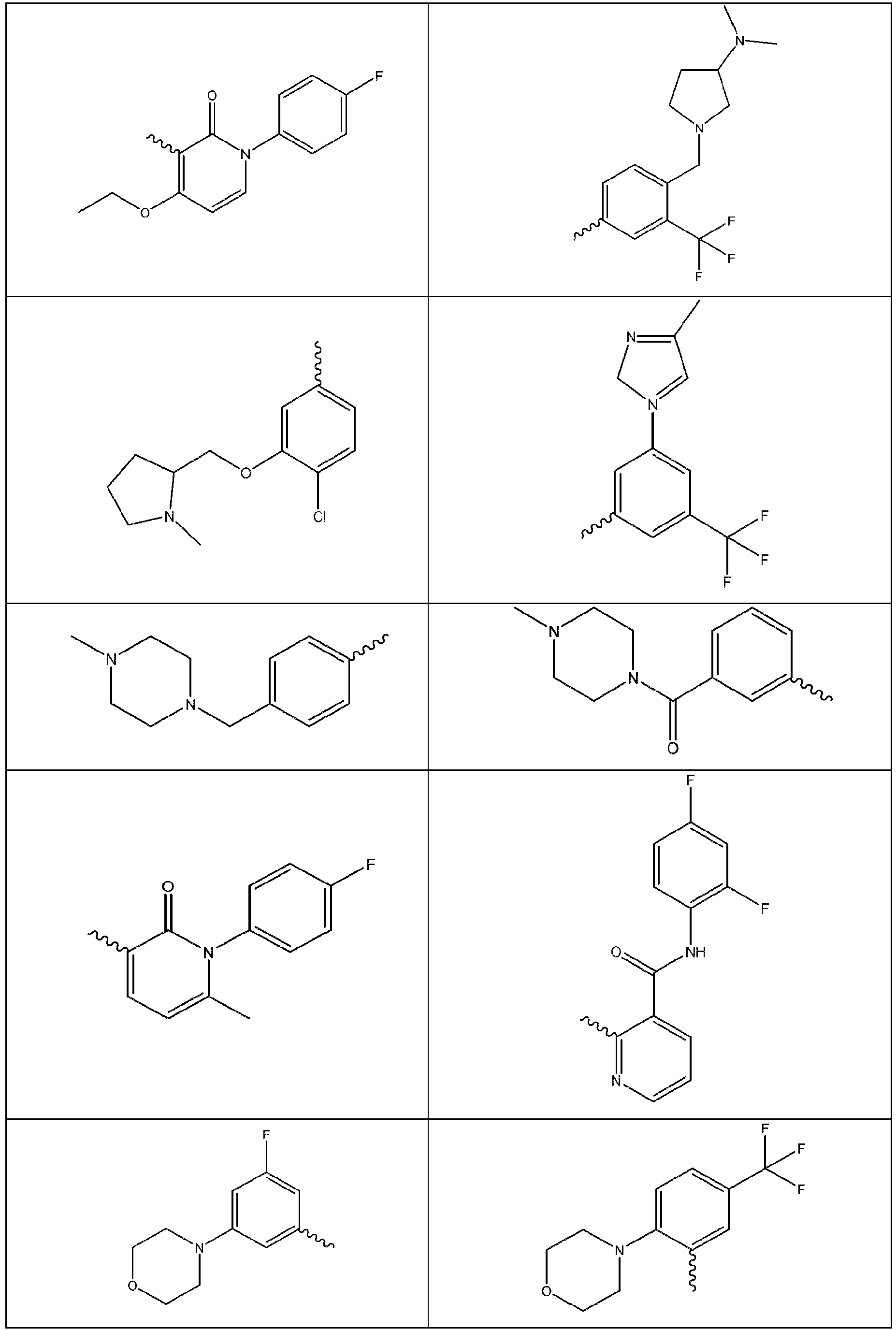

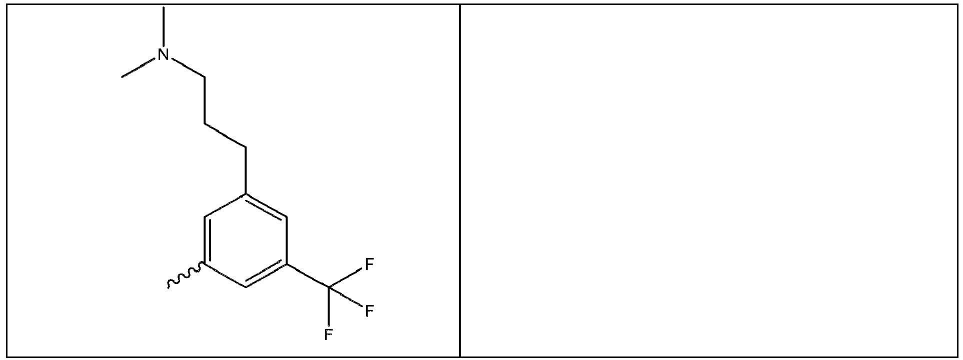

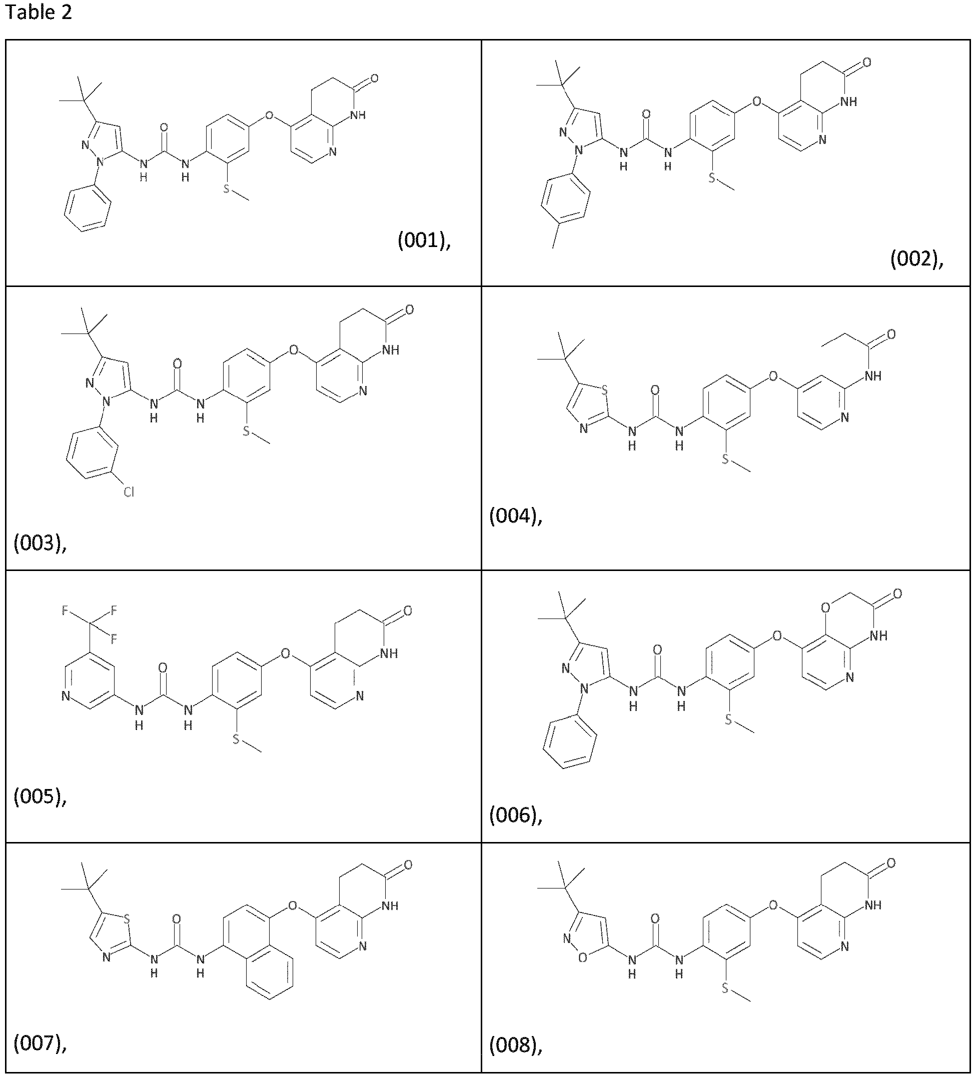

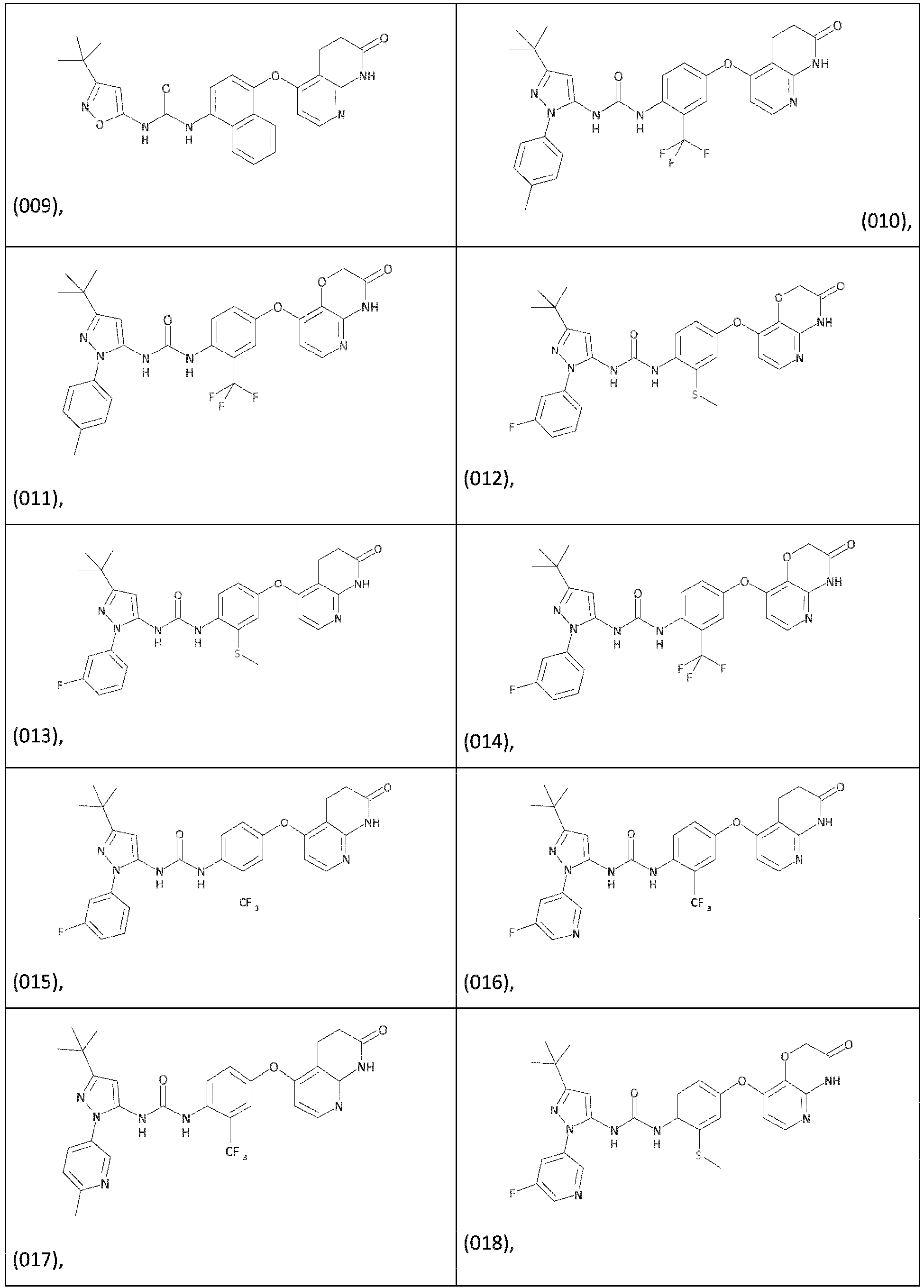

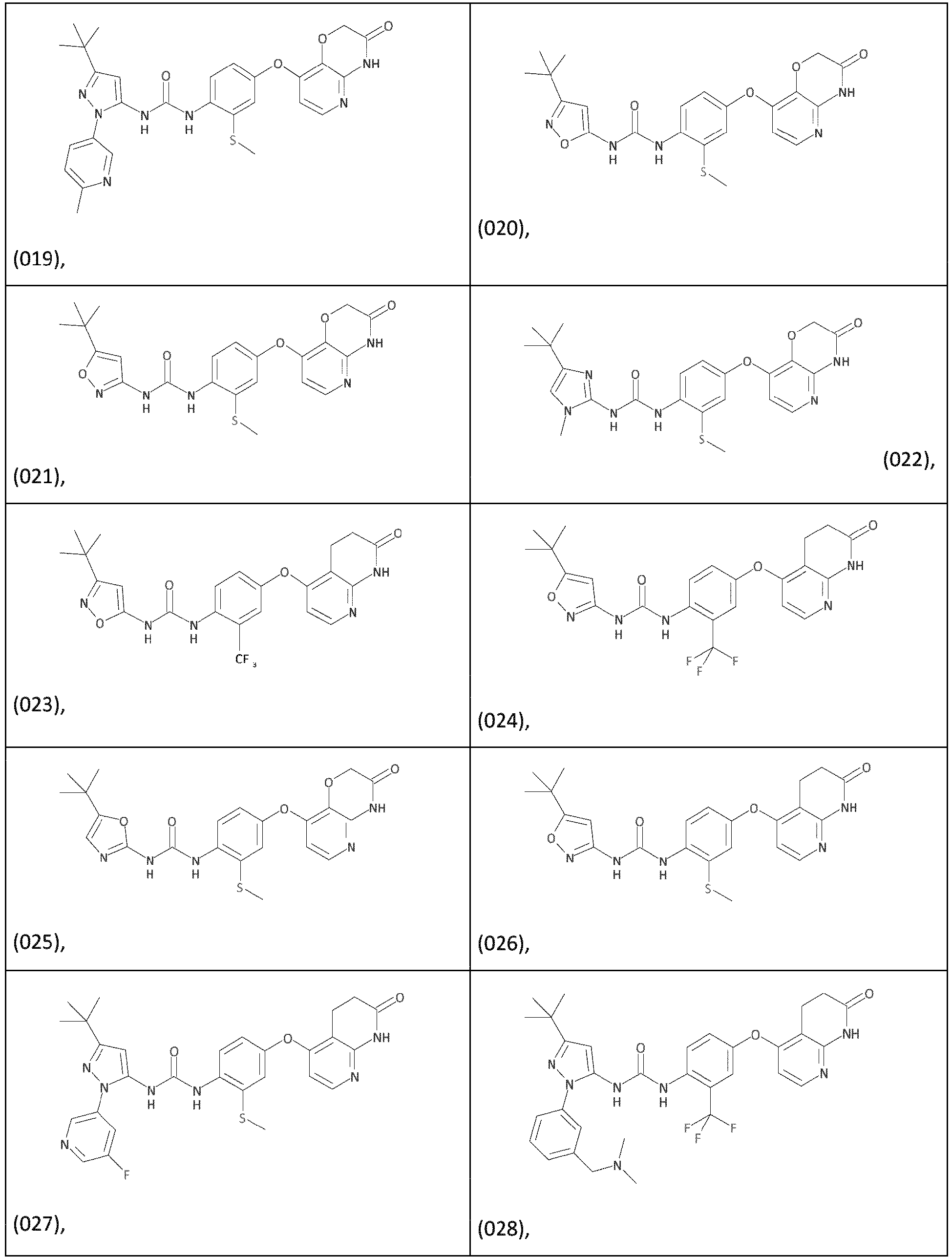

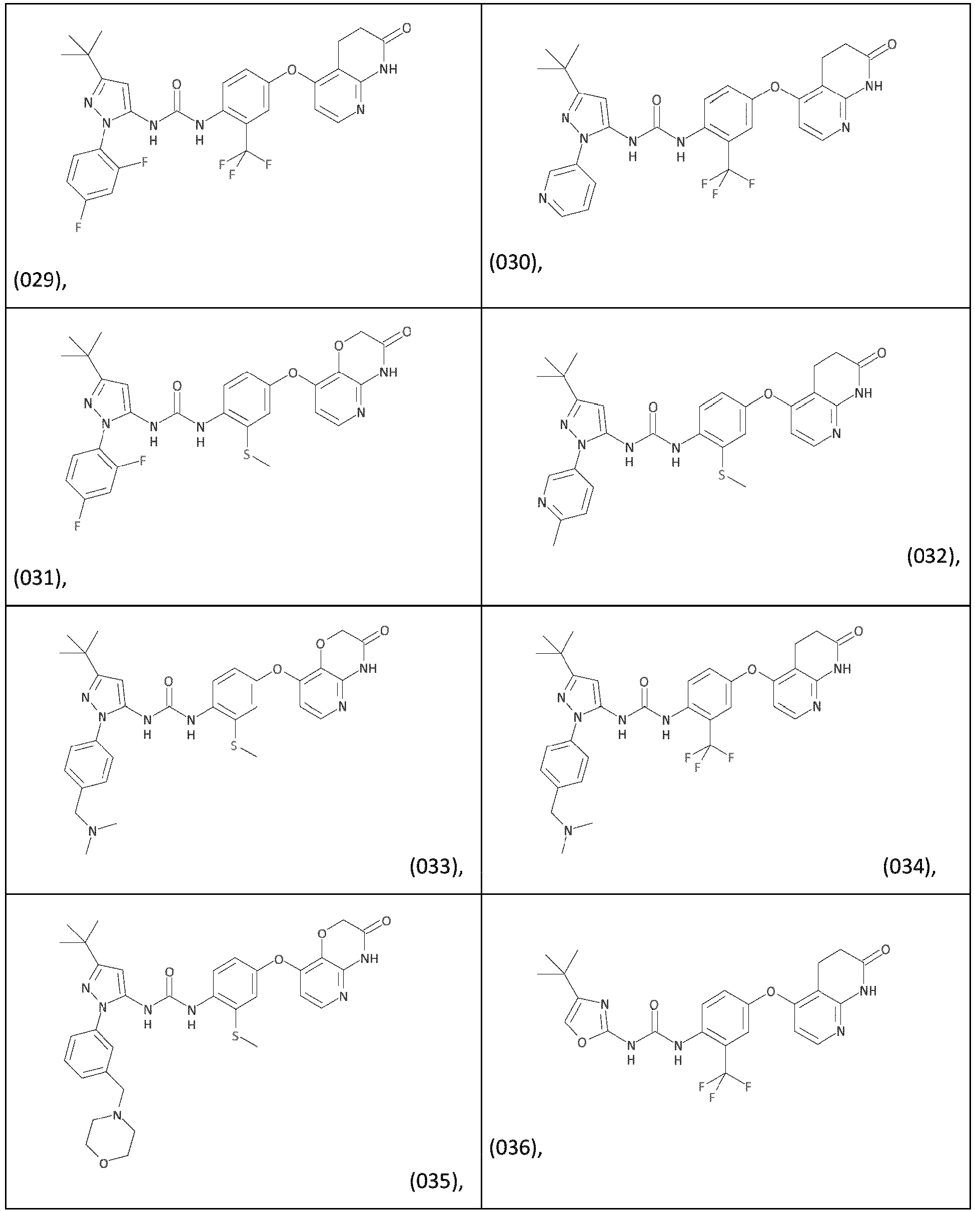

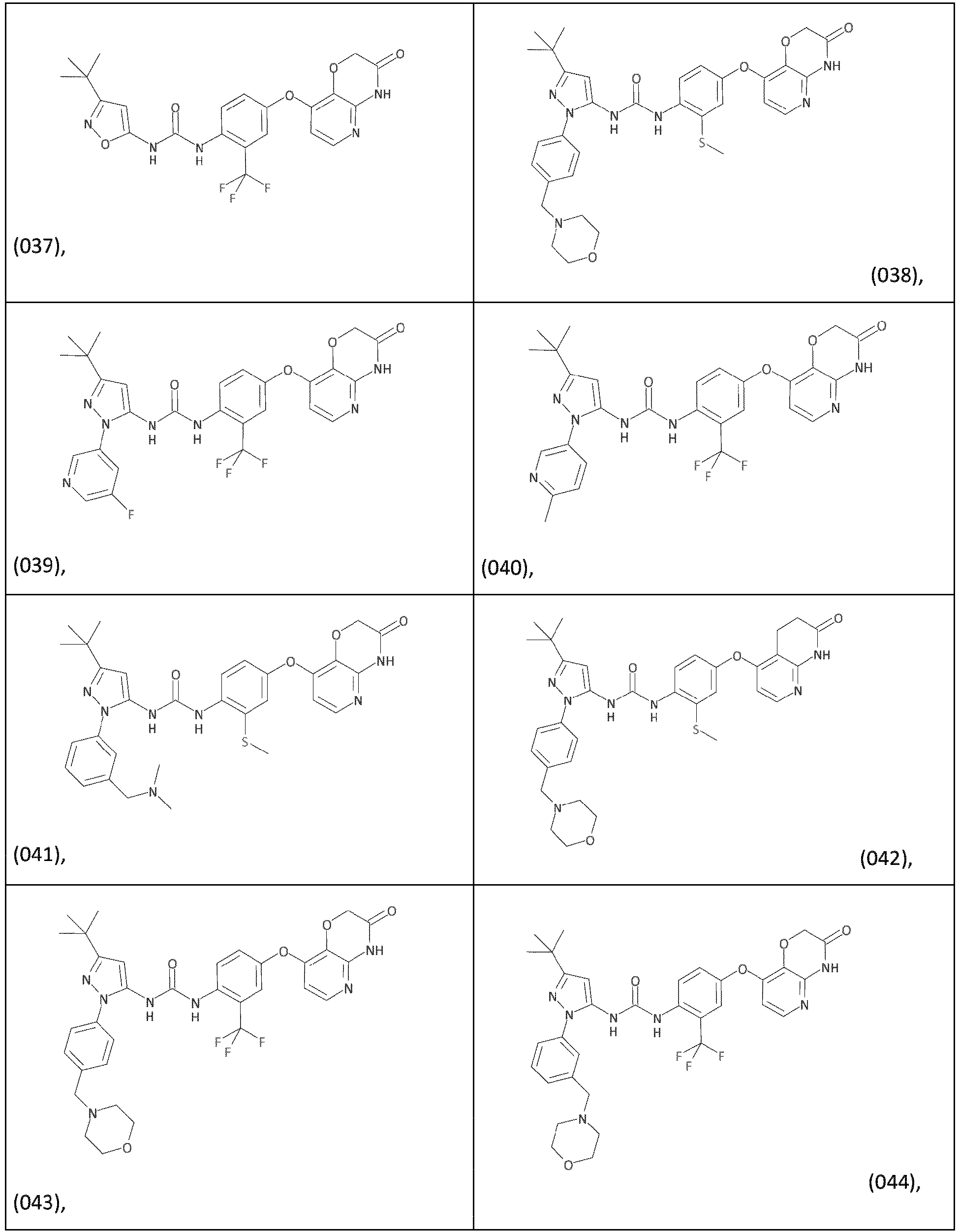

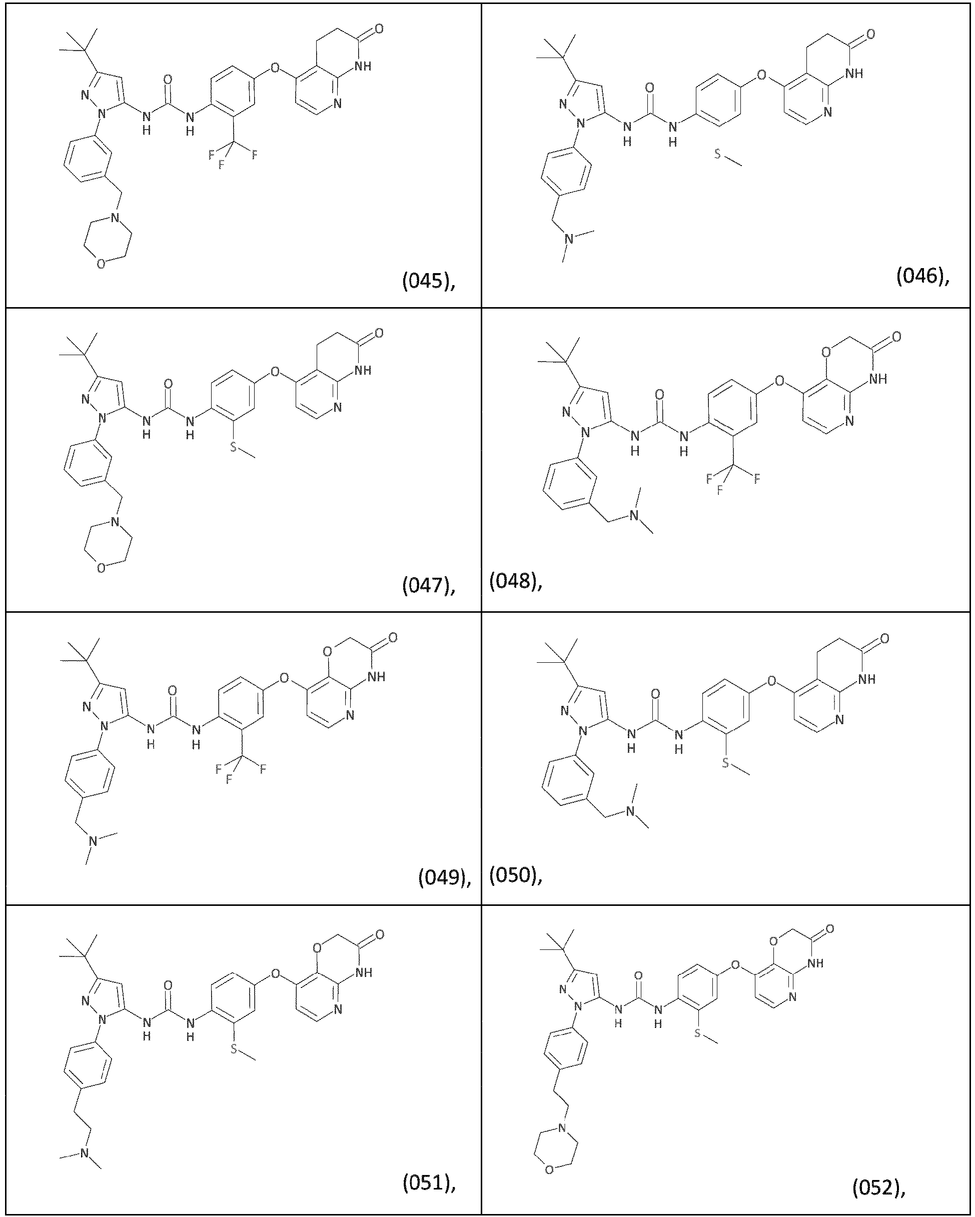

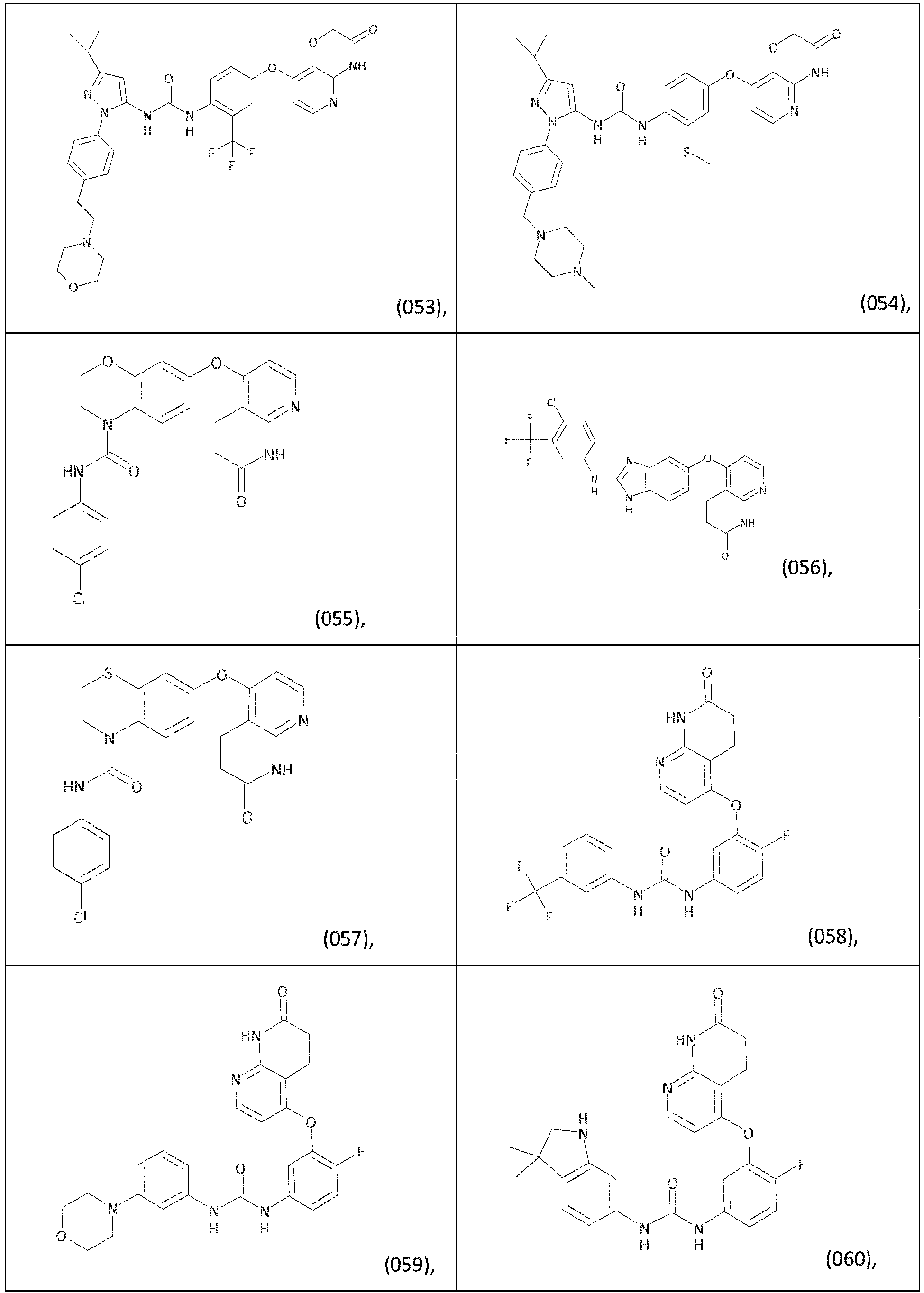

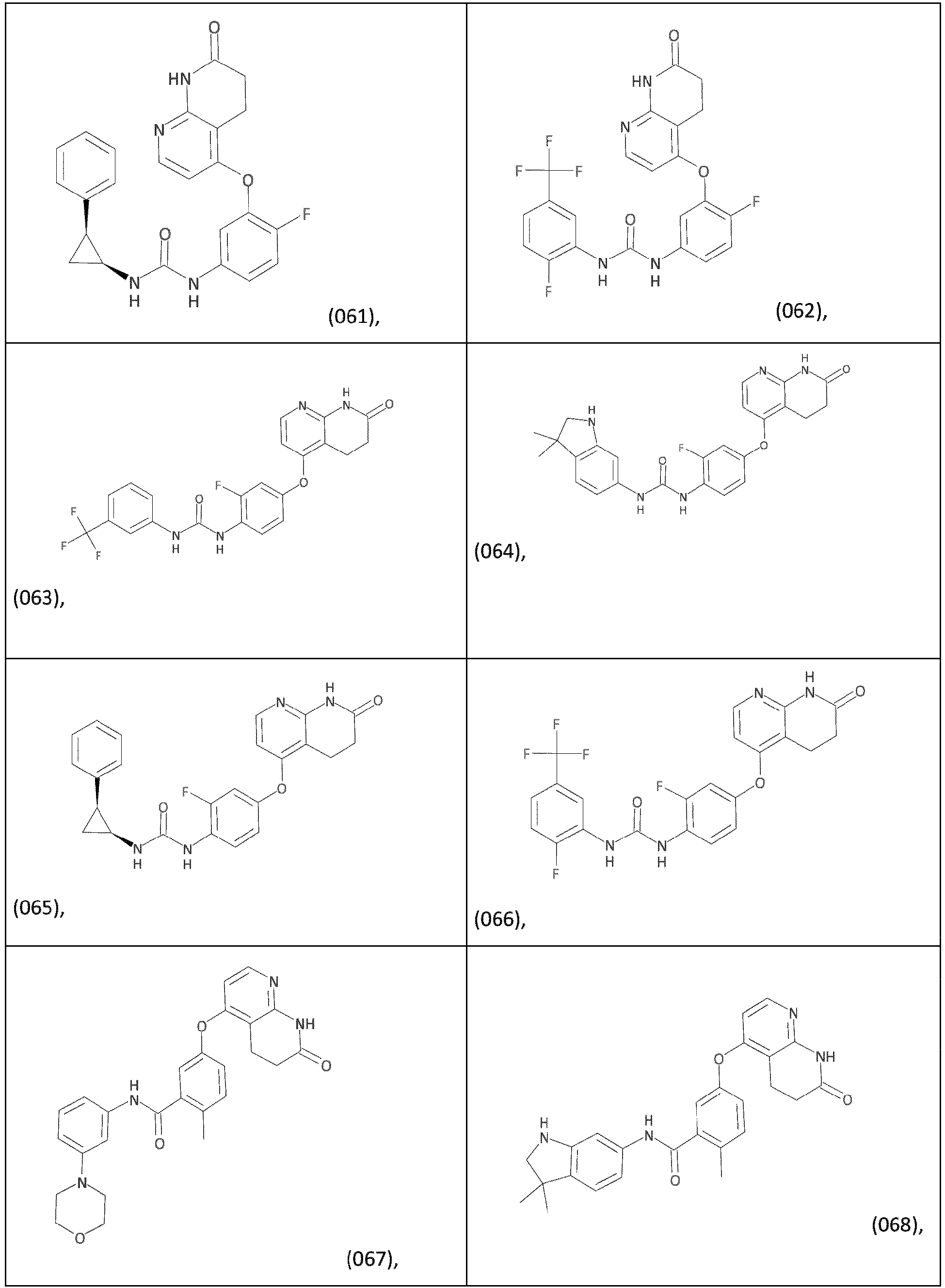

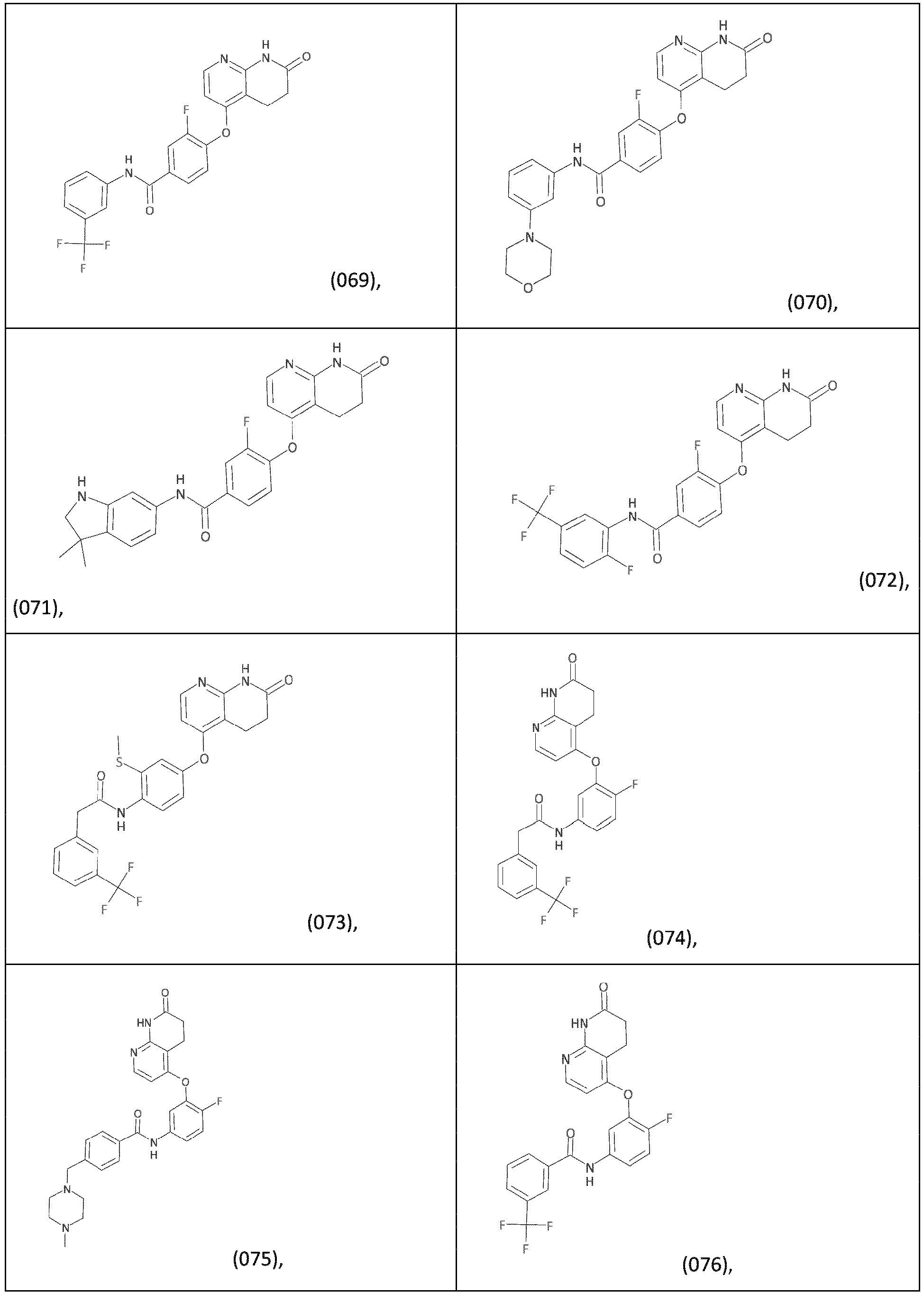

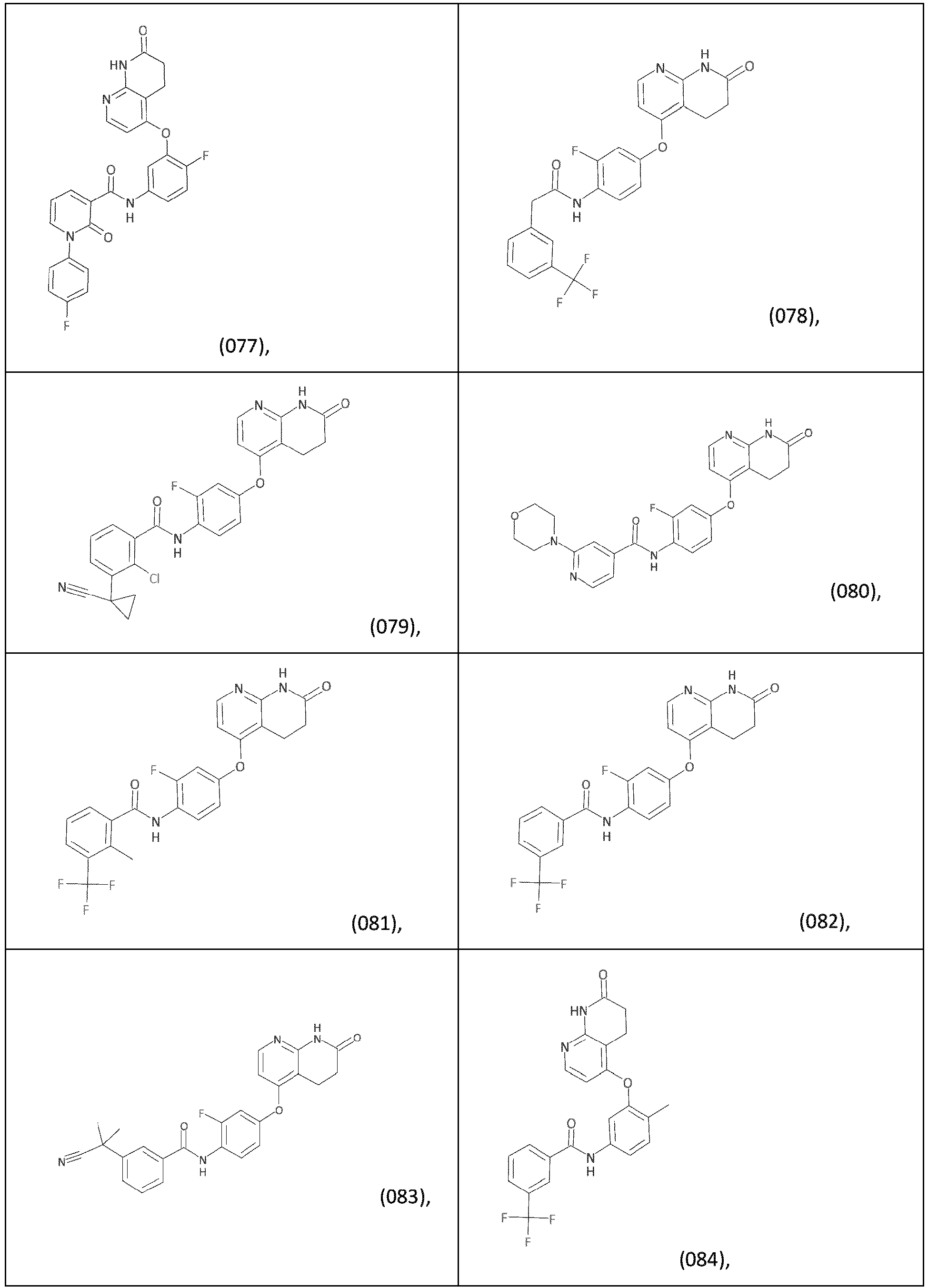

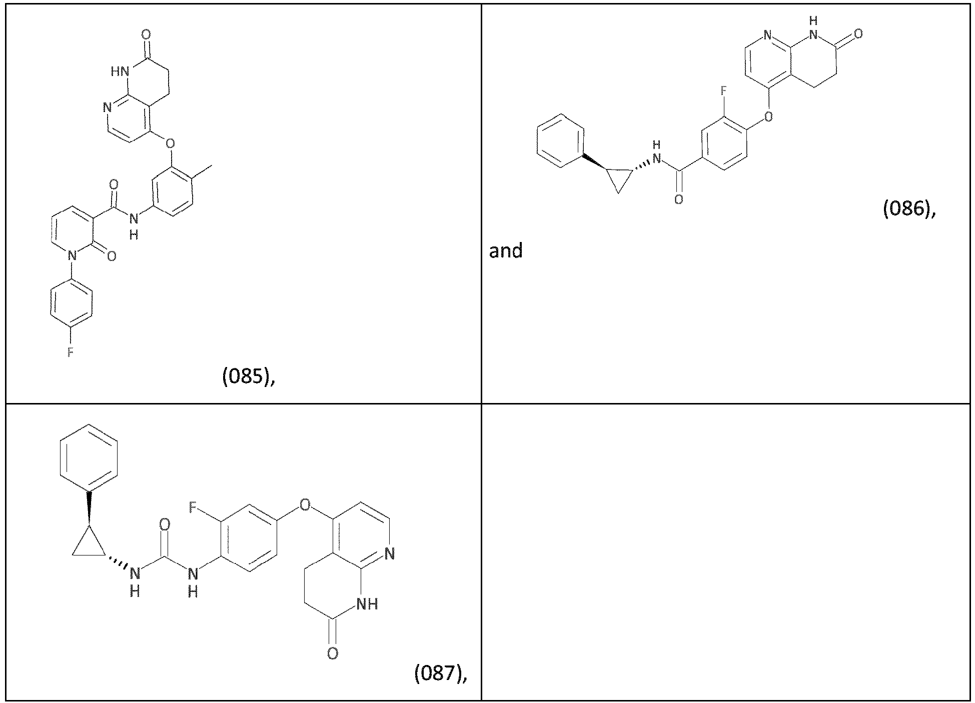

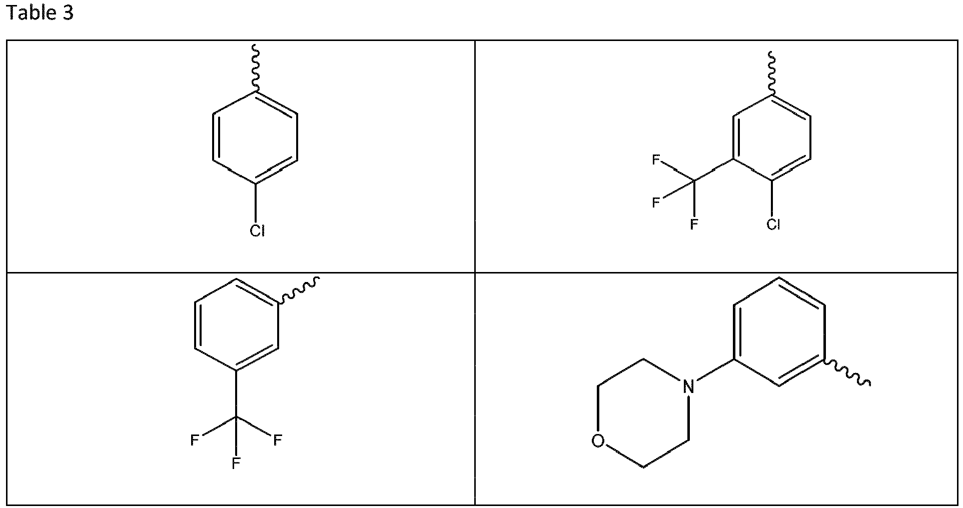

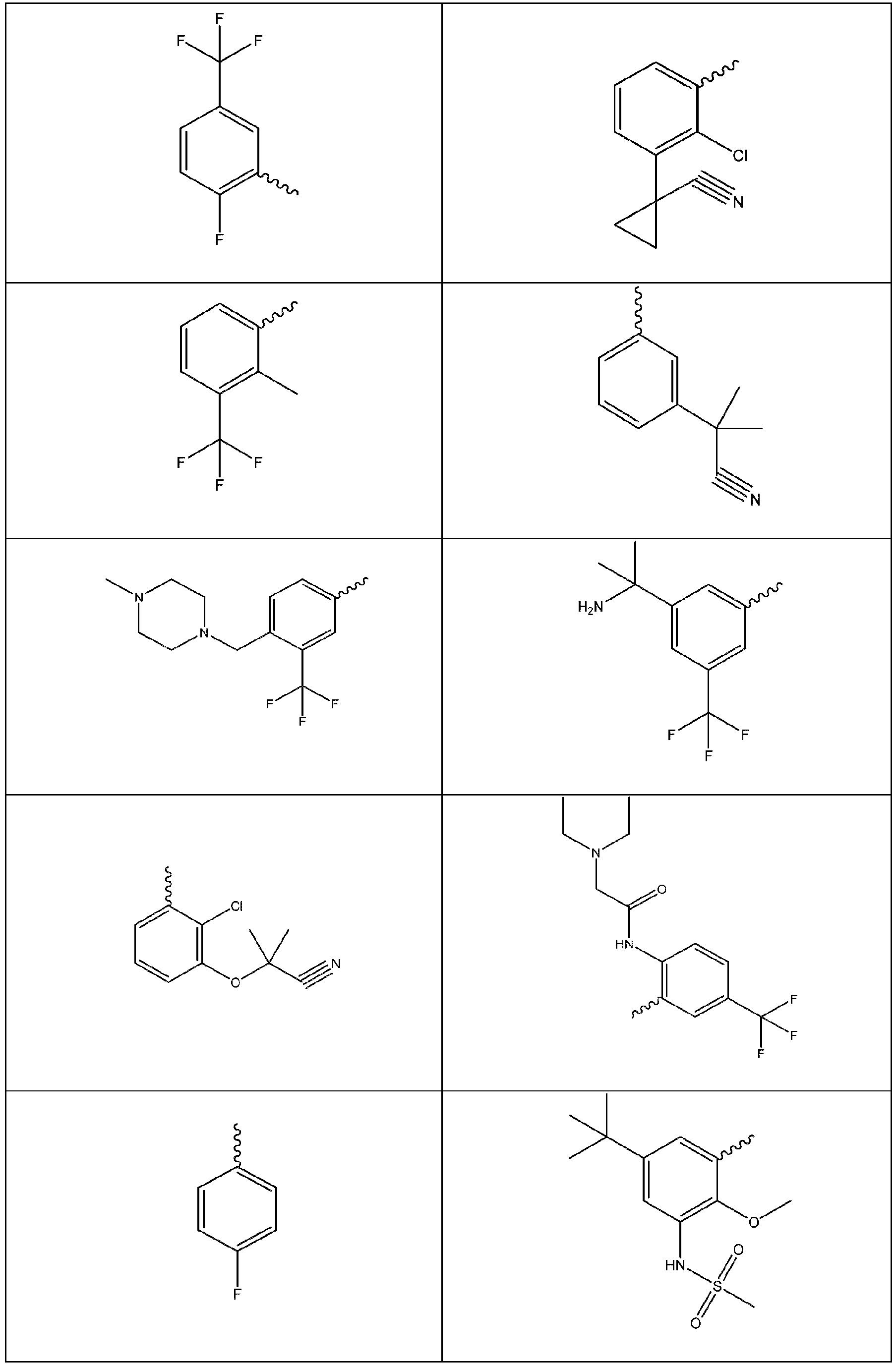

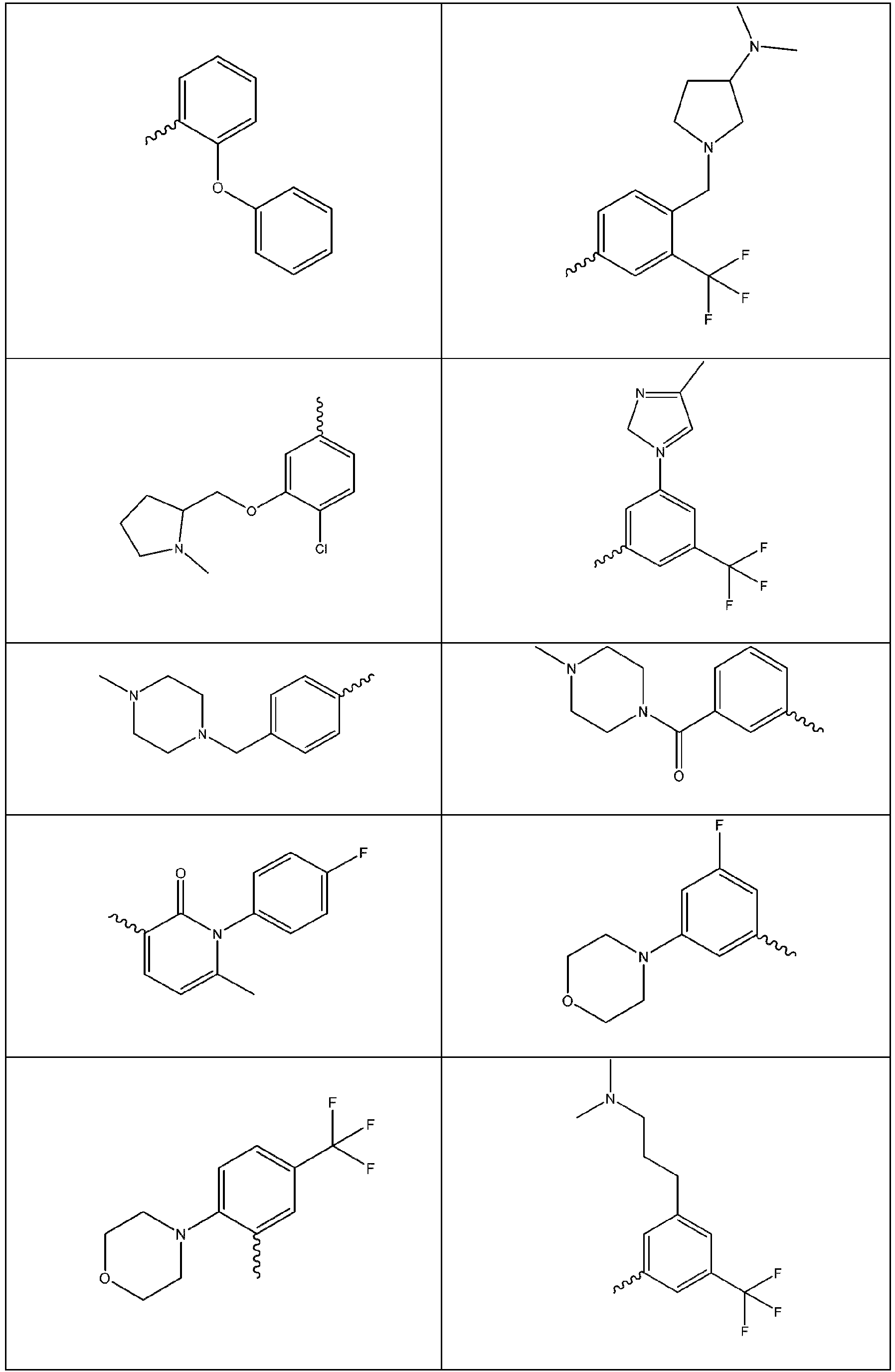

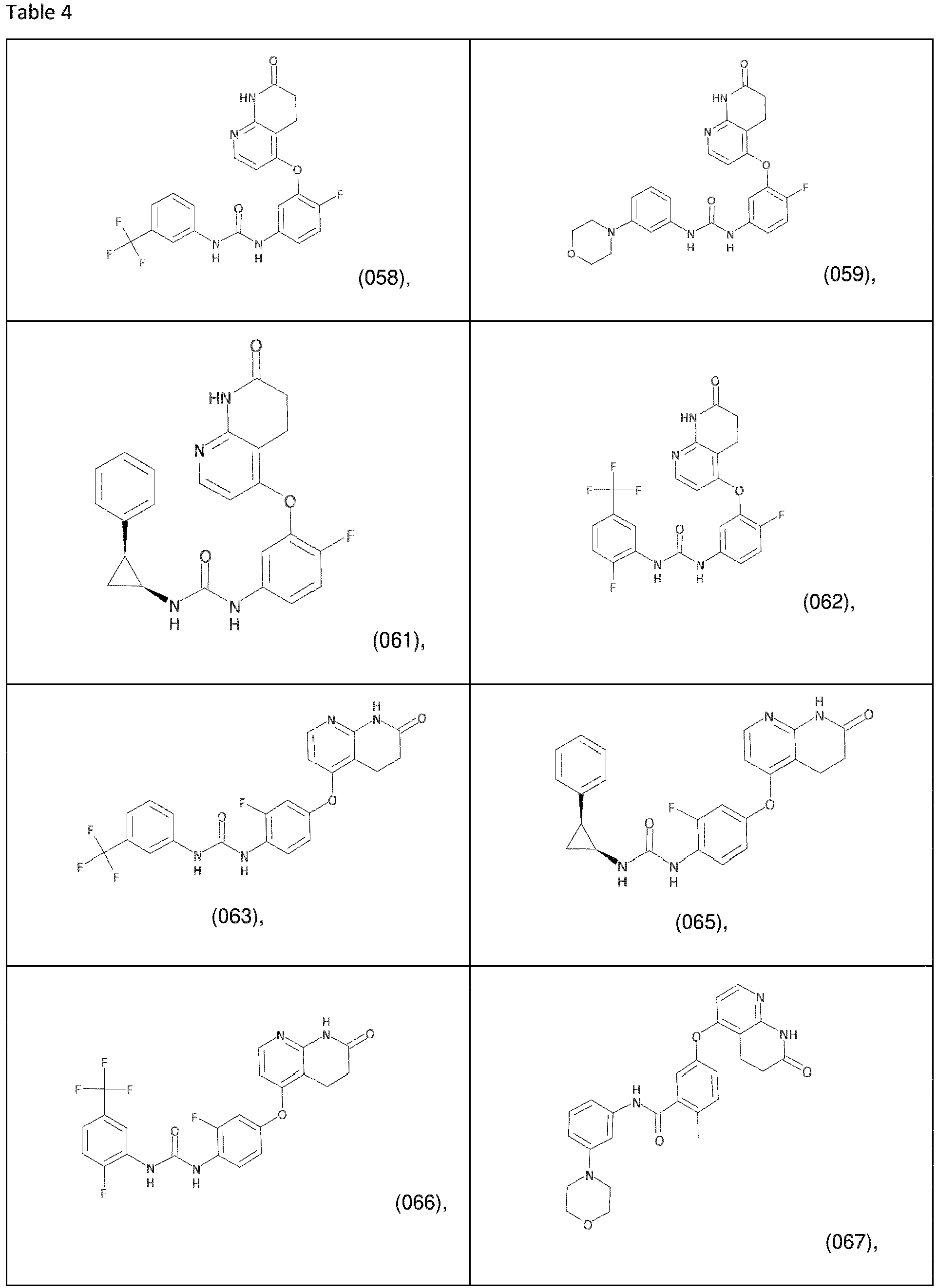

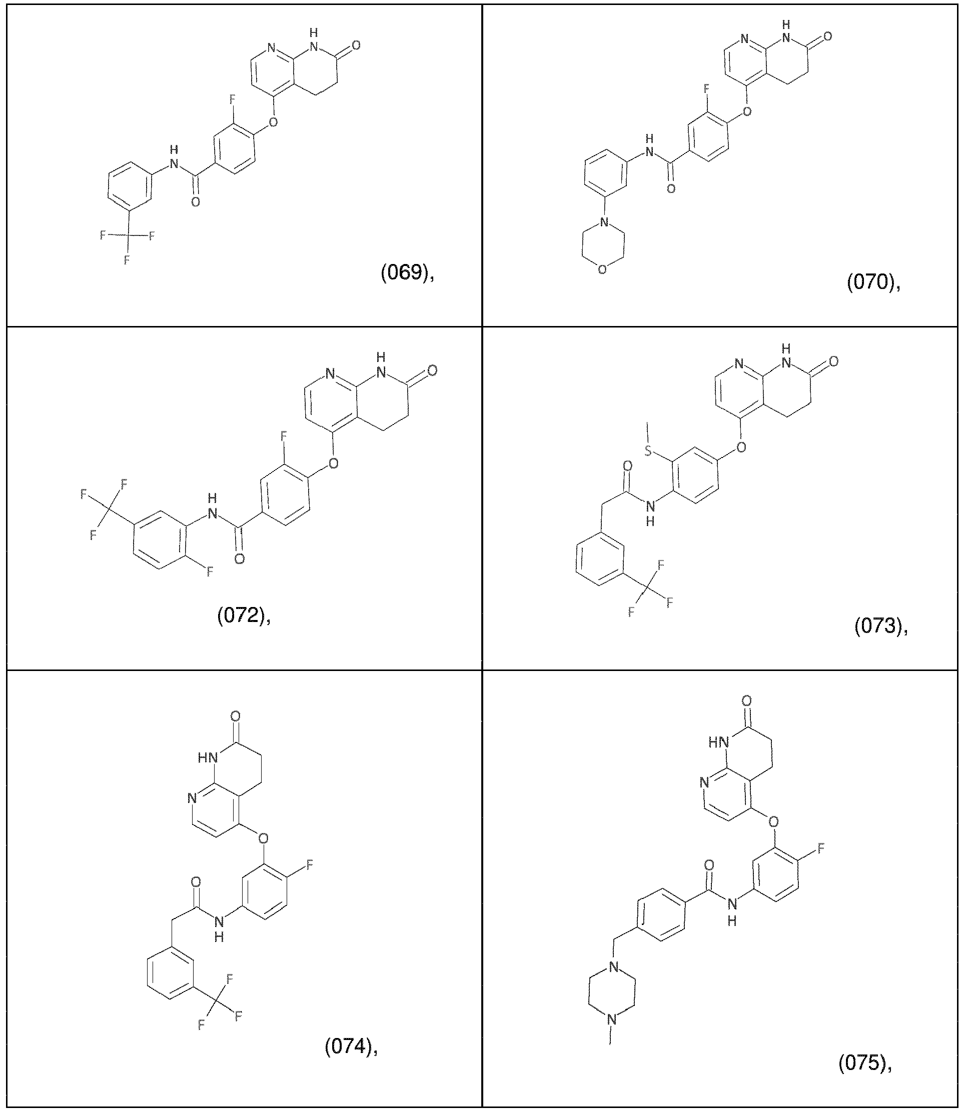

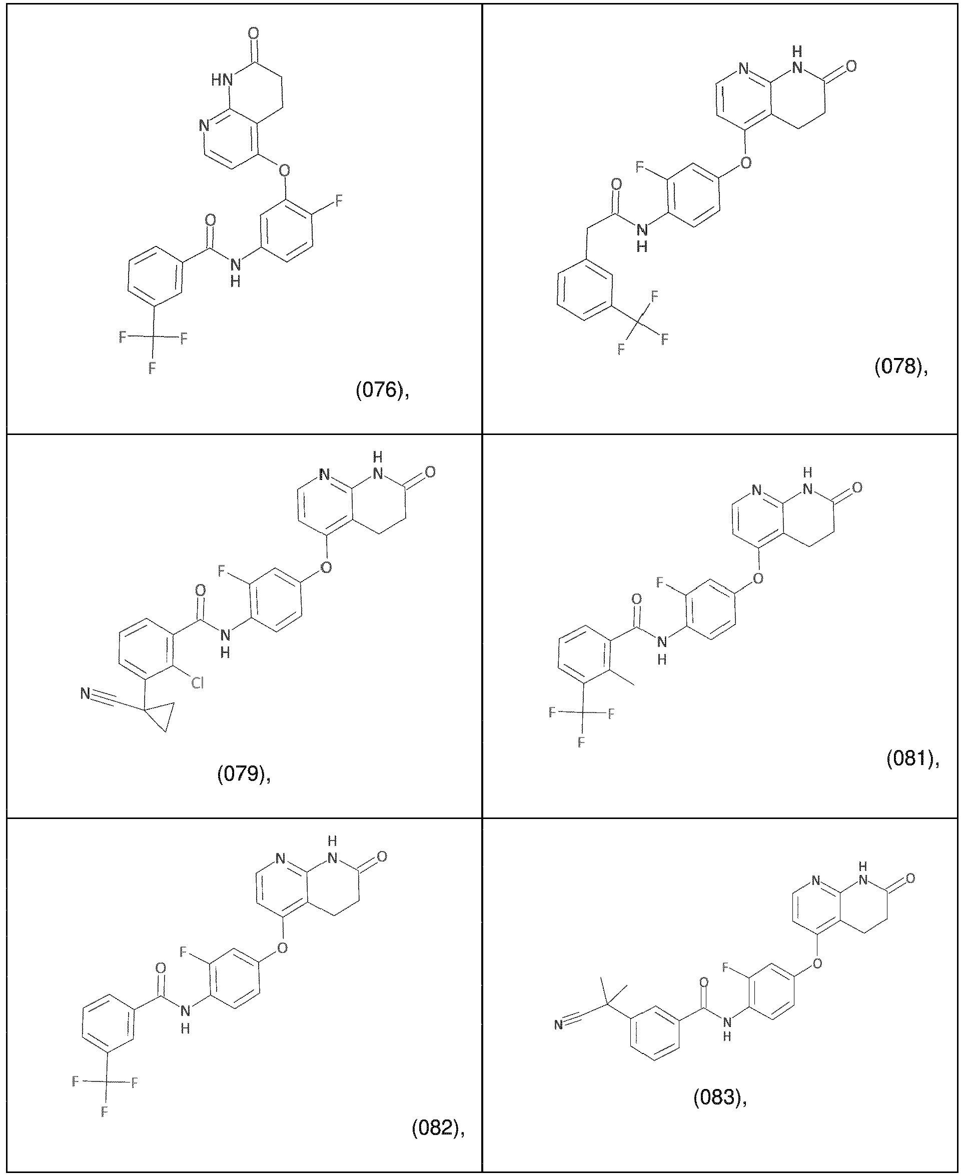

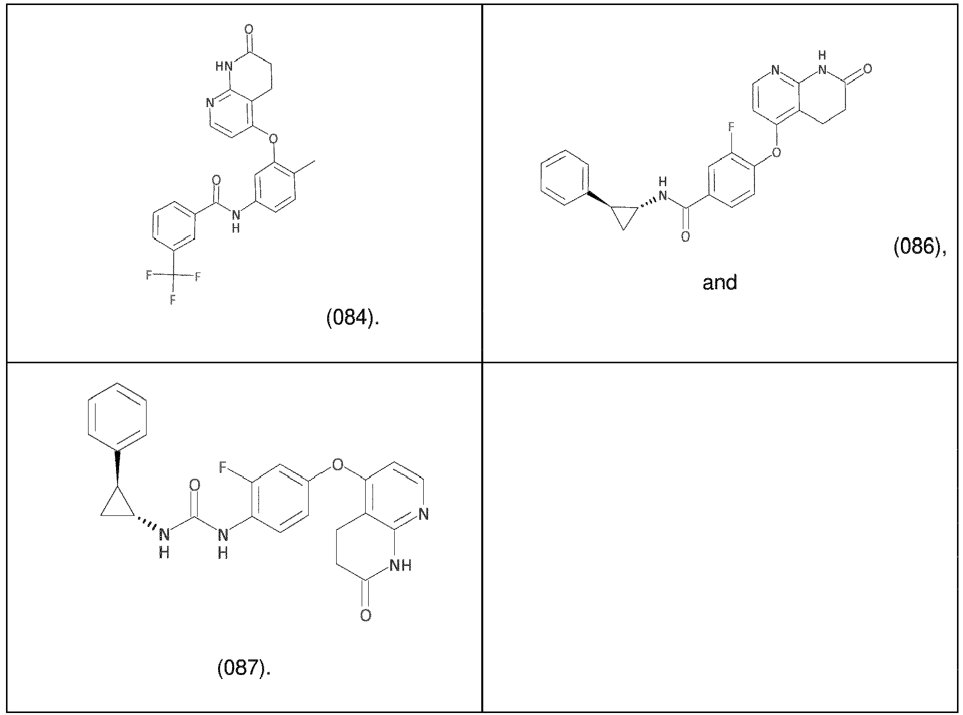

### EXAMPLES

The following examples are offered for illustrative purposes only, and are not intended to limit the scope of the present invention in any way.

### EXAMPLE 1: Synthesis of the compounds

### General synthetic protocol

The reagents and starting materials are commercially available and/or, using well-known techniques, can be readily synthesized by one of ordinary skill in the art. Unless otherwise noted, all commercially starting materials were used without further purification.

The following abbreviation may be used in the examples and throughout the specification: g (grams), mg (milligrams), L (liter), mL (milliliters), µL (microliters), M (molar), % (percentage), MHz (megahertz), mmol (millimoles), min (minutes), AcOEt or EtOAc or EA (ethyl acetate), Et₂O (diethyl ether), % (percent), DCM (dichloromethane), DMSO (dimethyl sulfoxide), DMSO-d6 (deuterated dimethyl sulfoxide), NEt₃ or TEA (triethylamine), TBDMS-Cl (tert-butyldimethylsilyl chloride), NH₄Cl (sat.) (saturated aqueous solution of ammonium chloride) DMF (dimethylformamide), EtOH (ethyl alcohol), DMA (dimethyl acetamide), Pd/C (palladium on carbon), H₂ (hydrogen gas), NaN₃ (sodium azide), DIPEA (diisopropyl ethyl amine), NaH (sodium hydride), Na₂CO₃ (sodium carbonate), NaHCO₃ (sodium hydrogenocarbonate), MeONa (sodium methoxide), CBr₄ (carbon tetrabromide), PPh₃ (triphenylphosphine), CO₂ (carbon dioxide), DBU (1,8-Diazabicyclo[5.4.0]undec-7-ene), TFA (trifluoroacetic acid), MeCN or CH₃CN (acetonitrile), K₂CO₃ (potassium carbonate), NMP (1-methyl-2-pyrrolidinone), CHCl₃ (chloroform), Cul (copper iodide), t-BuOK (potassium tert-butoxide), Cs₂CO₃ (cesium carbonate), CuCN (copper cyanide), i-AmONO (isoamyl nitrite), SnCl₂.2H₂O (stannous chloride dihydrate), Ac₂O (acetic anhydride), N₂H₄.H₂O (hydrazine monohydrate), PE (petroleum ether), NEt₃.3HF (triethylamine trifluoride), p-TSA (p-toluenesulfonic acid), NH₃ (ammoniac), Me₂CO (acetone), SOCl₂ (thionyl chloride), DCE (dichloroethane), DMAP (N,N-dimethylaminopyridine), (Boc)₂O (Di-tert-butyl dicarbonate), HATU (hexafluorophosphate de (diméthylamino)-N,N-diméthyl(3H-[1,2,3]triazolo[4,5-b]pyridin-3-yloxy)méthaniminium), EDCl.HCl (1-éthyl-3-(3-diméthylaminopropyl)carbodiimide hydrochloride), HOBt (1-hydroxybenzotriazole), T3P (Propanephosphonic acid anhydride), DCC (dicyclohexylcarbodiimide), °C (Celsius degrees), MeOH (methanol), Hz (Hertz), TLC (Thin Layer Chromatohraphy), LCMS (Liquid Chromatography Mass Spectrum), MS (Mass Spectrum), ES (Electrospray), HPLC (High Pressure Liquid Chromatography), NMR (Nuclear Magnetic Reasonance), 1H (proton), MgSO₄ (magnesium sulphate), SiO₂ (silica gel) R.T. (Room Temperature), KOH (potassium hydroxide), NaOH (sodium hydroxide), h (hour), HCI (hydrochloric acid), HCl conc (37%v/v hydrochloric acid), THF (tetrahydrofuran), N₂ (nitrogen), eq (equivalent), mm (millimeters), s (singulet), d (doublet), t (triplet), m (multiplet), q (quintuplet), bs (broad singulet), dd (doublet of doublet), dt (doublet of triplet), td (triplet of doublet), dq (doublet of quintuplet).

NMR spectra were registered on a 300 MHz or 400 MHz Bruker DPX and processed using Bruker XWinNMR software. All commercially obtained reagents were used without further purification.

All references to brine refer to a saturated aqueous solution of NaCl. Unless otherwise indicated, all temperatures are expressed in °C. All reactions are not conducted under an inert atmosphere at room temperature unless otherwise noted.

### Synthesis of compounds according to the invention

Compounds represented by structure (I) or (II) may be prepared by methods known in the art of organic synthesis as set forth in part by the following synthesis schemes. In all of the schemes described below, it is well understood that protecting groups for sensitive or reactive groups are employed where necessary in accordance with general principles of chemistry. Protecting groups are manipulated according to standard methods of organic synthesis (T.W. Green and P.G.M. Wuts (1991) Protecting Groups in Organic Synthesis, John Wiley et Sons). These groups are removed at a convenient stage of the compound synthesis using methods that are readily apparent to those skilled in the art. The selection of process as well as the reaction conditions and order of their execution shall be consistent with the preparation of compounds of structure (I) or (II).

The compound of structure (I) or (II) may be represented as a mixture of enantiomers, which may be resolved
into the individual pure R- or S-enantiomers. If for instance, a particular enantiomer of the compound of structure (I) or (II) is desired, it may be prepared by asymmetric synthesis, or by derivation with a chiral auxiliary, where the resulting diastereomeric mixture is separated and the auxiliary group cleaved to provide the pure desired enantiomers. Alternatively, where the molecule contains a basic functional group such as amino, or an acidic functional group such as carboxyl, this resolution may be conveniently performed by fractional crystallization from various solvents, of the salts of the compounds of structure (I) or (II) with optical active acid or by other methods known in the literature, e.g. chiral column chromatography. Resolution of the final product, an intermediate or a starting material may be performed by any suitable method known in the art as described by E.L. Eliel, S.H. Wilen and L.N. Mander (1984) Stereochemistry of Organic Compounds, Wiley-Interscience. All the products generated from the following reactions can be isolated and purified employing standard techniques, such as extraction, chromatography (silica gel flash chromatography, reverse phase flash chromatography, preparative TLC, preparative HPLC), crystallization, distillation, and the like.

General routes to prepare the Examples of the present invention are shown in the Schemes and examples that follow. All substituents in the synthetic Schemes unless otherwise indicated, are as previously defined. The compounds of structure (I) or (II) may be prepared by general route of synthesis as disclosed in the following methods

The compounds of structure (I) or (II) wherein Ar, A, Y, X1, X2 and R1 are as described above, may be prepared following methods described below.

### Method A

Compounds of Formula (I) and (II) wherein X1 and X2 are -NH- (compound F) can be prepared using scheme 1 and the procedure described in Kono, Mitsunori & al, Bioorganic & Medicinal Chemistry, 2013, 21(1), 28-41. To achieve this procedure **C1** is activated with trichloroethylchloroformiate to give the intermediate carbamate **D1** that reacts with the amine E to get the final urea compound F (WO2018132372).

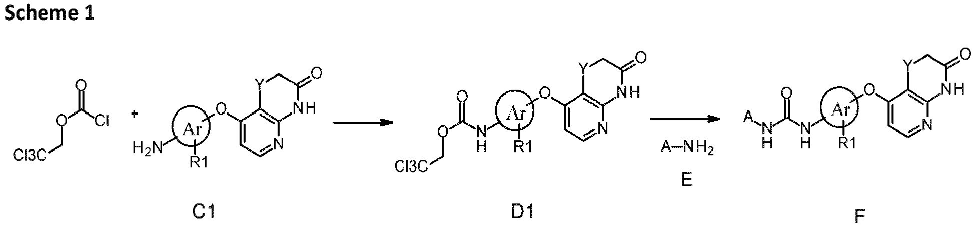

### Method B

Alternatively to method A, Compound of Formula (I) or (II) wherein X1 and X2 are -NH- (compound F) can be prepared using method scheme 2 with the same conditions than in method A but inversing the order of reactions. Amine E is transformed in activated carbamate G that reacts with compound of formula C1 to afford directly compound F.

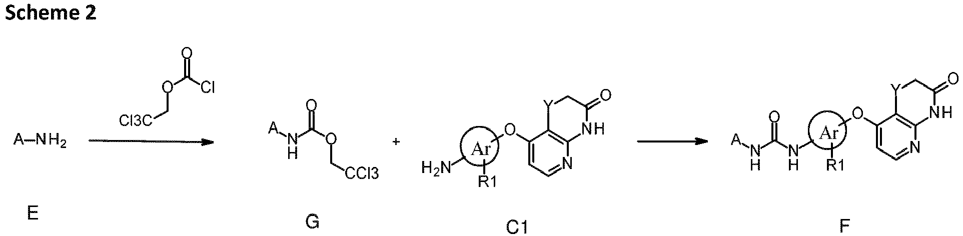

### Method C

Compound of formula (I) wherein X1 is -CH₂- or absent and X2 is -NH-, or compounds of formula (I-2) and (I-3)(compound J) may be prepared following scheme 3 using usual amide bond coupling reaction. The reaction may be carried out via activation of Acid H as acyl chloride with thionyl chloride or oxalyl chloride, following by coupling with the amine C1 in the presence of suitable base and solvent. The suitable base for the reaction may be triethylamine, N,N--di isopropylethylamine, pyridine or DMAP and solvent may be selected from, DMF, THF, chloroform, dichloromethane or 1,4-dioxane. Alternatively, the coupling reaction may be carried out in the presence of suitable coupling agent such as HATU, EDCI.HCI with or without HOBt, T3P or DCC. The reaction may be carried out in suitable solvent selected from DMF, THF, dichloromethane, dichloroethane, chloroform, 1,4-dioxane or a mixture thereof.

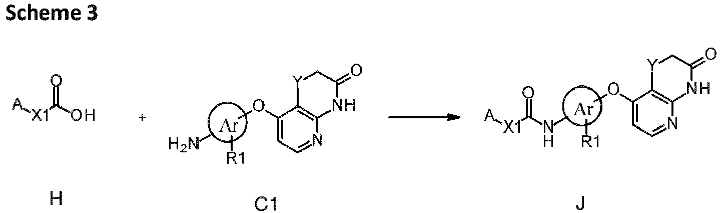

### Method D

Compound of formula (I) wherein X1 is -NH- and X2 is absent (compound K) may be prepared following scheme 4 using the same amide bond coupling reaction condition than in method C. The reaction may be carried out via activation of Acid **C2** as acyl chloride with thionyl chloride or oxalyl chloride, following by coupling with the amine E in the presence of suitable base and solvent. The suitable base for the reaction may be triethylamine, *N,N*--di isopropylethylamine, pyridine or DMAP and solvent may be selected from DMF, THF, chloroform, dichloromethane or 1,4-dioxane. Alternatively, the coupling reaction may be carried out in the presence of suitable coupling agent such as HATU, EDCl.HCl with or without HOBt, T3P or DCC. The reaction may be carried out in suitable solvent selected from DMF, THF, dichloromethane, dichloroethane, chloroform, 1,4-dioxane or a mixture thereof.

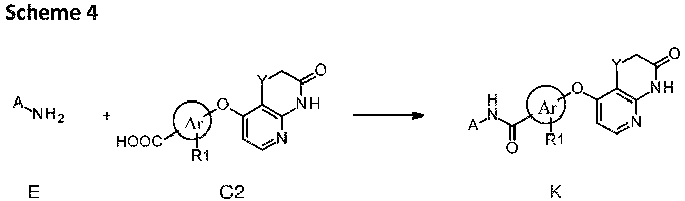

### Method E

Compound of formula C wherein Ar, Y and R1 are as describe above, may be prepared following scheme 5: Hydroxyaryl of formula A is activated using a suitable organic or inorganic base such as tBuOK, potassium or cesium carbonate in appropriate solvent such as DMSO, DMF or DMA and reacts generally at elevate temperature with a compound of formula **B** where W is an halogen such as F, Cl or Br (WO US20050245530; Menard et al., J. Med. Chem. 2009, 52 (8), 2255-2264; WO 2021/013712; WO2000042012).

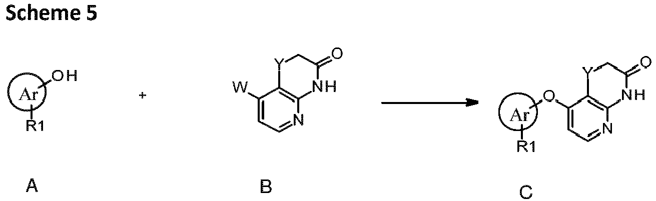

### Synthesis of Key intermediates

### Synthesis of 8-bromo-4-[(4-methoxyphenyl)methyl]pyrido[3,2-b][1,4]oxazin-3-one (4)

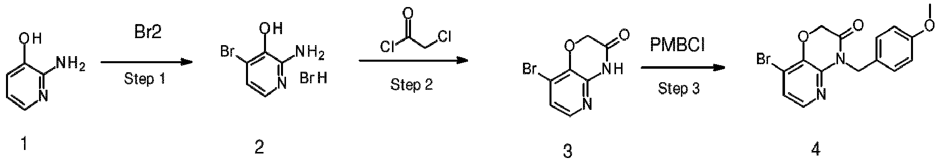

### Step 1. Synthesis of compound (2)

To a solution of 2-amino-2-hydroxypyridine (1) (10.0g, 91mmol) in absolute ethanol (50mL) at 0°C was dropwise added bromine (5.2ml, 102mmol) and the mixture was stirred at room temperature for 3 days. The solvent was removed under reduced pressure and low temperature. The residue was cooled to 0°C. AcOEt (100mL) was added and the mixture was stirred for 1h. The suspension was filtered off (washing with AcOEt). The red-brown solid recovered was dried under reduced pressure leading to the expected product (2) (Yield: 74%, 18.2g).

### Step 2. Synthesis of compound (3)

In a sealed tube at 0°C, sodium bicarbonate (2.3g, 27.9mmol) was added to a solution of 2-amino-4-bromo-pyridin-3-ol (2) (2.5g, 9.3mmol) in a mixture of 2-butanone and water (1:1, 18.6mL). After 10min, chloroacetyl chloride (0.74mL, 9.3mmol) was added. The mixture was stirred for 3h at 0°C. Then, the mixture was heated at 80°C for 18h. After cooling and filtration of the suspension, the dark-brown solid recovered was dried under vacuum leading to the expected product (Yield: 62%, 1.34g).

### Step 3. Synthesis of compound (4)

To a solution of 8-bromo-4H-pyrido[3,2-b][1,4]oxazin-3-one (3) (1.1g, 4.9mmol) in DMF (25mL) were added cesium carbonate (3.2g, 9.9mmol) followed by 4-methoxybenzyl chloride (1.2g, 7.4mmol) and the mixture was stirred for 18h at room temperature. After filtration, the solvent was removed in vacuo. The residue was solubilized in AcOEt (100mL) and washed with water (50mL) and NH₄Cl sat. (50mL). The organic fraction was dried over MgSO₄, filtered and concentrated in vacuo. The crude obtained was purified by flash chromatography (cyclohexane/AcOEt 100:0→40:60) leading to the expected product (4) (Yield: 78%, 1.35g) as a white solid.

### Synthesis of 4-amino-3-methylsulfanyl-phenol (5)

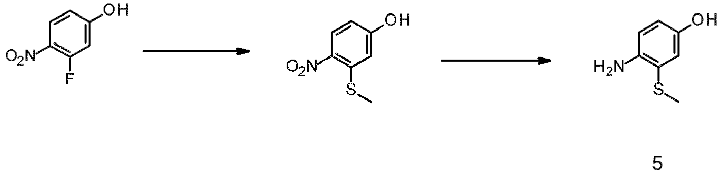

Step 1. To a solution of 3-fluoro-4-nitrophenol (5.6g, 35.7mmol) in 200mL of dry DMF were added portionwise sodium thiomethoxide (5g, 71.3mmol) and potassium carbonate (14.8g, 107mmol). The mixture was stirred at room temperature for 48 hours. The suspension was filtrated on a short pad ofcelite and rinsed with ethyl acetate. The filtrate was concentrated. Water was added, the aqueous phase was acidified to pH 4 with a 1 N solution of hydrochloric acid and extracted with ethyl acetate. The organic layers were dried over magnesium sulfate, filtered and evaporated under vacuo to afford 3-methylsulfanyl-4-nitro-phenol (Yield: 85%, 5.6g, 85%) as yellow solid.

Step 2. Iron powder (11.9g, 213.8mmol) was slowly added to a solution of 3-methylsulfanyl-4-nitrophenol (13.2g, 71.3mmol) in 300mL of acetic acid and 30mL of ethanol. The reaction mixture was heated up to 45°C for 18 h. After cooling down, the suspension was filtrated on a short pad of celite and rinsed with methanol. The filtrate was concentrated and adsorbed on silica gel to be purified by flash chromatography using cyclohexane and ethyl acetate. 6.1g of 4-amino-3-methylsulfanyl-phenol (5) (Yield: 55%) was obtained as brown solid.

### Synthesis of 5-(4-amino-3-methylsulfanyl-phenoxy)-3,4-dihydro-1H-1,8-naphthyridin-2-one (7)

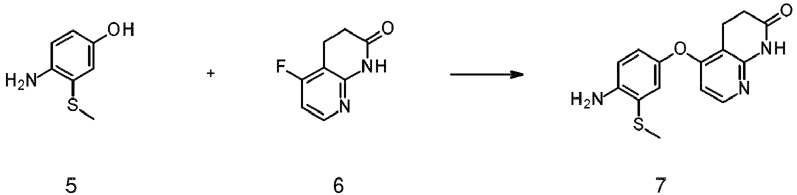

A suspension of 4-amino-3-(methylsulfanyl)phenol (5) (3.0g, 19.3mmol), 5-fluoro-1,2,3,4-tetrahydro-1,8-naphthyridin-2-one (6) (3.2g, 19.3mmol) and Cs₂CO₃ (8.5 g, 26.0mmol) in DMA (90mL) was stirred overnight at 180 °C. The reaction was quenched with water and the mixture was extracted with ethyl acetate (3 x 200mL). The organic layers were combined, dried over MgSO₄, filtered and concentrated under reduced pressure to give 4.8g (Yield: 82%) of the crude product. This material was used for the next step without further purification. 1H NMR (400 MHz, DMSO-d6) δ 10.43 (s, 1H), 7.92 (d, J = 5.8 Hz, 1H), 6.96 (d, J = 2.5 Hz, 1H), 6.82 -6.72 (m, 2H), 6.21 (d, J = 5.8 Hz, 1H), 5.14 (s, 2H), 2.97 - 2.88 (m, 2H), 2.56 - 2.52 (m, 2H), 2.36 (s, 3H).

### Synthesis of 5-[4-amino-3-(trifluoromethyl]phenoxyl-3,4-dihvdro-1H-1,8-naphthyridin-2-one (9)

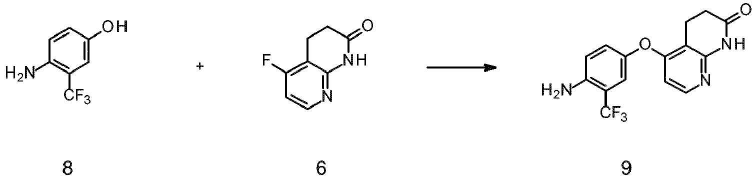

A suspension of 4-amino-3-(trifluoromethyl)phenol (8) (2.0g, 11mmol), 5-fluoro-1,2,3,4-tetrahydro-1,8-naphthyridin-2-one (6) (1.9g, 11mmol) and Cs₂CO₃ (5.0g, 14.8mmol) in DMA (60mL) was stirred overnight at 180 °C. The reaction was cooled down to room temperature, water was added, and the resulting mixture was extracted with ethyl acetate (3 x 200mL). The organic layers were combined, dried over MgSO₄, filtered, and concentrated under reduced pressure to give 2.4g (Yield: 66%) of the crude product. This material was used for the next step without further purification. 1H NMR (400 MHz, DMSO-d6) δ 10.46 (s, 1H), 7.94 (d, J = 5.8 Hz, 1H), 7.15 (d, J = 8.6 Hz, 2H), 6.92 (d, J = 8.5 Hz, 1H), 6.21 (d, J = 5.8 Hz, 1H), 5.65 (s, 2H), 2.98 - 2.87 (m, 2H), 2.57 - 2.52 (m, 2H).

### Synthesis of 8-[4-amino-3-(trifluoromethyl)phenoxyl-4H-pyrido[3,2-b][1,4]oxazin-3-one (11)

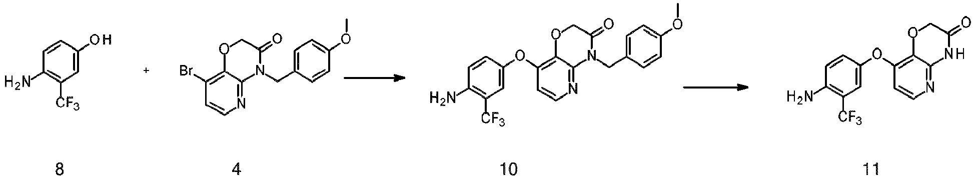

Step 1: A suspension of 4-amino-3-(trifluoromethyl)phenol (8) (4.0g, 22mmol), 8-bromo-4-[(4-methoxyphenyl)methyl]-2H,3H,4H-pyrido[3,2-b][1,4]oxazin-3-one (4) (8.0g, 22mmol) and Cs₂CO₃ (9.9g, 29.7mmol) in DMA (120mL) was stirred overnight at 180 °C. The reaction was cooled down to room temperature, water was added, and the resulting mixture was extracted with diethyl ether (3 x 300mL). The organic layers were combined, dried over MgSO₄, filtered and concentrated under reduced pressure to give 8.1g (Yield: 81%) of the desired product (10). This material was used for the next step without further purification. 1H NMR (400 MHz, DMSO-d6) δ 7.86 (d, J = 5.7 Hz, 1H), 7.30 - 7.24 (m, 2H), 7.15 (d, J = 8.3 Hz, 2H), 6.91 (d, J = 8.5 Hz, 1H), 6.87 - 6.83 (m, 2H), 6.44 (d, J = 5.7 Hz, 1H), 5.64 (s, 2H), 5.18 (s, 2H), 4.84 (s, 2H), 3.71 (s, 3H).

Step 2: 8-[4-amino-3-(trifluoromethyl)phenoxy]-4-[(4-methoxyphenyl)methyl]-2H,3H,4H-pyrido[3,2-b][1,4]- oxazin-3-one (10) (8.1g, 17.8mmol) was dissolved in DCM (160mL). Then to the solution were added TFA (4.4mL, 55.2mmol) and trifluoromethanesulfonic acid (8.1 mL, 89.0mmol). The reaction mixture was stirred overnight at room temperature, then cooled down to 0 °C, and treated with aqueous solution of NaOH to get pH=9. The phases were separated, and aqueous layer was extracted with DCM (2 x 300mL). The combined organic layers were washed with brine, dried over MgSO₄, filtered, and concentrated in vacuo. The residue was triturated with Et₂O (2 x 50mL) to give 3.5g of the desire product (11) (Yield: 59%). This material was used for the next step without further purification. 1H NMR (300 MHz, DMSO-d6) δ 11.25 (s, 1H), 7.75 (d, J = 5.7 Hz, 1H), 7.13 (d, J = 9.1 Hz, 2H), 6.90 (d, J = 8.6 Hz, 1H), 6.35 (d, J = 5.7 Hz, 1H), 5.62 (s, 2H), 4.65 (s, 2H).

### Synthesis of 8-(4-amino-3-methylsulfanyl-phenoxy)-4H-pyrido[3,2-b][1,4]oxazin-3-one (13)

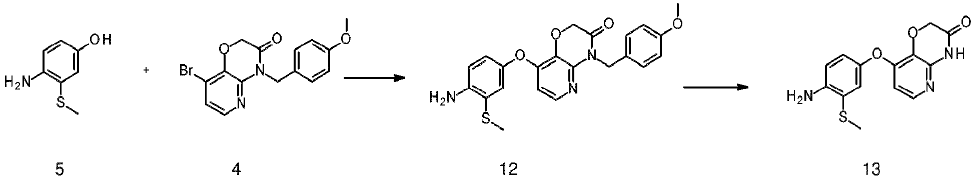

According to the procedure described for (11), starting from 4-amino-3-(methylsulfanyl)phenol (5) (5.0g, 32.2mmol), 8-bromo-4-[(4-methoxyphenyl)methyl]-2H,3H,4H-pyrido[3,2-b][1,4]oxazin-3-one (4) (11.2g, 32.2mmol) and Cs₂CO₃ (14.1g, 43.4mmol) in DMA (150mL), crude intermediate (12) was obtained (11.0g) that was directly deprotected using a mixture of TFA (6.2mL, 80.5mmol) and trifluoromethanesulfonic acid (11.5ml, 129.8mmol) to give after work-up 6.4g of 8-(4-amino-3-methylsulfanyl-phenoxy)-4H-pyrido[3,2-b][1,4]oxazin-3-one (13) (Yield: 82%). This material was used for the next step without further purification.

### Synthesis of (5-fluoro-3-pyridyl)hydrazine (14)

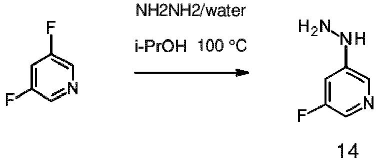

According to the procedure described in WO2021090030, a solution of 3,5-difluoropirydine (3.0g, 26.1mmol), hydrazine (50-60% in water) (25.1g, 390mmol), and iPrOH (90mL) was heated at 100 °C in a sealed tube and stirred overnight. The resulting mixture was cooled down, the solvents were carefully removed under reduced pressure to obtain off-white solid (3.38 g) of the crude material that was used for the next step without further purification.

### Synthesis of 5-tert-butyl-2-(5-fluoro-3-pyridyl)pyrazol-3-amine (15)

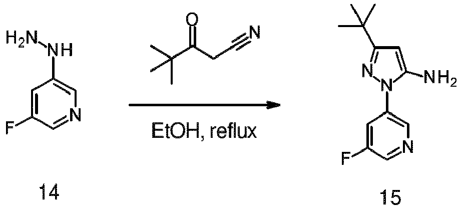

3-Fluoro-5-hydrazinylpyridine (3.4g, 26.7mmol) was dissolved in EtOH (80mL) and pivaloyl acetonitrile (4.0g, 32.0mmol) was added. The reaction mixture was refluxed for 16 hours, then was cooled down to room temperature, and quenched with saturated sodium bicarbonate solution. The mixture was extracted with DCM (2x 200mL), the combined organic layers were dried over MgSO₄, filtered, and the solvent was removed in vacuo. The crude product was purified by column chromatography using DCM/MeOH (100/0->9/1) as an eluent to afford 5-tert-butyl-2-(5-fluoro-3-pyridyl)pyrazol-3-amine (15) as an oil (3.6g, Y=58%).

### Synthesis of 5-tert-butyl-2-(6-methyl-3-pyridyl)pyrazol-3-amine (16)

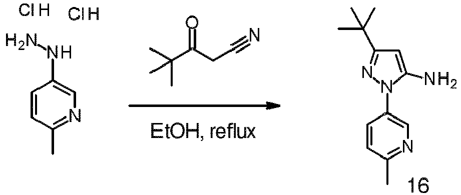

Following the procedure described for compound (15), starting from 2-Methyl-5-hydrazinylpyridine bis-hydrochloride (1.0g, 5.1mmol) instead of 3-Fluoro-5-hydrazinylpyridine and pivaolyl acetonitrile (0.96g, 7.7mmol) in EtOH (20 mL), 1.6g of 5-tert-butyl-2-(6-methyl-3-pyridyl)pyrazol-3-amine (16) were obtained (Yield: 85%).

### Synthesis of 5-tert-butyl-2-(2,4-difluorophenyl)pyrazol-3-amine (17)

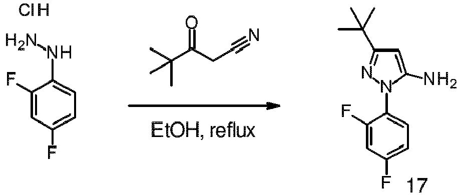

Following the procedure described for compound (15), starting from 2,4-difluorophenylhydrazine hydrochloride (2.0g, 11mmol) instead of 3-fluoro-5-hydrazinylpyridine and pivaloyl acetonitrile (1.9g (15.2mmol) in EtOH (20 mL), 2.7g of 5-tert-butyl-2-(2,4-difluorophenyl)pyrazol-3-amine (17) were obtained (Yield: 86%).

### Synthesis of 5-tert-butyl-2-(p-tolyl)pyrazol-3-amine (18)

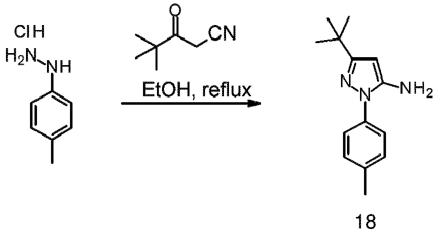

Following the procedure described for compound (15), starting from 4-methylphenylhydrazine hydrochloride (2.0g, 12.6mmol) instead of 3-fluoro-5-hydrazinylpyridine and pivaloyl acetonitrile (1.9g (15.2mmol) in EtOH (40 mL), 2.4g of 5-tert-butyl-2-(p-tolyl)pyrazol-3-amine (18) were obtained (Yield: 82%).

### Synthesis of 5-tert-butyl-2-(3-fluorophenyl)pyrazol-3-amine (19)

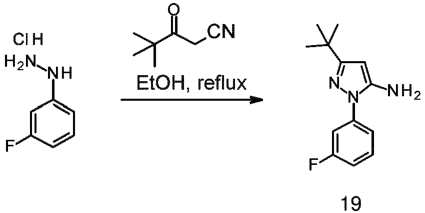

Following the procedure described for compound (15), starting from 3-fluorophenylhydrazine hydrochloride (1.2g, 7.2mmol) instead of 3-fluoro-5-hydrazinylpyridine and pivaloyl acetonitrile (1.1g (8.6mmol) in EtOH (23 mL), 1.36g of 5-tert-butyl-2-(3-fluorophenyl)pyrazol-3-amine (19) were obtained (Yield: 82%).

### Synthesis of 5-tert-butyl-2-[4-[(dimethylamino)methyl]phenyl]pyrazol-3-amine (23)

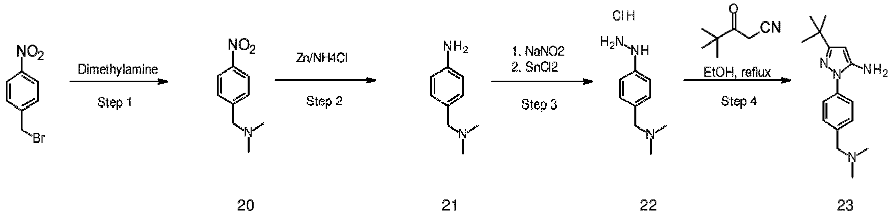

### Step1: synthesis of compound 20 :

To a solution of 4-nitrobenzyl bromide (4.32g, 20mmol) in diethyl ether (20mL) was added dimethylamine solution (40% in water, 12.6mL, 100mmol) and the reaction mixture was stirred vigorously at room temperature overnight. The layers were separated, and the organic layer was washed with 10% solution of citric acid (2x30mL). The combined aqueous layers were basified with 20% NaOH to bring the pH to 11. The product was extracted with AcOEt (3x30mL). The combined organic layers were washed with brine, dried over sodium sulfate, filtered, and the solvent was evaporated in vacuo to give 3.6g of dimethyl[(4-nitrophenyl)methyl]amine (20) as of yellow oil ( Yield=99%).

### Step2: synthesis of compound 21:

To a solution of dimethyl[(4-nitrophenyl)methyl]amine (20) (3.6g, 19.9mmol) in MeOH (55mL) at 0 °C was added zinc powder (6.53g, 99.9mmol) followed by dropwise addition of saturated solution of ammonium chloride (18.0mL). The reaction mixture was stirred for 20 min at 0 °C, next at room temperature overnight. The resulting mixture was filtered through a pad of Celite, and the filtrate was partially concentrated under reduced pressure. The aqueous phase was diluted with brine (50 mL), and DCM (50mL) was added. The layers were separated, and the aqueous phase was extracted with DCM (5 x 50mL). The combined organic layers were washed with brine, dried over sodium sulphate, filtered, and evaporated under reduced pressure to yield 2.7g of 4-[(dimethylamino)methyl]aniline (21) as light brown solid (Yield=90%).

### Step 3: synthesis of compound 22:

To a solution of 4-[(dimethylamino)methyl]aniline (21) (2.7g, 18.0mmol) in a mixture of water (60mL) and concentrated HCl solution (8.8mL, 360mmol) at -5 °C was dropwise added a solution of sodium nitrate (1.86g, 27mmol) in water (13mL) keeping the internal temperature around 0 °C. Next, the reaction mixture was stirred at 0 °C for 1h. Then, a freshly prepared solution of tin(IV) chloride (17.0g, 89.7mmol) in water (80mL) was dropwise added keeping the internal temperature around 0 °C. The resulting mixture was stirred at 0 °C for 0.5h and next at room temperature for 2h. The reaction mixture was used for the next step 4 as a crude solution of [(4-hydrazinylphenyl)methyl] dimethylamine (22).

### Step 4: synthesis of compound 23:

To the crude solution of [(4-hydrazinylphenyl)methyl] dimethylamine (22, step 3) was added EtOH 60mL) followed by pivaloyl acetonitrile (2.7g, 21.6mmoml). The reaction mixture was next stirred at 90 °C overnight. EtOH was removed under reduced pressure, and the remaining solution was basified with 20% solution of NaOH to pH=10-11. The obtained emulsion was evaporated in vacuo to dryness. The residue was stirred with 10% MeOH/DCM solution (100mL) at room temperature for 1 h, and it was filtered off. The filtrate was concentrated under reduced pressure, and the crude product was purified by flash chromatography (0% to 10% MeOH in DCM) to afford 1.1g of 5-tert-butyl-2-[4-[(dimethylamino)methyl]phenyl]pyrazol-3-amine (23) as light brown solid (Yield=22%, 2 steps).

### Synthesis of 5-tert-butyl-2-[4-(morpholinomethyl)phenyl]pyrazol-3-amine (27)

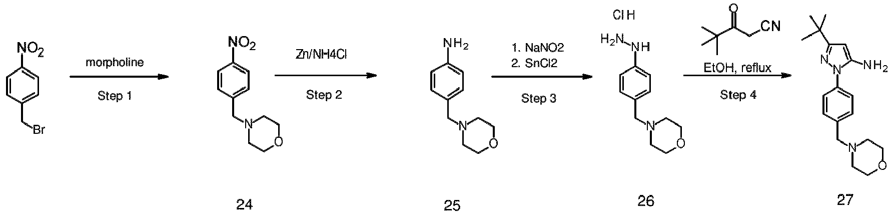

### Step1: synthesis of compound 24 :

Following the procedure described for compound (20), starting from 4-nitro-benzyl bromide (4.2g, 20.0mmol) morpholine (3.5mL, 40.0mmol) instead of dimethylamine and potassium carbonate (4.15g, 30.0 mmol) in DMSO (20 mL) instead of diethylether, 4.4g of 4-[(4-nitrophenyl)methyl]morpholine (24) were obtained (Yield: 99%).

### Step2: synthesis of compound 25:

Following the procedure described for compound (21), starting from 4-[(4-nitrophenyl)methyl]morpholine (24) (4.2g, 20.0mmol) instead of N,N-dimethyl-1-(4-nitrophenyl)methanamine, Zinc dust (6.5g, 99.0mmol), NH₄Cl (sat.) (22mL) in methanol (55mL) , 3.9g of 4-[(4-aminophenyl)methyl]morpholine (25) were obtained (Yield: 100%).

### Step 3: synthesis of compound 26:

Following the procedure described for compound (22), starting from 4-[(4-aminophenyl)methyl]morpholine (25) (3.8g, 19.8mmol) instead of N,N-dimethyl-1-(4-aminophenyl)methanamine, sodium nitrite (2.1g, 29.7mmol), HCl conc (9.7mL, 395mmol), Tin(II) chloride (18.7g, 98.8mmol) in water (80mL) , crude reaction mixture of [4-(morpholinomethyl)phenyl]hydrazine (26) was obtained and used directly in the next step 4.

### Step 4: synthesis of compound 27:

Following the procedure described for compound (23), starting from crude reaction mixture of [4-(morpholinomethyl)phenyl]hydrazine (26) (from step 3) instead of [4-(N,N-dimethylaminomethy)phenyl]hydrazine, pivaloyl acetonitrile (3.7g, 29.7mmol) in ethanol (80mL), 1.2g of 5-tert-butyl-2-[4-(morpholinomethyl)phenyl]pyrazol-3-amine (27) were obtained (Yield: 20%, 2 steps).

### Synthesis of 5-tert-butyl-2-[3-(morpholinomethyl)phenyl]pyrazol-3-amine (31)

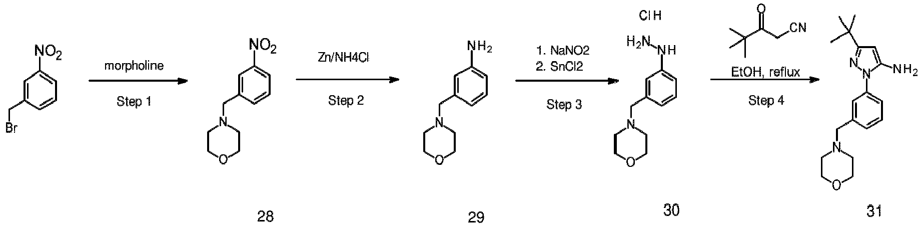

### Step1: synthesis of compound 28:

Following the procedure described for compound (20), starting from 3-nitro-benzyl bromide (4.3g, 20.0mmol) morpholine (3.5mL, 40.0mmol) instead of dimethylamine and potassium carbonate (4.15g, 30.0 mmol) in DMSO (20 mL), 4.2g of 4-[(3-nitrophenyl)methyl]morpholine (28) were obtained (Yield: 94%).

### Step2: synthesis of compound 29:

Following the procedure described for compound (21), starting from 4-[(3-nitrophenyl)methyl]morpholine (28) (4.15g, 18.7mmol) instead of N,N-dimethyl-1-(4-nitrophenyl)methanamine, Zinc dust (6.1g, 93.5mmol), NH₄Cl (sat.) (6.5mL) in methanol (20mL) , 3.1g of 4-[(3-aminophenyl)methyl]morpholine (29) were obtained (Yield: 85%).

### Step 3: synthesis of compound 30:

Following the procedure described for compound (22), starting from 4-[(3-aminophenyl)methyl]morpholine (29) (3.05g, 15.9mmol) instead of N,N-dimethyl-1-(4-aminophenyl)methanamine, sodium nitrite (1.6g, 23.8mmol), HCI conc (7.8mL, 317mmol), Tin(II) chloride (15.0g, 79.3mmol) in water (80mL) , crude reaction mixture of [3-(morpholinomethyl)phenyl]hydrazine (30) was obtained and used directly in the next step 4.

### Step 4: synthesis of compound 31:

Following the procedure described for compound (23), starting from crude reaction mixture of [3-(morpholinomethyl)phenyl]hydrazine (30) (from step 3) instead of [4-(N,N-dimethylaminomethy)phenyl]hydrazine, pivaloyl acetonitrile (2.9g, 23.8mmol) in ethanol (80mL) , 1.05g of 5-tert-butyl-2-[3-(morpholinomethyl)phenyl]pyrazol-3-amine (31) were obtained (Yield: 21%, 2 steps).

### Synthesis of 5-tert-butyl-2-[3-[(dimethylamino)methyl]phenyl]pyrazol-3-amine (35)

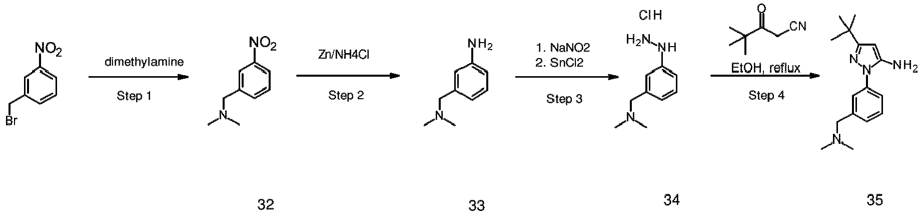

### Step1: synthesis of compound 32:

Following the procedure described for compound (20), starting from 3-nitro-benzyl bromide (4.3g, 20.0mmol) instead of 4-nitro-benzyl bromide, dimethylamine (40% in water) (12.6mL, 100.0mmol) in diethyl ether (20 mL), 3.6g of N,N-dimethyl-1-(3-nitrophenyl)methanamine (32) were obtained (Yield: 100%).

### Step2: synthesis of compound 33:

Following the procedure described for compound (21), starting from N,N-dimethyl-1-(3-nitrophenyl)methanamine (32) (3.6g, 19.9mmol) instead of N,N-dimethyl-1-(4-nitrophenyl)methanamine, Zinc dust (6.5g, 99.9mmol), NH₄Cl (sat.) (18.0mL) in methanol (55mL) , 2.7g of 3-[(dimethylamino)methyl]aniline (33) were obtained (Yield: 90%).

### Step 3: synthesis of compound 34:

Following the procedure described for compound (22), starting from 3-[(dimethylamino)methyl]aniline (33) (2.7g, 18.0mmol) instead of 4-[(dimethylamino)methyl]aniline, sodium nitrite (1.9g, 27.0mmol), HCI conc (8.8mL, 360mmol), Tin(II) chloride (17.0g, 89.7mmol) in water (60mL) , crude reaction mixture of 1-(3-hydrazinophenyl)-N,N-dimethyl-methanamine (34) was obtained and used directly in the next step 4.

### Step 4: synthesis of compound 35:

Following the procedure described for compound (23), starting from crude reaction mixture of 1-(3-hydrazinophenyl)-N,N-dimethyl-methanamine (34) (from step 3) instead of [4-(N,N-dimethylaminomethy)phenyl]hydrazine, pivaloyl acetonitrile (2.7g, 21.6mmol) in ethanol (60mL) , 1.05g of 5-tert-butyl-2-[3-[(dimethylamino)methyl]phenyl]pyrazol-3-amine (35) were obtained (Yield: 21%, 2 steps).

### Synthesis of 5-tert-butyl-2-[4-[2-(dimethylamino)ethyl]phenyl]pyrazol-3-amine (39)

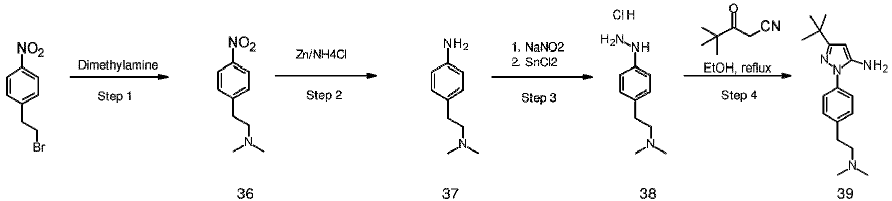

### Step1: synthesis of compound 36:

Following the procedure described for compound (20), starting from 1-(2-bromoethyl)-4-nitrobenzene (5.0g, 21.7mmol) instead of 4-nitro-benzyl bromide, dimethylamine (40% in water) (13.7mL, 108.7mmol) in diethyl ether (23 mL), 3.5g of N,N-dimethyl-2-(4-nitrophenyl)ethanamine (36) were obtained (Yield: 82%).

### Step2: synthesis of compound 37:

Following the procedure described for compound (21), starting from N,N-dimethyl-2-(4-nitrophenyl)ethanamine (36) (3.5g, 17.8mmol) instead of N,N-dimethyl-1-(4-nitrophenyl)methanamine, Zinc dust (5.8g, 89.0mmol), NH₄Cl (sat.) (17.5mL) in methanol (53mL), 2.4g of 4-[2-(dimethylamino)ethyl]aniline (37) were obtained (Yield: 83%).

### Step 3: synthesis of compound 38:

Following the procedure described for compound (22), starting from 4-[2-(dimethylamino)ethyl]aniline (37) (2.4g, 14.8mmol) instead of 4-[(dimethylamino)methyl]aniline, sodium nitrite (1.53g, 22.2mmol), HCl conc (7.2mL, 296mmoL), Tin(II) chloride (14.0g, 74.0mmol) in water (60mL) , crude reaction mixture of 2-(4-hydrazinophenyl)-N,N-dimethyl-ethanamine (38) was obtained and used directly in the next step 4.

### Step 4: synthesis of compound 39:

Following the procedure described for compound (23), starting from crude reaction mixture of 2-(4-hydrazinophenyl)-N,N-dimethyl-ethanamine (38) (from step 3) instead of [4-(N,N-dimethylaminomethy)phenyl]hydrazine, pivaloyl acetonitrile (2.8g, 22.2mmol) in ethanol (60mL) , 1.2g of 5-tert-butyl-2-[4-[2-(dimethylamino)ethyl]phenyl]pyrazol-3-amine (39) were obtained after silicagel flash chromatography (Yield: 28%, 2 steps).

### Synthesis of 55-tert-butyl-2-[4-(2-morpholinoethyl)phenyl]pyrazol-3-amine (43)

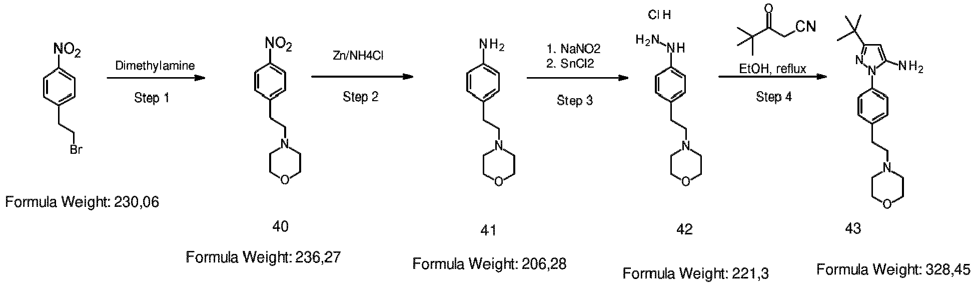

### Step1: synthesis of compound 40:

Following the procedure described for compound (20), starting from 1-(2-bromoethyl)-4-nitrobenzene (5.0g, 21.7mmol) instead of 4-nitro-benzyl bromide, morpholine (9.5mL, 43.4mmol) instead of dimethylamine and potassium carbonate (11.3g, 81.9 mmol) in DMSO (23 mL), 5.1g of 4-[2-(4-nitrophenyl)ethyl]morpholine (40) were obtained (Yield: 100%).

### Step2: synthesis of compound 41:

Following the procedure described for compound (21), starting from 4-[2-(4-nitrophenyl)ethyl]morpholine (40) (5.1g, 21.7mmol) instead of N,N-dimethyl-1-(4-nitrophenyl)methanamine, Zinc dust (7.2g, 109.6mmol), NH₄Cl (sat.) (25.0mL) in methanol (78mL), 4.06g of 4-(2-morpholinoethyl)aniline (41) were obtained (Yield: 91%).

### Step 3: synthesis of compound 42:

Following the procedure described for compound (22), starting from 4-(2-morpholinoethyl)aniline (41) (4.0g, 19.4mmol) instead of 4-[(dimethylamino)methyl]aniline, sodium nitrite (2.0g, 29.1mmol), HCl conc (9.5mL, 388mmol), Tin(II) chloride (18.4g, 97.0mmol) in water (80mL) , crude reaction mixture of [4-(2-morpholinoethyl)phenyl]hydrazine (42) was obtained and used directly in the next step 4.

### Step 4: synthesis of compound 43:

Following the procedure described for compound (23), starting from crude reaction mixture of [4-(2-morpholinoethyl)phenyl]hydrazine (42) (from step 3) instead of [4-(N,N-dimethylaminomethy)phenyl]hydrazine, pivaloyl acetonitrile (3.6g, 29.1mmol) in ethanol (80mL) , 1.03g of 5-tert-butyl-2-[4-(2-morpholinoethyl)phenyl]pyrazol-3-amine (43) were obtained (Yield: 16%, 2 steps).

### Synthesis of 5-tert-butyl-2-[4-[(4-methylpiperazin-1-yl)methyl]phenyl]pyrazol-3-amine (47)

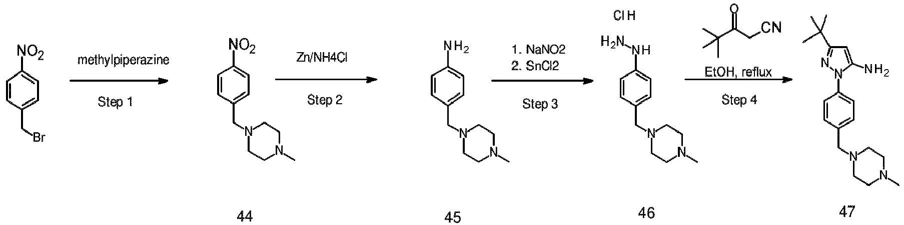

### Step1: synthesis of compound 44:

Following the procedure described for compound (20), starting from 4-nitro-benzyl bromide (4.3g, 20.0mmol) N-methylpiperazine (4.5mL, 40.0mmol) instead of dimethylamine and potassium carbonate (6.9g, 50.0 mmol) in DMF (20 mL), 4.3g of 1-methyl-4-[(4-nitrophenyl)methyl]piperazine (44) were obtained (Yield: 90%).

### Step2: synthesis of compound 45:

Following the procedure described for compound (21), starting from 1-methyl-4-[(4-nitrophenyl)methyl]piperazine (44) (4.2g, 17.9mmol) instead of N,N-dimethyl-1-(4-nitrophenyl)methanamine, Zinc dust (5.8g, 89.3mmol), NH₄Cl (sat.) (21mL) in methanol (65mL) , 3.6g of 4-[(4-methylpiperazin-1-yl)methyl]aniline (45) were obtained (Yield: 98%).

### Step 3: synthesis of compound 46:

Following the procedure described for compound (22), starting from 4-[(4-methylpiperazin-1-yl)methyl]aniline (45) (3.6g, 17.5mmol) instead of N,N-dimethyl-1-(4-aminophenyl)methanamine, sodium nitrite (1.45g, 21.0mmol), HCl 1N (70mL), Tin(II) chloride dihydrate (13.2g, 58.4mmol) in a mixture of water (20mL) and Acetic acid (35mL), 7.5g of crude [4-[(4-methylpiperazin-1-yl)methyl]phenyl]hydrazine (46) were obtained after work-up and used directly in the next step 4 without further purification.

### Step 4: synthesis of compound 47:

Following the procedure described for compound (23), starting from crude [4-[(4-methylpiperazin-1-yl)methyl]phenyl]hydrazine (46) (from step 3) instead of [4-(N,N-dimethylaminomethy)phenyl]hydrazine, pivaloyl acetonitrile (3.8g, 30.7mmol) in ethanol (100mL), 0.71g of 5-tert-butyl-2-[4-[(4-methylpiperazin-1-yl)methyl]phenyl]pyrazol-3-amine (47) were obtained (Yield: 12%, 2 steps).

### Synthesis of 2,2,2-trichloroethyl (3-(tert-butyl)-1-phenyl-1H-pyrazol-5-yl)carbamate (48)

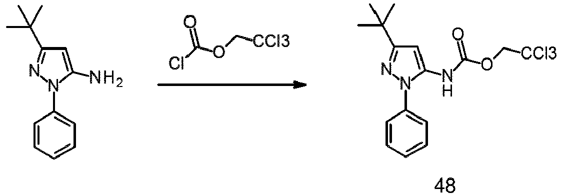

At 0°C, to a solution of 3-(tert-butyl)-1-phenyl-1H-pyrazol-5-amine (690mg, 3.2mmol) in dry THF (16 mL) was added dropwise dry pyridine (1.1eq) followed by 2,2,2-trichloroethylchloroformate (0.48mL, 3.52mmol). The reaction mixture was stirred at room temperature for 18h. Water was added, and the reaction mixture was extracted with ethyl acetate. The organic layers were combined, dried over magnesium sulphate, filtered and evaporated to give 1.2g of 2,2,2-trichloroethyl (3-(tert-butyl)-1-phenyl-1H-pyrazol-5-yl)carbamate (48) as white powder (Yield: 95%) and was used without further purification.

### Synthesis 2,2,2-trichloroethyl (3-(tert-butyl)-1-(p-tolyl)-1H-pyrazol-5-yl)carbamate (49)

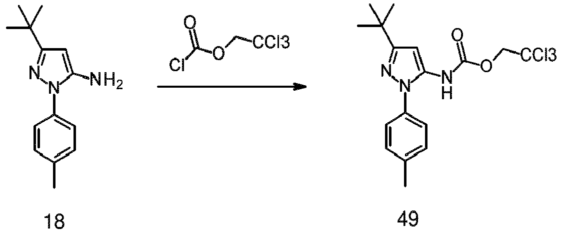

At 0°C, to a THF solution of 5-tert-butyl-2-(p-tolyl)pyrazol-3-amine (18) (550mg, 2.4mmol) was added dropwise dry pyridine (1.1eq) followed by 2,2,2-trichloroethylchloroformate (0.36mL, 2.64mmol). The reaction mixture was stirred at room temperature for 18h. Water was added, and the reaction mixture was extracted with ethyl acetate. The organic layers were combined, dried over magnesium sulphate, filtered and evaporated. 950 mg of 2,2,2-trichloroethyl (3-(tert-butyl)-1-(p-tolyl)-1H-pyrazol-5-yl)carbamate (49) were obtained as white powder (Yield: 98%) and used without further purification.

### Synthesis of 2,2,2-trichloroethyl (5-(tert-butyl)thiazol-2-yl)carbamate (50)

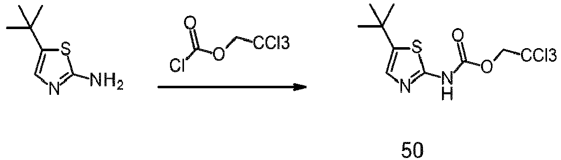

Following the procedure described for compound (49), starting from 5-tert-butylthiazol-2-yl)amine (500mg, 3.2mmol) instead of 3-(tert-butyl)-1-phenyl-1H-pyrazol-5-amine and 2,2,2-trichloroethylchloroformate (0.48mL, 3.52mmol), 1.0g of 2,2,2-trichloroethyl (5-(tert-butyl)thiazol-2-yl)carbamate (50) were obtained as white powder (Yield: 94%).

### Synthesis of 2,2,2-trichloroethyl N-[5-tert-butyl-2-(3-chlorophenyl)pyrazol-3-yl]carbamate (51)

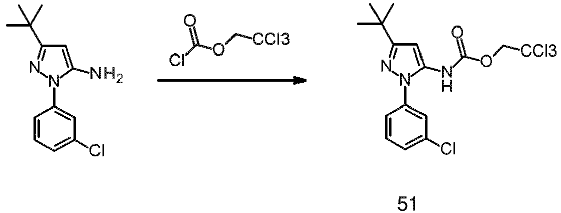

Following the procedure described for compound (49), starting from 5-tert-butyl-2-(3-chlorophenyl)pyrazol-3-yl]amine (500mg, 2.0mmol) instead of 3-(tert-butyl)-1-phenyl-1H-pyrazol-5-amine and 2,2,2-trichloroethylchloroformate (0.31mL, 2.2mmol), 0.93g of 2,2,2-trichloroethyl N-[5-tert-butyl-2-(3-chlorophenyl)pyrazol-3-yl]carbamate (51) were obtained as brown gum (Yield: 100%).

### Synthesis of 2,2,2-trichloroethyl (5-(trifluoromethyl)pyridin-3-yl)carbamate (52)

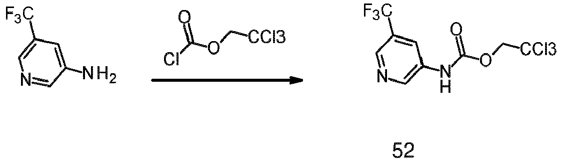

Following the procedure described for compound (49), starting from 5-(trichloromethyl)pyridin-3-amine (423mg, 2.0mmol) instead of 3-(tert-butyl)-1-phenyl-1H-pyrazol-5-amine and 2,2,2-trichloroethylchloroformate (0.31mL, 2.2mmol), 0.77g of 2,2,2-trichloroethyl (5-(trifluoromethyl)pyridin-3-yl)carbamate (52) were obtained as brown gum (Yield: 100%).

### Synthesis of 2,2,2-trichloroethyl (3-(tert-butvl)isoxazol-5-vl)carbamate (53)

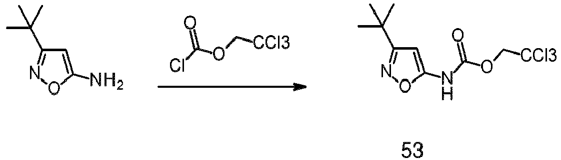

Following the procedure described for compound (49), starting from 3-tert-butylisoxazol-5-amine (5.0g, 35.7mmol) instead of 3-(tert-butyl)-1-phenyl-1H-pyrazol-5-amine and 2,2,2-trichloroethylchloroformate (5.4mL, 39.2mmol), 11.5g of 2,2,2-trichloroethyl (3-(tert-butyl)isoxazol-5-yl)carbamate (52) were obtained as pale yellow powder (Yield: 100%).

### Synthesis of 2,2,2-trichloroethyl N-[5-tert-butyl-2-(3-florophenyl)pyrazol-3-yl]carbamate (54)

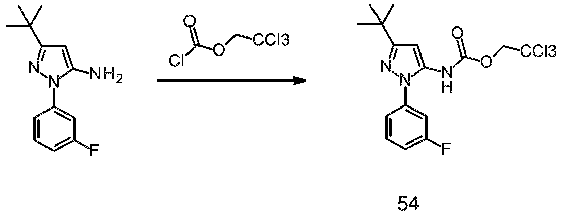

Following the procedure described for compound (49), starting from 5-tert-butyl-2-(3-florophenyl)pyrazol-3-yl]amine (19) (0.1g, 0.43mmol) instead of 3-(tert-butyl)-1-phenyl-1H-pyrazol-5-amine and 2,2,2-trichloroethylchloroformate (0.071mL, 0.51mmol), 0.123g of 2,2,2-trichloroethyl N-[5-tert-butyl-2-(3-chlorophenyl)pyrazol-3-yl]carbamate (51) were obtained (Yield: 70%).

### Synthesis of 2,2,2-trichloroethyl N-[5-tert-butyl-2-(5-fluoro-3-pyridyl)pyrazol-3-yl]carbamate (55)

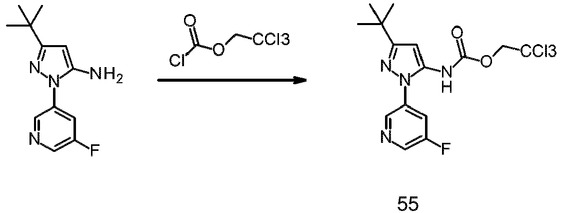

Following the procedure described for compound (49), starting from 5-tert-butyl-2-(5-fluoro-3-pyridyl)pyrazol-3-amine (15) (0.3g, 0.43mmol) instead of 3-(tert-butyl)-1-phenyl-1H-pyrazol-5-amine, 2,2,2-trichloroethylchloroformate (0.212mL, 1.2mmol) and pyridine (0.41mL, 5.1mmol) in THF (9mL), 0.260g of 2,2,2-trichloroethyl N-[5-tert-butyl-2-(5-fluoro-3-pyridyl)pyrazol-3-yl]carbamate (55) were obtained after flash chromatography purification (Yield: 51%).

### Synthesis of 2,2,2-trichloroethyl N-[5-tert-butyl-2-(6-methyl-3-pyridyl)pyrazol-3-yl]carbamate (56)

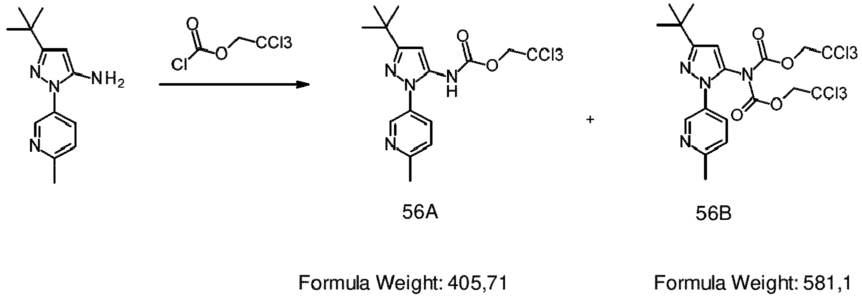

Following the procedure described for compound (49), starting from 5-tert-butyl-2-(6-methyl-3-pyridyl)pyrazol-3-amine (16) (0.3g, 1.3mmol) instead of 3-(tert-butyl)-1-phenyl-1H-pyrazol-5-amine, 2,2,2-trichloroethylchloroformate (0.210mL, 1.5mmol) and pyridine (0.42mL, 5.2mmol) in THF (9mL), 0.120g of 2,2,2-trichloroethyl N-[5-tert-butyl-2-(6-methyl-3-pyridyl)pyrazol-3-yl]carbamate (56A) (Yield: 36%) and 0.270mg of 2,2,2-trichloroethyl N-[5-tert-butyl-2-(6-methyl-3-pyridyl)pyrazol-3-yl]-N-(2,2,2-trichloroethoxycarbonyl)carbamate (56B) (Yield: 23%) were obtained after flash chromatography purification.

### Synthesis of 2,2,2-trichloroethyl (5-(tert-butvl)isoxazol-3-vl)carbamate (57)

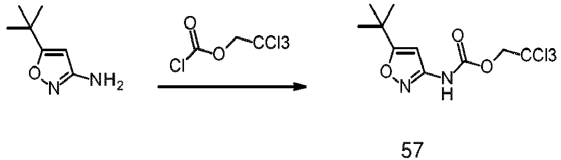

Following the procedure described for compound (49), starting from 5-tert-butylisoxazol-3-amine (1.0g, 7.1mmol) instead of 3-(tert-butyl)-1-phenyl-1H-pyrazol-5-amine and 2,2,2-trichloroethylchloroformate (1.1mL, 7.8mmol), 2.26g of 2,2,2-trichloroethyl (5-(tert-butyl)isoxazol-3-yl)carbamate (57) were obtained (Yield: 100%) and used without further purification.

### Synthesis of 2,2,2-trichloroethyl N-(5-tert-butyl-2-methyl-pyrazol-3-yl)carbamate (58)

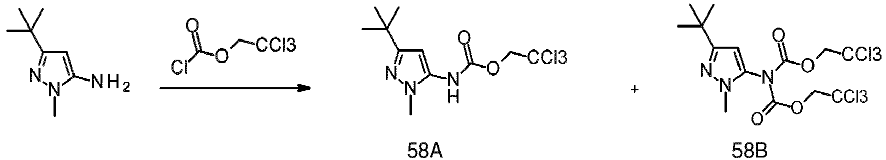

Following the procedure described for compound (49), starting from 5-tert-butyl-2-methyl-pyrazol-3-amine (0.3g, 2.0mmol) instead of 3-(tert-butyl)-1-phenyl-1H-pyrazol-5-amine and 2,2,2-trichloroethylchloroformate (0.3mL, 2.2mmol), 0.7g of a 90/10 mixture of 2,2,2-trichloroethyl (5-(tert-butyl)isoxazol-3-yl)carbamate (58A) and 2,2,2-trichloroethyl N-(5-tert-butyl-2-methyl-pyrazol-3-yl)-N-(2,2,2-trichloroethoxycarbonyl)carbamate (58B) were obtained (Yield: 100%) and used without further purification.

### Synthesis of 2,2,2-trichloroethyl N-(5-tert-butvloxazol-2-vl)carbamate (59)

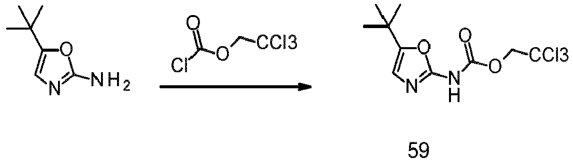

Following the procedure described for compound (49), starting from 5-tert-butyloxazol-2-amine (0.12g, 0.86mmol) instead of 3-(tert-butyl)-1-phenyl-1H-pyrazol-5-amine and 2,2,2-trichloroethylchloroformate (0.13mL, 0.94mmol), 0.26g of 2,2,2-trichloroethyl N-(5-tert-butyloxazol-2-yl)carbamate (59) and were obtained (Yield: 100%) and used without further purification.

### Synthesis of 2,2,2-trichloroethyl N-[5-tert-butyl-2-[3-[(dimethylamino)methyl]phenyl]pyrazol-3-vllcarbamate (60)

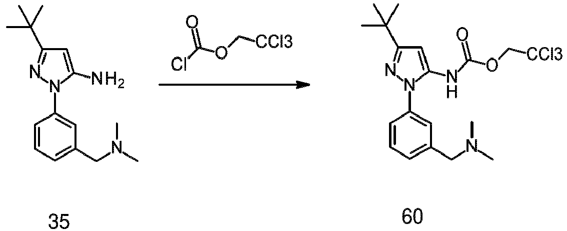

Following the procedure described for compound (49), starting from 5-tert-butyl-2-[3-[(dimethylamino)methyl]phenyl]pyrazol-3-amine (35) (0.20g, 0.73mmol) instead of 3-(tert-butyl)-1-phenyl-1H-pyrazol-5-amine, 2,2,2-trichloroethylchloroformate (0.11mL, 0.81mmol) and pyridine (0.089mL, 1.1mmol) in THF (4mL), 0.360g of 2,2,2-trichloroethyl N-[5-tert-butyl-2-[3-[(dimethylamino)methyl]phenyl]pyrazol-3-yl]carbamate (60) were obtained and used without further purification.

### Synthesis of 2 2,2,2-trichloroethyl N-[5-tert-butyl-2-(2,4-difluorophenyl)pyrazol-3-yl]carbamate (61)

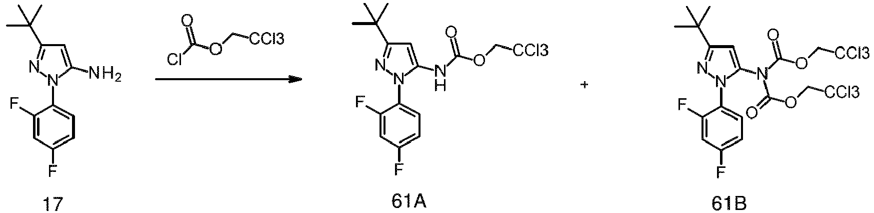

Following the procedure described for compound (49), starting from 5-tert-butyl-2-(2,4-difluorophenyl)pyrazol-3-amine (17) (0.5g, 2.0mmol) instead of 3-(tert-butyl)-1-phenyl-1H-pyrazol-5-amine and 2,2,2-trichloroethylchloroformate (0.55mL, 4.0mmol), 1.26g of a crude mixture of 2,2,2-trichloroethyl N-[5-tert-butyl-2-(2,4-difluorophenyl)pyrazol-3-yl]carbamate (61A) and 2,2,2-trichloroethyl N-[5-tert-butyl-2-(2,4-difluorophenyl)pyrazol-3-yl]-N-(2,2,2-trichloroethoxycarbonyl)carbamate (61B) was obtained and used for the next step without further purification.

### Synthesis of 2,2,2-trichloroethyl N-[5-tert-butyl-2-(3-pyridyl)pyrazol-3-yl]carbamate (62)

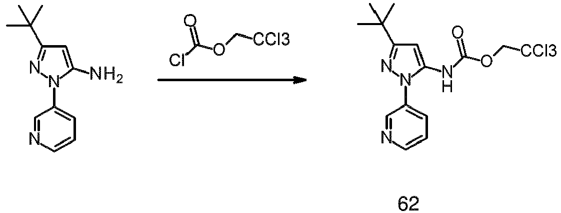

Following the procedure described for compound (49), starting from 5-tert-butyl-2-(3-pyridyl)pyrazol-3-amine (15) (0.15g, 0.69mmol) instead of 3-(tert-butyl)-1-phenyl-1H-pyrazol-5-amine, 2,2,2-trichloroethylchloroformate (0.115mL, 0.83mmol) and pyridine (0.22mL, 2.8mmol) in THF (3mL), 0.270g of 2,2,2-trichloroethyl N-[5-tert-butyl-2-(3-pyridyl)pyrazol-3-yl]carbamate (62) were obtained (Yield: 100%) and used without further purification.

### Synthesis of 2,2,2-trichloroethyl N-[5-tert-butyl-2-[4-[(dimethylamino)methyl]phenyl]pyrazol-3-yllcarbamate (63)

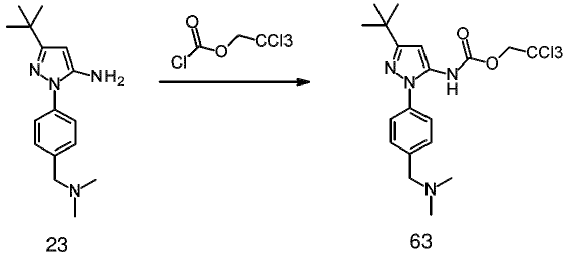

Following the procedure described for compound (49), starting from 5-tert-butyl-2-[4-[(dimethylamino)methyl]phenyl]pyrazol-3-amine (23) (0.1g, 0.3mmol) instead of 3-(tert-butyl)-1-phenyl-1H-pyrazol-5-amine, 2,2,2-trichloroethylchloroformate (0.056mL, 0.4mmol) and pyridine (0.044mL, 0.55mmol) in THF (3mL), 0.170g of crude 2,2,2-trichloroethyl N-[5-tert-butyl-2-[4-[(dimethylamino)methyl]phenyl]pyrazol-3-yl]carbamate (63) were obtained and used without further purification.

### Synthesis of 2,2,2-trichloroethyl N-[5-tert-butyl-2-[3-(morpholinomethyl)phenyl]pyrazol-3-vllcarbamate (64)

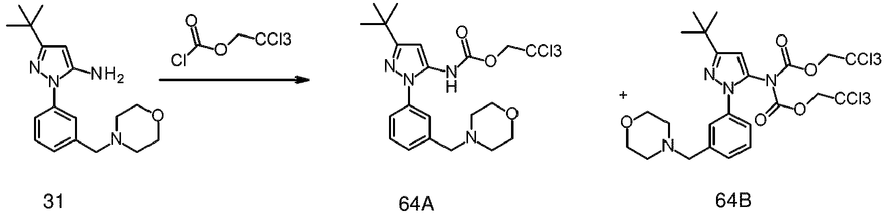

Following the procedure described for compound (49), starting from 5-tert-butyl-2-[3-(morpholinomethyl)methyl]phenyl]pyrazol-3-amine (31) (0.20g, 0.6mmol) instead of 3-(tert-butyl)-1-phenyl-1H-pyrazol-5-amine, 2,2,2-trichloroethylchloroformate (0.096mL, 0.7mmol) and pyridine (0.1mL, 1.2mmol) in THF (8mL), 0.175g of a mixture of 2,2,2-trichloroethyl N-[5-tert-butyl-2-[3-(morpholinomethyl)phenyl]pyrazol-3-yl]carbamate (64A) and 2,2,2-trichloroethyl N-[5-tert-butyl-2-[3-(morpholinomethyl)phenyl]pyrazol-3-yl]-N-(2,2,2-trichloroethoxycarbonyl)carbamate (64B) were obtained after flash chromatography purification.

### Synthesis of 2,2,2-trichloroethyl N-[5-tert-butyl-2-[4-(morpholinomethyl)phenyl]pyrazol-3-vllcarbamate (65)

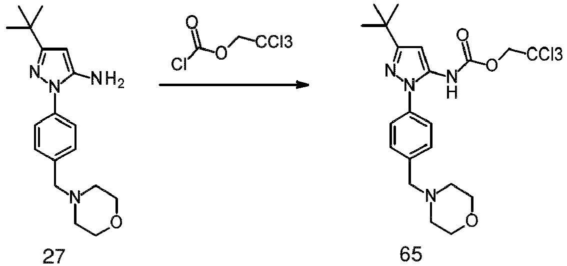

Following the procedure described for compound (49), starting from 5-tert-butyl-2-[4-(morpholinomethyl)methyl]phenyl]pyrazol-3-amine (27) (0.30g, 0.95mmol) instead of 3-(tert-butyl)-1-phenyl-1H-pyrazol-5-amine, 2,2,2-trichloroethylchloroformate (0.144mL, 1.05mmol) and pyridine (0.116mL, 6.6mmol) in THF (9mL), 0.55g of crude 2,2,2-trichloroethyl N-[5-tert-butyl-2-[4-(morpholinomethyl)phenyl]pyrazol-3-yl]carbamate (65) were obtained and used without further purification.

### Synthesis of 5-((4-aminonaphthalen-1-yl)oxy)-3,4-dihydro-1,8-naphthyridin-2(1H)-one (66)

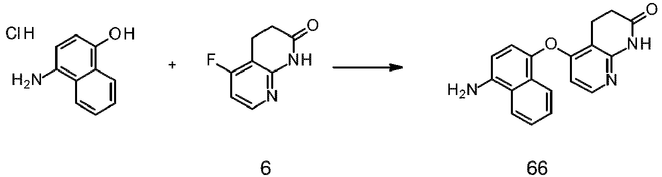

According to scheme 1: In a sealed tube, tBuOK (0.45mg, 4.0mmol) followed by 5-fluoro-3,4-dihydro-1H-1,8-naphthyridin-2-one (6) (0.3g, 1.8mmol) were added at RT to a solution of 4-aminonaphtol hydrochloride (0.53g, 2.7mmol) in dry DMA (15mL). The reaction mixture was stirred at 120°C for 2h. After cooling to room temperature, water (50mL), aq. sat. NH₄Cl (50mL) and AcOEt (100mL) were added. The aqueous layer was extracted with AcOEt (2x30mL). The combined organic fractions were dried over MgSO₄, filtered and concentrated in vacuo. The crude obtained was purified by flash chromatography (SiO₂, cyclohexane/AcOEt /CH₃OH) leading to 0.08g of 5-[(4-amino-1-naphthyl)oxy]-3,4-dihydro-1H-1,8-naphthyridin-2-one (66) as a dark powder.

### Synthesis of 2,2,2-trichloroethyl N-[4-[(3-oxo-4H-pyrido[3,2-b][1,4]oxazin-8-yl)oxyl-2-(trifluoromethyl)phenyl]carbamate (67)

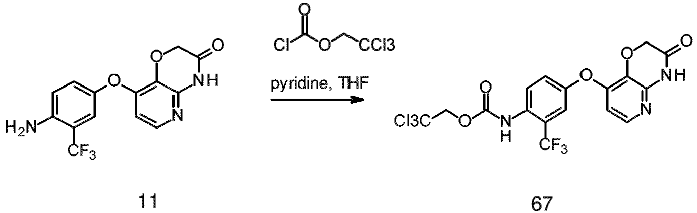

A solution of 8-[4-amino-3-(trifluoromethyl)phenoxy]-2H,3H,4H-pyrido[3,2-b][1,4]oxazin-3-one (11) (2.5g, 7.7mmol) and pyridine (0.74mL, 9.2mmol) in THF (75mL) was cooled down to 0 °C, and next treated with 2,2,2-trichloroethylchloroformate (1.27mL, 9.2mmol). The reaction mixture was stirred overnight at room temperature. The reaction was quenched with water, and extracted with ethyl acetate (2 x 300mL). The solution was dried over MgSO4, filtered, and the solvent was removed in vacuo to give 3.68g (Yield: 96%) of crude material (67) that was used for the next step without further purification.

### Synthesis of 2,2,2-trichloroethyl N-[4-[(7-oxo-6,8-dihydro-5H-1,8-naphthyridin-4-yl)oxyl-2 (trifluoromethyl)phenyl]carbamate (68)

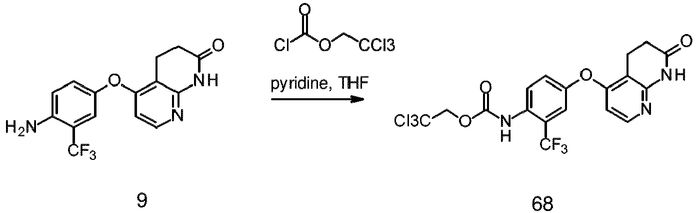

A solution of 5-[4-amino-3-(trifluoromethyl)phenoxy]-3,4-dihydro-1H-1,8-naphthyridin-2-one (9) (0.5g, 1.55mmol) and pyridine (0.19mL, 2.35mmol) in THF was cooled down to 0 °C, and next treated with 2,2,2-trichloroethylchloroformate (0.235mL, 1.7mmol). The reaction mixture was stirred overnight at room temperature. The reaction was quenched with water, and extracted with ethyl acetate. The solution was dried over MgSO4, filtered, and the solvent was removed in vacuo to give 0.9g of crude material (68) that was used for the next step without further purification.

### Synthesis of 2,2,2-trichloroethyl N-[2-methylsulfanyl-4-[(7-oxo-6,8-dihydro-5H-1,8-naphthyridin-4-yl)oxy]phenyl]carbamate (69)

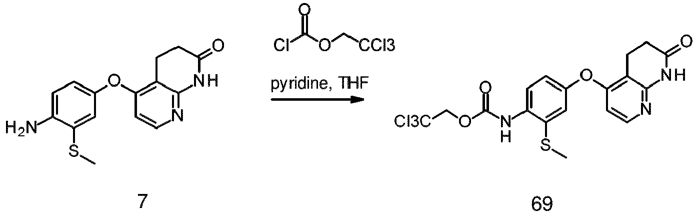

A solution of 5-(4-amino-3-methylsulfanyl-phenoxy)-3,4-dihydro-1H-1,8-naphthyridin-2-one (7) (0.5g, 1.7mmol) and pyridine (0.16mL, 2.0mmol) in THF (20mL) was cooled down to 0 °C, and next treated with 2,2,2-trichloroethylchloroformate (0.25mL, 1.8mmol). The reaction mixture was stirred overnight at room temperature. The reaction was quenched with water, and extracted with ethyl acetate. The solution was dried over MgSO4, filtered, and the solvent was removed in vacuo to give 0.78g of crude material (69) (yield: 95%) that was used for the next step without further purification.

### Synthesis of 2,2,2-trichloroethyl N-[2-methylsulfanyl-4-[(7-oxo-6,8-dihydro-5H-1,8-naphthyridin-4-vl)oxy]pheny]carbamate (70)

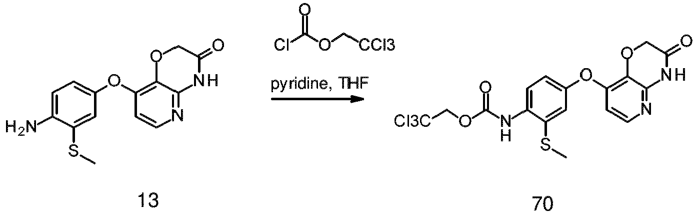

A solution of 8-(4-amino-3-methylsulfanyl-phenoxy)-4H-pyrido[3,2-b][1,4]oxazin-3-one (13) (0.6g, 1.9mmol) and pyridine (0.63mL, 7.9mmol) in THF (20mL) was cooled down to 0 °C, and next treated with 2,2,2-trichloroethylchloroformate (0.320mL, 2.3mmol). The reaction mixture was stirred overnight at room temperature. The reaction was quenched with water, and extracted with ethyl acetate. The solution was dried over MgSO4, filtered, and the solvent was removed in vacuo, the residue was triturated in AcOEt to give 0.6g of crude solid material (70) (yield: 60%) that was used for the next step without further purification.

### Synthesis of compounds of the invention

These detailed descriptions fall within the scope, and serve to exemplify, the above described General Synthetic Procedures, which form part of the invention. These detailed descriptions are presented for illustrative purposes only and are not intended as a restriction on the scope of the invention.

### Example 001

### 1-(3-(tert-butyl)-1-phenyl-1H-pyrazol-5-yl)-3-(2-(methylthio)-4-((7-oxo-5,6, 7,8-tetrahydro-1,8-naphthyridin-4-yl)oxy)phenyl)urea

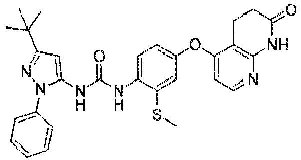

According to method B step2: In a sealed tube containing 5-(4-amino-3-methylsulfanyl-phenoxy)-3,4-dihydro-1H-1,8-naphthyridin-2-one (7) (0.1g, 0.34mmol), DIEA (0.35mL, 2.0mmol) in acetonitrile (3.4mL) was added 2,2,2-trichloroethyl N-(5-tert-butyl-2-phenyl-pyrazol-3-yl)carbamate (48) (0.16g, 0.40mmol). The reaction mixture was stirred at 70°C for 18h to furnish, after filtration and purification by semi-preparative HPLC (elution: water/CH₃CN) 0.047g of the title compound (Yield: 25%) as a pink powder. MS m/z (ES) [M+H]⁺: 543.25, ¹H NMR (300 MHz, DMSO-d₆) δ 10.48 (s, 1H), 8.96 (s, 1H), 8.34 (s, 1H), 7.95 (d, J = 5.7 Hz, 1H), 7.70 (d, J = 8.8 Hz, 1H), 7.52-7.49 (m, 4H), 7.42-7.36 (m, 1H), 7.09 (d, J = 2.6 Hz, 1H), 6.91 (dd, J = 8.8, 2.7 Hz, 1H), 6.34 (s, 1H), 6.29 (d, J = 5.8 Hz, 1H), 2.90 (t, J = 7.3 Hz, 2H), 2.51 (t, J = 7.3 Hz, 2H), 2.40 (s, 3H), 1.26 (s, 9H).

### Example 002

### 1-(3-(tert-butyl)-1-(p-tolyl)-1H-pyrazol-5-yl)-3-(2-(methylthio)-4-((7-oxo-5,6,7,8-tetrahydro-1,8-naphthyridin-4-yl)oxy)phenyl)urea

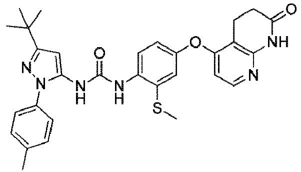

According to method B Step 2: In a sealed tube containing 5-(4-amino-3-methylsulfanyl-phenoxy)-3,4-dihydro-1H-1,8-naphthyridin-2-one (7) (0.095g, 0.31mmol), DIEA (0.30mL, 1.9mmol) in DMSO (3.1mL) was added 2,2,2-trichloroethyl N-[5-tert-butyl-2-(p-toly)pyrazol-3-yl]carbamate (49) (0.19g, 0.47mmol). The reaction mixture was stirred at 70°C for 24h to afford after filtration and semi-preparative HPLC, 0.037g of the title compound as a white solid (Yield: 21%). MS m/z (ES) [M+H]⁺: 557.42. ¹H NMR (300 MHz, DMSO-d₆) δ 10.49 (s, 1H), 8.91 (s, 1H), 8.35 (s, 1H), 7.95 (d, J = 5.7 Hz, 1H), 7.70 (d, J = 8.8 Hz, 1H), 7.38 (d, J = 8.3 Hz, 2H), 7.31 (d, J = 8.5 Hz, 2H), 7.09 (d, J = 2.6 Hz, 1H), 6.91 (dd, J = 8.8, 2.6 Hz, 1H), 6.32 (s, 1H), 6.29 (d, J = 5.8 Hz, 1H), 2.90 (t, J = 7.4 Hz, 2H), 2.51 (t, J = 7.4 Hz, 2H), 2.40 (s, 3H), 2.35 (s, 3H), 1.25 (s, 9H).

### Example 003

### 1-(3-(tert-butyl)-1-(3-chlorophenyl)-1H-pyrazol-5-yl)-3-(2-(methylthio)-4-((7-oxo-5,6,7,8-tetrahydro-1,8-naphthyridin-4-yl)oxy)phenyl)urea

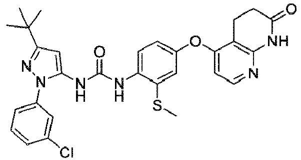

According to method B step 2: In a sealed tube containing 5-(4-amino-3-methylsulfanyl-phenoxy)-3,4-dihydro-1H-1,8-naphthyridin-2-one (7) (0.12g, 0.40mmol), DIEA (0.40mL, 2.40mmol) in DMSO (4mL) was added 2,2,2-trichloroethyl N-[5-tert-butyl-2-(3-chlorophenyl)pyrazol-3-yl]carbamate (51) (0.245g, 0.60mmol). The reaction mixture was stirred at 70°C for 18h to furnish after filtration 0.155g of the title compound (Yield: 67%) as a white solid. MS m/z (ES) [M+H]⁺: 577.33. ¹H NMR (300 MHz, DMSO-d₆) δ 10.49 (s, 1H), 8.99 (s, 1H), 8.33 (s, 1H), 7.95 (d, J = 5.8 Hz, 1H), 7.65 (d, J = 8.8 Hz, 1H), 7.60 (s, 1H), 7.54-7.52 (m, 2H), 7.48-7.42 (m, 1H), 7.09 (d, J = 2.7 Hz, 1H), 6.88 (dd, J = 8.8, 2.6 Hz, 1H), 6.35 (s, 1H), 6.29 (d, J = 5.7 Hz, 1H), 2.90 (t, J = 8.0 Hz, 2H), 2.51 (t, J = 8.0 Hz, 2H), 2.41 (s, 3H), 1.26 (s, 9H).

### Example 004

### 1-(5-(tert-butyl)thiazol-2-yl)-3-(2-(methylthio)-4-((7-oxo-5,6,7,8-tetrahydro-1,8-naphthyridin-4-yl)oxy)phenyl)urea

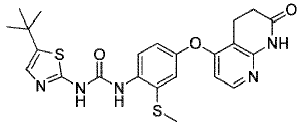

According to method B step 2: In a sealed tube containing 5-(4-amino-3-methylsulfanyl-phenoxy)-3,4-dihydro-1H-1,8-naphthyridin-2-one (7) (0.095g, 0.31mmol), DIEA (0.30mL, 1.9mmol) in DMSO (3.1mL) was added 2,2,2-trichloroethyl N-(5-tert-butylthiazol-2-yl)carbamate (50) (0.16g, 0.47mmol). The reaction mixture was stirred at 70°C for 24h to furnish after filtration 0.115g of the title compound (Yield: 76%) as a beige powder. MS m/z (ES) [M+H]⁺: 484.33. ¹H NMR (300 MHz, DMSO-d₆) δ 10.91 (br s, 1H), 10.49 (s, 1H), 8.58 (br s, 1H), 7.96 (d, J = 5.8 Hz, 1H), 7.84 (d, J = 8.8 Hz, 1H), 7.14 (d, J = 2.7 Hz, 1H), 7.05 (s, 1H), 6.96 (dd, J = 8.8, 2.7 Hz, 1H), 6.32 (d, J = 5.8 Hz, 1H), 2.91 (t, J = 7.4 Hz, 2H), 2.51 (t, J = 8.1 Hz, 2H), 2.44 (s, 3H), 1.29 (s, 9H).

### Example 005

### 1-(2-(methylthio)-4-((7-oxo-5,6,7,8-tetrahydro-1,8-naphthyridin-4-yl)oxy)phenyl)-3-(5-(trifluoromethyl)pyridin-3-yl)urea

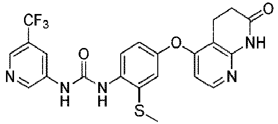

According to method B step 2: In a sealed tube containing 5-(4-amino-3-methylsulfanyl-phenoxy)-3,4-dihydro-1H-1,8-naphthyridin-2-one (7) (0.085g, 0.28mmol), DIEA (0.30mL, 1.9mmol) in DMSO (2.8mL) was added 2,2,2-trichloroethyl N-[5-(trifluoromethyl)-3-pyridyl]carbamate (52) (0.16mg, 0.47mmol). The reaction mixture was stirred at 70°C for 18h to furnish after flash chromatography (SiO₂, cyclohexane/AcOEt/CH₃OH) and semi-preparative HPLC (ACN/water), 0.007g of the title compound (Yield: 5%) as a white solid. MS m/z (ES) [M+H]⁺: 490.25. ¹H NMR (300 MHz, DMSO-d₆) δ 10.50 (s, 1H), 9.86 (s, 1H), 8.74 (d, J = 2.2 Hz, 1H), 8.55 (s, 1H), 8.43 (s, 1H), 8.37 (s, 1H), 7.97 (d, J = 5.8 Hz, 1H), 7.77 (d, J = 8.8 Hz, 1H), 7.14 (d, J = 2.7 Hz, 1H), 6.95 (dd, J = 8.8, 2.7 Hz, 1H), 6.31 (d, J = 5.7 Hz, 1H), 2.91 (t, J = 7.7 Hz, 2H), 2.51 (t, J = 8.1 Hz, 2H), 2.44 (s, 3H).

### Example 006

### 1-(3-(tert-butyl)-1-phenyl-1H-pyrazol-5-yl)-3-(2-(methylthio)-4-((3-oxo-3,4-dihydro-2H-pyrido[3,2-b][1,4]oxazin-8-yl)oxy)phenyl)urea

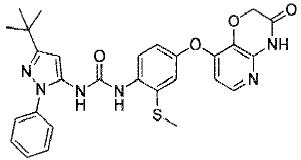

According to method B step 2: In a sealed tube containing 8-(4-amino-3-methylsulfanyl-phenoxy)-4H-pyrido[3,2-b][1,4]oxazin-3-one hydrochloride (13) (0.116mg, 0.38mmol), DIEA (0.40mL, 2.29mmol) in DMSO (3.8mL) was added 2,2,2-trichloroethyl N-(5-tert-butyl-2-phenyl-pyrazol-3-yl)carbamate (48) (0.224g, 0.57mmol). The reaction mixture was stirred at 70°C for 3 days. After filtration and semi-preparative HPLC (ACN/water), 0.063g of the title compound was obtained as a white powder (Yield: 30%). MS m/z (ES) [M+H]⁺: 545.50. ¹H NMR (300 MHz, DMSO-*d₆*) δ 11.30 (s, 1H), 8.93 (s, 1H), 8.32 (s, 1H), 7.77 (d, J = 5.6 Hz, 1H), 7.65 (d, J = 8.8 Hz, 1H), 7.52-7.50 (m, 4H), 7.42-7.37 (m, 1H), 7.06 (d, J = 2.7 Hz, 1H), 6.88 (dd, J = 8.8, 2.7 Hz, 1H), 6.48 (d, J = 5.6 Hz, 1H), 6.33 (s, 1H), 4.63 (s, 2H), 2.40 (s, 3H), 1.25 (s, 9H).

### Example 007

### 1-(5-(tert-butyl)thiazol-2-yl)-3-(4-((7-oxo-5,6,7,8-tetrahydro-1,8-naphthyridin-4-yl)oxy)naphthalen-1-yl)urea

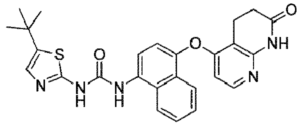

### Synthesis of 5-((4-aminonaphthalen-1-yl)oxy)-3,4-dihydro-1,8-naphthyridin-2(1H)-one (Exemple 007)

According to method B step 2: In a sealed tube containing 5-[(4-amino-1-naphthyl)oxy]-3,4-dihydro-1H-1,8-naphthyridin-2-one (66) (0.120g, 0.2mmol), DIEA (0.30mL, 1.9mmol) in DMSO (2mL) was added 2,2,2-trichloroethyl N-(5-tert-butylthiazol-2-yl)carbamate (50) (0.1mg, 0.3mmol). The reaction mixture was stirred at 70°C for 18h to furnish after flash chromatography (SiO₂, cyclohexane/AcOEt /CH₃OH) and semi-preparative HPLC (ACN/Water), 0.035g of the title compound (Yield: 36%) as a purple solid. MS m/z (ES) [M+H]⁺: 488.33. ¹H NMR (300 MHz, DMSO-d₆) δ 10.70 (br s, 1H), 10.52 (s, 1H), 9.27 (s, 1H), 8.14 (d, J = 8.5 Hz, 1H), 7.92-7.86 (m, 3H), 7.69 (t, J = 7.0 Hz, 1H), 7.59 (t, J = 7.3 Hz, 1H), 7.27 (d, J = 8.4 Hz, 1H), 7.08 (s, 1H), 6.10 (d, J = 5.8 Hz, 1H), 3.07 (t, J = 7.4 Hz, 2H), 2.62 (t, J = 7.5 Hz, 2H), 1.31 (s, 9H).

### Example 008

### 1-(3-(tert-butyl)isoxazol-5-yl)-3-(2-(methylthio)-4-((7-oxo-5,6,7,8-tetrahydro-1,8-naphthyridin-4-yl)oxy)phenyl)urea

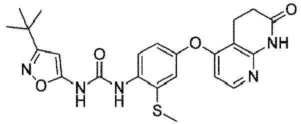

According to method B step 2: In a sealed tube containing 5-(4-amino-3-methylsulfanyl-phenoxy)-3,4-dihydro-1H-1,8-naphthyridin-2-one (7) (0.040g, 0.13mmol), DIEA (0.15ml, 0.8mmol) in DMSO (1.3mL) was added 2,2,2-trichloroethyl N-(3-tert-butylisoxazol-5-yl)carbamate (53) (0.082g, 0.2mmol). The reaction mixture was stirred at 70°C for 18h to furnish, after filtration and semi-preparative HPLC (ACN/Water), 0.018g of the title compound (Yield: 29%) as a beige foam. MS m/z (ES) [M+H]⁺: 468.33. ¹H NMR (300 MHz, DMSO-*d₆*) δ 10.68 (br s, 1H), 10.51 (s, 1H), 8.27 (s, 1H), 7.96 (d, J = 5.8 Hz, 1H), 7.79 (d, J = 8.8 Hz, 1H), 7.15 (d, J = 2.4 Hz, 1H), 6.95 (dd, J = 8.8, 2.5 Hz, 1H), 6.31 (s, 1H), 6.01 (s, 1H), 2.90 (t, J = 7.7 Hz, 2H), 2.51 (t, J = 7.7 Hz, 2H), 2.43 (s, 3H), 1.23 (s, 9H).

### Example 009

### 1-(3-(tert-butyl)isoxazol-5-yl)-3-(4-((7-oxo-5,6,7,8-tetrahydro-1,8-naphthyridin-4-yl)oxy)naphthalen-1-yl)urea

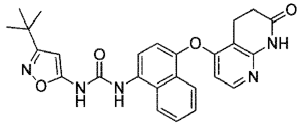

According to method B step 2: In a sealed tube containing 5-[(4-amino-1-naphthyl)oxy]-3,4-dihydro-1H-1,8-naphthyridin-2-one (66) (0.080g, 0.25mmol), DIEA (0.30mL, 1.6mmol) in DMSO (2.5mL) was added 2,2,2-trichloroethyl N-(5-tert-butylthiazol-2-yl)carbamate (53) (0.125g, 0.38mmol). The reaction mixture was stirred at 70°C for 18h to furnish, after flash chromatography (SiO₂, cyclohexane/AcOEt /CH₃OH) and semi-preparative HPLC (ACN/water), 0.012mg of the title compound (Yield: 10%) as a purple powder. MS m/z (ES) [M+H]⁺: 472.33. ¹H NMR (300 MHz, DMSO-*d₆*) δ 10.54 (s, 1H), 10.45 (br s, 1H), 9.02 (s, 1H), 8.11 (d, J = 8.5 Hz, 1H), 7.94-7.87 (m, 3H), 7.69 (t, J = 7.2 Hz, 1H), 7.59 (t, J = 7.6 Hz, 1H), 7.27 (d, J = 8.3 Hz, 1H), 6.11 (d, J = 5.7 Hz, 1H), 6.05 (s, 1H), 3.07 (t, J = 7.5 Hz, 2H), 2.62 (t, J = 7.5 Hz, 2H), 1.24 (s, 9H).

### Example 010

### 1-[5-tert-butyl-2-(p-tolyl)pyrazol-3-yl]-3-[4-[(7-oxo-6,8-dihydro-5H-1,8-naphthyridin-4-yl)oxy]-2-(trifluoromethyl)phenyl]urea

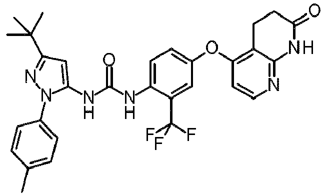

According to method B step 2: 2,2,2-trichloroethyl (3-(tert-butyl)-1-(p-tolyl)-1H-pyrazol-5-yl)carbamate (49) (0.15g, 0.37mmol) and 5-[4-amino-3-(trifluoromethyl)phenoxy]-3,4-dihydro-1H-1,8-naphthyridin-2-one (9) (0.08g, 0.25mmol) were dissolved in a mixture of solvents ACN/DMSO (2mL/0.4mL). To the solution was added DIPEA (0.043mL, 0.25mmol) and the mixture was stirred at 70 °C overnight. The reaction was quenched with water, and the resulting mixture was extracted with ethyl acetate. The combined organic layers were evaporated under reduced pressure. The residue was purified by Preparative TLC then preparative HPLC to give 0.017g of the title compound (yield: 12%). MS m/z (ES) [M+H]⁺: 579.45. ¹H NMR (400 MHz, DMSO-*d₆*) δ 10.55 (s, 1H), 8.98 (s, 1H), 8.43 (s, 1H), 8.01 (d, J = 5.8 Hz, 1H), 7.82 (d, J = 8.8 Hz, 1H), 7.49-7.29 (m, 6H) 6.38 (d, J = 5.8 Hz, 1H), 6.34 (s, 1H), 2.91 (t, J = 7.7 Hz, 2H), 2.57-2.52 (m, 2H), 2.37 (s, 3H), 1.27 (s, 9H).

### Example 011

### 1-[5-tert-butyl-2-(p-tolyl)pyrazol-3-yl]-3-[4-[(3-oxo-4H-pyrido[3,2-b][1,4]oxazin-8-yl)oxy]-2-(trifluoromethyl)phenyl]urea

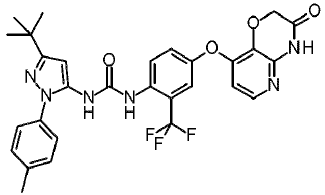

According to method A step 2 : 2,2,2-Trichloroethyl N-[4-({3-oxo-2H,3H,4H-pyrido[3,2-b][1,4]oxazin-8-yl}oxy)-2(trifluoromethyl)-phenyl]carbamate (67) (0.245g, 0.49mmol) and 5-tert-butyl-2-(p-tolyl)pyrazol-3-amine (18) (0.07g, 0.31mmol) were dissolved in a mixture of solvents ACN/DMSO (2mL:2mL). To the solution was added DIPEA (0.053mL, 0.31mmol) and the reaction was stirred overnight at 70 °C. The reaction was quenched with water, and the resulting mixture was extracted with ethyl acetate (2 x 300 mL). The combined organic layers were evaporated under reduced pressure. The residue was purified by Preparative TLC then preparative HPLC to give 0.011g of the title compound (yield: 6%). MS m/z (ES) [M+H]⁺: 581.2. ¹H NMR (400 MHz, DMSO-*d₆*) δ 11.36 (s, 1H), 8.95 (s, 1H), 8.40 (s, 1H), 7.85 (d, J = 5.6 Hz, 1H), 7.76 (d, J = 8.8 Hz, 1H), 7.46-7.28 (m, 6H) 6.63 (d, J = 5.6 Hz, 1H), 6.33 (s, 1H), 4.64 (s, 2H), 2.37 (s, 3H), 1.26 (s, 9H).

### Example 012

### 1-[5-tert-butyl-2-(3-fluorophenyl)pyrazol-3-yl]-3-[2-methylsulfanyl-4-[(3-oxo-4H-pyrido[3,2-b][1,4]oxazin-8-yl)oxy]phenyl]urea

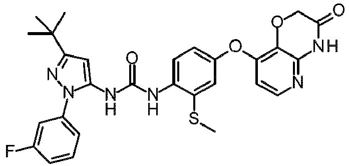

According to method B step 2: 2,2,2-trichloroethyl N-[5-tert-butyl-2-(3-florophenyl)pyrazol-3-yl]carbamate (54) (0.3g, 0.74mmol) and 8-(4-amino-3-methylsulfanyl-phenoxy)-4H-pyrido[3,2-b][1,4]oxazin-3-one (13) (0.15g, 0.49mmol) were dissolved in a mixture of solvents ACN/DMSO (6mL/0.6mL). To the solution was added DIPEA (0.083mL, 0.49mmol) and the mixture was stirred at 70 °C overnight. The reaction was quenched with water, and the resulting mixture was extracted with ethyl acetate. The combined organic layers were evaporated under reduced pressure. The residue was triturated in water, washed with ACN and purified by flash chromatography to give 0.036g of the title compound (yield: 13%). MS m/z (ES) [M+H]⁺: 563.4. ¹H NMR (300 MHz, DMSO-*d₆*) δ 11.30 (s, 1H), 8.98 (s, 1H), 8.34 (s, 1H), 7.80 (d, J = 5.5 Hz, 1H), 7.60 (dd, J = 20.8, 8.8 Hz, 2H), 7.42 (d, *J* = 8.3 Hz, 2H), 7.26 (d, J = 8.2 Hz, 1H), 7.08 (d, J = 2.8 Hz, 1H), 6.90 (dd, J = 8.7, 2.8 Hz, 1H), 6.51 (d, J = 5.5 Hz, 1H), 6.37 (s, 1H), 4.65 (s, 2H), 2.42 (s, 3H), 1.27 (s, 9H).

### Example 013

### 1-[5-tert-butyl-2-(3-fluorophenyl)pyrazol-3-yl]-3-[2-methylsulfanyl-4-[(7-oxo-6,8-dihydro-5H-1,8-naphthyridin-4-yl)oxy]phenyl]urea

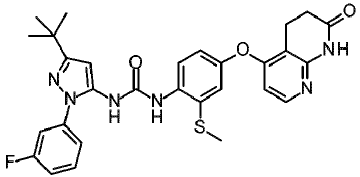

According to method B step 2: 2,2,2-trichloroethyl N-[5-tert-butyl-2-(3-florophenyl)pyrazol-3-yl]carbamate (54) (0.14g, 0.35mmol) and 5-(4-amino-3-methylsulfanyl-phenoxy)-3,4-dihydro-1H-1,8-naphthyridin-2-one (7) (0.07g, 0.23mmol) were dissolved in a mixture of solvents THF/ACN (1mL/1mL). To the solution was added DIPEA (0.12mL, 0.69mmol) and the mixture was stirred at 60 °C overnight. The reaction was quenched with water, and the resulting mixture was extracted with ethyl acetate. The combined organic layers were evaporated under reduced pressure. The residue was purified by flash chromatography to give 0.031g of the title compound (yield: 24%). MS m/z (ES) [M+H]⁺: 561.4. ¹H NMR (300 MHz, DMSO-*d₆*) δ 10.50 (s, 1H), 9.00 (s, 1H), 8.37 (s, 1H), 7.97 (d, J = 5.8 Hz, 1H), 7.68 (d, J = 8.8 Hz, 1H), 7.52 (m, 1H), 7.45 - 7.38 (m, 2H), 7.29 - 7.20 (m, 1H) 7.11 (d, J = 2.7 Hz, 1H), 6.93 (dd, J = 8.8, 2.7 Hz, 1H), 6.37 (s, 1H) 6.32 (d, J = 5.8 Hz, 1H), 2.92 (t, J = 7.7 Hz, 2H), 2.54 (d, J = 8.0 Hz, 2H), 2.43 (s, 3H), 1.28 (s, 9H).

### Example 014

### 1-[5-tert-butyl-2-(3-fluorophenyl)pyrazol-3-yl]-3-[4-[(3-oxo-4H-pyrido[3,2-b][1,4]oxazin-8-yl)oxy]-2-(trifluoromethyl)phenyl]urea

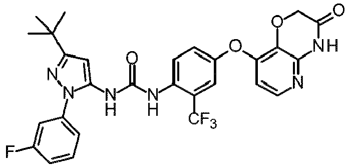

According to method B step 2: 2,2,2-trichloroethyl N-[5-tert-butyl-2-(3-florophenyl)pyrazol-3-yl]carbamate (54) (0.50g, 1.2mmol) and 8-[4-amino-3-(trifluoromethyl)phenoxy]-4H-pyrido[3,2-b][1,4]oxazin-3-one (11) (0.20g, 0.61mmol) were dissolved in a mixture of solvents DMSO/ACN (4mL/4mL). To the solution was added DIPEA (0.107mL, 0.61mmol) and the mixture was stirred at 70 °C overnight. The reaction was quenched with water, and the resulting mixture was extracted with ethyl acetate. The combined organic layers were evaporated under reduced pressure. The residue was purified by flash reverse phase chromatography to give 0.05g of the title compound (yield: 14%). MS m/z (ES) [M+H]⁺: 585.5. ¹H NMR (300 MHz, DMSO-*d₆*) δ 11.37 (s, 1H), 9.01 (s, 1H), 8.41 (s, 1H), 7.85 (d, J = 5.6 Hz, 1H), 7.71 (d, J = 8.9 Hz, 1H), 7.56 (td, J = 8.3, 6.5 Hz, 1H), 7.43 - 7.40 (m, 2H), 7.40 - 7.34 (m, 2H), 7.30 - 7.18 (m, 1H), 6.63 (d, J = 5.6 Hz, 1H), 6.36 (s, 1H), 4.64 (s, 2H), 1.27 (s, 9H).

### Example 015

### 1-[5-tert-butyl-2-(3-fluorophenyl)pyrazol-3-yl]-3-[4-[(7-oxo-6,8-dihydro-5H-1,8-naphthyridin-4-yl)oxy]-2-(trifluoromethyl)phenyl]urea

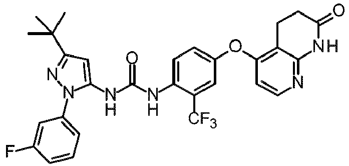

According to method B step 2: 2,2,2-trichloroethyl N-[5-tert-butyl-2-(3-florophenyl)pyrazol-3-yl]carbamate (54) (0.284g, 0.70mmol) and 5-[4-amino-3-(trifluoromethyl)phenoxy]-3,4-dihydro-1H-1,8-naphthyridin-2-one (9) (0.15g, 0.46mmol) were dissolved in a mixture of solvents DMSO/ACN (1mL/4mL). To the solution was added DIPEA (0.081mL, 0.46mmol) and the mixture was stirred at 70 °C overnight. The reaction was quenched with water, and the resulting mixture was extracted with ethyl acetate. The combined organic layers were evaporated under reduced pressure. The residue was purified by flash chromatography then preparative HPLC to give 0.041g of the title compound (yield: 15%). MS m/z (ES) [M+H]⁺: 583.2. ¹H NMR (300 MHz, DMSO-*d₆*) δ 10.55 (s, 1H), 9.04 (s, 1H), 8.44 (s, 1H), 8.01 (d, J = 5.8 Hz, 1H), 7.78 (d, J = 8.9 Hz, 1H), 7.56 (td, J = 8.3, 6.5 Hz, 1H), 7.48 - 7.37 (m, 4H) 7.28 - 7.20 (m, 1H), 6.38 (d, J = 6.8 Hz, 2H), 2.91 (dd, J = 8.5, 6.9 Hz, 2H), 2.54 (d, J = 7.1 Hz, 2H), 1.28 (s, 9H).

### Example 016

### 1-[5-tert-butyl-2-(5-fluoro-3-pyridyl)pyrazol-3-yl]-3-[4-[(7-oxo-6,8-dihydro-5H-1,8-naphthyridin-4-yl)oxy]-2-(trifluoromethyl)phenyl]urea

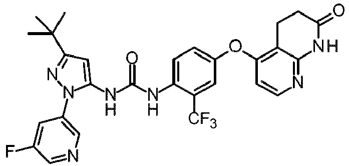

According to method B step 2: 2,2,2-trichloroethyl N-[5-tert-butyl-2-(5-fluoro-3-pyridyl)pyrazol-3-yl]carbamate (55) (0.380g, 0.93mmol) and 5-[4-amino-3-(trifluoromethyl)phenoxy]-3,4-dihydro-1H-1,8-naphthyridin-2-one (9) (0.20g, 0.62mmol) were dissolved in a mixture of solvents DMSO/ACN (2mL/6mL). To the solution was added DIPEA (0.108mL, 0.62mmol) and the mixture was stirred at 70 °C overnight. The reaction was quenched with water, and the resulting mixture was extracted with ethyl acetate. The combined organic layers were evaporated under reduced pressure. The residue was purified by flash chromatography then preparative HPLC to give 0.032g of the title compound (yield: 9%). MS m/z (ES) [M+H]+: 584.2. ¹H NMR (300 MHz, DMSO-*d₆*) δ 10.55 (s, 1H), 9.11 (s, 1H), 8.71 (t, *J* = 1.7 Hz, 1H), 8.63 (d, *J* = 2.6 Hz, 1H), 8.46 (s, 1H), 8.03 - 7.94 (m, 2H), 7.74 (d, J = 8.9 Hz, 1H), 7.50 - 7.38 (m, 2H) 6.44 - 6.35 (m, 2H), 2.91 (dd, J = 8.5, 6.9 Hz, 2H), 2.54 (d, J = 6.9 Hz, 2H), 1.29 (s, 9H).

### Example 017

### 1-[5-tert-butyl-2-(6-methyl-3-pyridyl)pyrazol-3-yl]-3-[4-[(7-oxo-6,8-dihydro-5H-1,8-naphthyridin-4-yl)oxy]-2-(trifluoromethyl)phenyl]urea

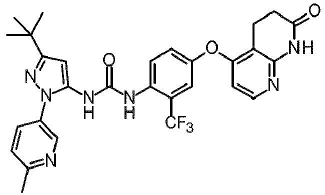

According to method B step 2: 2,2,2-trichloroethyl N-[5-tert-butyl-2-(6-methyl-3-pyridyl)pyrazol-3-yl]carbamate (56A) (0.264g, 0.65mmol) and 5-[4-amino-3-(trifluoromethyl)phenoxy]-3,4-dihydro-1H-1,8-naphthyridin-2-one (9) (0.14g, 0.43mmol) were dissolved in a mixture of solvents DMSO/ACN (1mL/3mL). To the solution was added DIPEA (0.075mL, 0.43mmol) and the mixture was stirred at 70 °C overnight. The reaction was quenched with water, and the resulting mixture was extracted with ethyl acetate. The combined organic layers were evaporated under reduced pressure. The residue was purified by flash chromatography then preparative HPLC to give 0.022g of the title compound (yield: 9%). MS m/z (ES) [M+H]+: 580.2. ¹H NMR (300 MHz, DMSO-*d₆*) δ 10.55 (s, 1H), 9.06 (s, 1H), 8.61 (d, J = 2.6 Hz, 1H), 8.41 (s, 1H), 8.01 (d, *J* = 5.7 Hz, 1H), 7.87 - 7.77 (m, 2H), 7.48 - 7.39 (m, 3H), 6.38 (t, J = 2.9 Hz, 2H), 2.91 (t, J = 7.7 Hz, 2H), 2.55 (m, 5H), 1.27 (s, 9H).

### Example 018

### 1-[5-tert-butyl-2-(5-fluoro-3-pyridyl)pyrazol-3-yl]-3-[2-methylsulfanyl-4-[(3-oxo-4H-pyrido[3,2-b][1,4]oxazin-8-yl)oxy]phenyl]urea

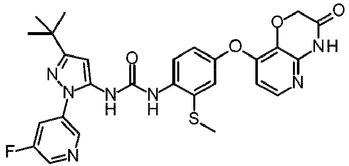

According to method B step 2: 2,2,2-trichloroethyl N-[5-tert-butyl-2-(5-fluoro-3-pyridyl)pyrazol-3-yl]carbamate (55) (0.240g, 0.59mmol) and 8-(4-amino-3-methylsulfanyl-phenoxy)-4H-pyrido[3,2-b][1,4]oxazin-3-one (13) (0.10g, 0.33mmol) were dissolved in a mixture of solvents DMSO/ACN (2mL/2mL). To the solution was added DIPEA (0.058mL, 0.33mmol) and the mixture was stirred at 70 °C overnight. The reaction was quenched with water, and the resulting mixture was extracted with ethyl acetate. The combined organic layers were evaporated under reduced pressure. The residue was purified by flash chromatography to give 0.023g of the title compound (yield: 13%). MS m/z (ES) [M+H]⁺: 586.2. ¹H NMR (300 MHz, DMSO-*d₆*) δ 11.31 (s, 1H), 9.06 (s, 1H), 8.72 (t, *J* = 1.7 Hz, 1H), 8.62 (d, *J* = 2.6 Hz, 1H), 8.35 (s, 1H), 7.99 (dt, J = 9.9, 2.3 Hz, 1H), 7.80 (d, J = 5.6 Hz, 1H), 7.60 (d, J = 8.8 Hz, 1H), 7.08 (d, J = 2.8 Hz, 1H), 6.89 (dd, J = 8.8, 2.8 Hz, 1H) 6.51 (d, J = 5.6 Hz, 1H), 6.41 (s, 1H), 4.65 (s, 2H), 2.42 (s, 3H), 1.29 (s, 9H).

### Example 019

### 1-[5-tert-butyl-2-(6-methyl-3-pyridyl)pyrazol-3-yl]-3-[2-methylsulfanyl-4-[(3-oxo-4H-pyrido[3,2-b][1,4]oxazin-8-yl)oxy]phenyl]urea

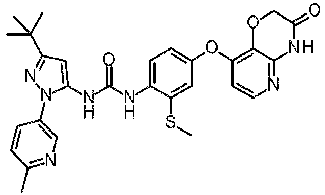

According to method B step 2: 2,2,2-trichloroethyl N-[5-tert-butyl-2-(6-methyl-3-pyridyl)pyrazol-3-yl]carbamate (56A) (0.270g, 0.47mmol) and 8-(4-amino-3-methylsulfanyl-phenoxy)-4H-pyrido[3,2-b][1,4]oxazin-3-one (13) (0.08g, 0.26mmol) were dissolved in a mixture of solvents DMSO/ACN (3mL/3mL). To the solution was added DIPEA (0.046mL, 0.26mmol) and the mixture was stirred at 70 °C overnight. The reaction was quenched with water, and the resulting mixture was extracted with ethyl acetate. The combined organic layers were evaporated under reduced pressure. The residue was purified by flash chromatography to give 0.025g of the title compound (yield: 17%). MS m/z (ES) [M+H]⁺: 560.2. ¹H NMR (300 MHz, DMSO-*d₆*) δ 11.31 (s, 1H), 9.00 (s, 1H), 8.61 (d, J = 2.6 Hz, 1H), 8.31 (s, 1H), 7.84 (dd, J = 8.3, 2.7 Hz, 1H), 7.79 (d, J = 5.6 Hz, 1H), 7.66 (d, J = 8.9 Hz, 1H), 7.43 (d, J = 8.3 Hz, 1H), 7.08 (d, J = 2.8 Hz, 1H), 6.90 (dd, *J* = 8.8, 2.8 Hz, 1H) 6.50 (d, *J* = 5.6 Hz, 1H), 6.37 (s, 1H), 4.65 (s, 2H), 2.54 (s, 3H), 2.42 (s, 3H), 1.27 (s, 9H).

### Example 020

### 1-(3-tert-butylisoxazol-5-yl)-3-[2-methylsulfanyl-4-[(3-oxo-4H-pyrido[3,2-b][1,4]oxazin-8-yl)oxy]phenyl]urea

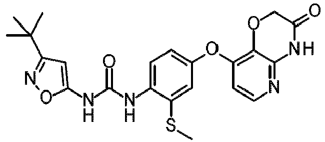

According to method B step 2: 2,2,2-trichloroethyl (3-(tert-butyl)isoxazol-5-yl)carbamate (53) (0.280g, 0.89mmol) and 8-(4-amino-3-methylsulfanyl-phenoxy)-4H-pyrido[3,2-b][1,4]oxazin-3-one (13) (0.15g, 0.49mmol) were dissolved in a mixture of solvents DMSO/ACN (4mL/4mL). To the solution was added DIPEA (0.086mL, 0.49mmol) and the mixture was stirred at 70 °C overnight. The reaction was quenched with water, and the resulting mixture was extracted with ethyl acetate. The combined organic layers were evaporated under reduced pressure. The solid residue was purified by preparative HPLC to give 0.068g of the title compound (yield: 30%). MS m/z (ES) [M+H]⁺: 470.1. ¹H NMR (300 MHz, DMSO-*d₆*) δ 11.32 (s, 1H), 10.66 (s, 1H), 8.24 (s, 1H), 7.81 (d, J = 5.6 Hz, 1H), 7.77 (d, J = 8.8 Hz, 1H), 7.14 (d, J = 2.8 Hz, 1H), 6.94 (dd, *J* = 8.9, 2.8 Hz, 1H), 6.53 (d, *J* = 5.7 Hz, 1H), 6.02 (s, 1H), 4.66 (s, 2H), 2.45 (s, 3H), 1.25 (s, 9H).

### Example 021

### 1-(5-tert-butylisoxazol-3-yl)-3-[2-methylsulfanyl-4-[(3-oxo-4H-pyrido[3,2-b][1,4]oxazin-8-yl)oxy]phenyl]urea

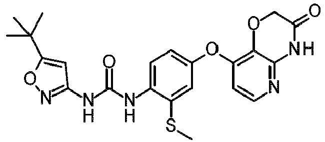

According to method B step 2: 2,2,2-trichloroethyl (5-(tert-butyl)isoxazol-3-yl)carbamate (57) (0.280g, 0.89mmol) and 8-(4-amino-3-methylsulfanyl-phenoxy)-4H-pyrido[3,2-b][1,4]oxazin-3-one (13) (0.15g, 0.49mmol) were dissolved in a mixture of solvents DMSO/ACN (4mL/4mL). To the solution was added DIPEA (0.086mL, 0.49mmol) and the mixture was stirred at 70 °C overnight. The reaction was quenched with water, and the resulting mixture was extracted with ethyl acetate. The combined organic layers were evaporated under reduced pressure. The solid residue was purified by preparative HPLC to give 0.047g of the title compound (yield: 20%). MS m/z (ES) [M+H]⁺: 470.0. ¹H NMR (300 MHz, DMSO-*d₆*) δ 11.31 (s, 1H), 10.08 (s, 1H), 8.41 (s, 1H), 7.83 - 7.76 (m, 2H), 7.12 (d, J = 2.8 Hz, 1H), 6.93 (dd, J = 8.9, 2.8 Hz, 1H), 6.52 (d, J = 5.7 Hz, 1H), 6.42 (s, 1H), 4.66 (s, 2H), 2.45 (s, 3H), 1.29 (s, 9H).

### Example 022

### 1-(5-tert-butyl-2-methyl-pyrazol-3-yl)-3-[2-methylsulfanyl-4-[(3-oxo-4H-pyrido[3,2-b][1,4]oxazin-8-yl)oxy]phenyl]urea

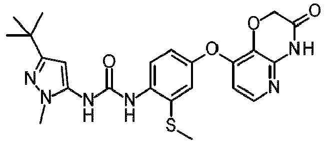

According to method A step 2 : 2,2,2-trichloroethyl N-[2-methylsulfanyl-4-[(7-oxo-6,8-dihydro-5H-1,8-naphthyridin-4-yl)oxy]phenyl]carbamate (69) (0.234g, 0.49mmol) and 5-tert-butyl-2-methyl-pyrazol-3-amine (0.05g, 0.33mmol) were dissolved in a mixture of solvents ACN/DMSO (0.5mL:2mL). To the solution was added DIPEA (0.057mL, 0.33mmol) and the reaction was stirred overnight at 70 °C. The reaction was quenched with water, and the resulting mixture was extracted with ethyl acetate. The combined organic layers were evaporated under reduced pressure. The residue was purified by preparative HPLC to give 0.031g of the title compound (yield: 13%). MS m/z (ES) [M+H]⁺: 483.0. ¹H NMR (300 MHz, DMSO-*d₆*) δ 11.31 (s, 1H), 9.10 (s, 1H), 8.26 (s, 1H), 7.83 - 7.76 (m, 2H), 7.12 (d, J = 2.8 Hz, 1H), 6.92 (dd, J = 5.7, 2.6 Hz, 1H), 6.52 (d, J = 5.7 Hz, 1H), 6.05 (s, 1H), 4.64 (s, 2H), 3.61 (s, 3H), 2.48 (s, 3H), 1.24 (s, 9H).

### Example 023

### 1-(3-tert-butylisoxazol-5-yl)-3-[4-[(7-oxo-6,8-dihydro-5H-1,8-naphthyridin-4-yl)oxy]-2-(trifluoromethyl)phenyl]urea

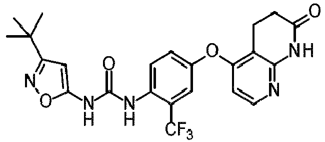

According to method B step 2: 2,2,2-trichloroethyl (3-(tert-butyl)isoxazol-5-yl)carbamate (53) (0.22g, 0.69mmol) and 5-[4-amino-3-(trifluoromethyl)phenoxy]-3,4-dihydro-1H-1,8-naphthyridin-2-one (9) (0.15g, 0.46mmol) were dissolved in a mixture of solvents DMSO/ACN (2mL/0.4mL). To the solution was added DIPEA (0.081mL, 0.46mmol) and the mixture was stirred at 70 °C overnight. The reaction was quenched with water, and the resulting mixture was extracted with ethyl acetate. The combined organic layers were evaporated under reduced pressure. The residue was purified by preparative TLC to give 0.020g of the title compound (yield: 9%). MS m/z (ES) [M+H]⁺: 490.1. ¹H NMR (300 MHz, DMSO-*d₆*) δ 10.70 (br s, 1H), 10.56 (br s, 1H), 8.29 (br s, 1H), 8.02 (d, J = 5.7 Hz, 1H), 7.94 (d, J = 8.9 Hz, 1H), 7.52 - 7.42 (m, 2H), 6.40 (2, J = 5.8 Hz, 1H), 6.03 (s, 1H), 2.92 (dd, J = 8.4, 6.9 Hz, 2H), 2.58 - 2.53 (m, 2H), 1.25 (s, 9H).

### Example 024

### 1-(5-tert-butylisoxazol-3-yl)-3-[4-[(7-oxo-6,8-dihydro-5H-1,8-naphthyridin-4-yl)oxy]-2-(trifluoromethyl)phenyl]urea

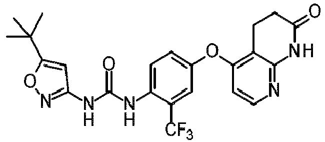

According to method A step 2 : 2,2,2-trichloroethyl N-[4-[(7-oxo-6,8-dihydro-5H-1,8-naphthyridin-4-yl)oxy]-2-(trifluoromethyl)phenyl]carbamate (68) (0.510g, 1.0mmol) and 5-tert-butylisoxazol-3-amine (0.10g, 0.71mmol) were dissolved in a mixture of solvents ACN/DMSO (3mL:3mL). To the solution was added DIPEA (0.125mL, 0.71mmol) and the reaction was stirred overnight at 70 °C. The reaction was quenched with water, and the resulting mixture was extracted with ethyl acetate. The combined organic layers were evaporated under reduced pressure. The residue was purified by Flash chromatography to give 0.120g of the title compound (yield: 35%). MS m/z (ES) [M+H]⁺: 490.1. ¹H NMR (300 MHz, DMSO-*d₆*) δ 10.10 (s, 1H), 9.70 (s, 1H), 8.03 (s, 1H), 7.57 (d, J = 5.8 Hz, 1H), 7.49 (d, *J* = 8.9 Hz, 1H), 7.05 (d, J = 2.8 Hz, 1H), 7.00 (dd, J = 8.9, 2.9 Hz, 1H), 5.98 (s, 1H), 5.95 (d, J = 5.8 Hz, 1H), 2.47 (t, J = 7.7 Hz, 2H), 2.13 - 2.07 (m, 2H), 0.84 (s, 9H).

### Example 026

### 1-(5-tert-butylisoxazol-3-yl)-3-[2-methylsulfanyl-4-[(7-oxo-6,8-dihydro-5H-1,8-naphthyridin-4-yl)oxy]phenyl]urea

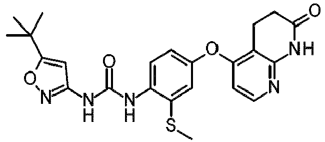

According to method B step 2: 2,2,2-trichloroethyl (5-(tert-butyl)isoxazol-3-yl)carbamate (57) (0.37g, 1.1mmol) and 5-(4-amino-3-methylsulfanyl-phenoxy)-3,4-dihydro-1H-1,8-naphthyridin-2-one (7) (0.20g, 0.66mmol) were dissolved in a mixture of solvents DMSO/ACN (3mL/3mL). To the solution was added DIPEA (0.11mL, 0.66mmol) and the mixture was stirred at 70 °C overnight. The reaction was quenched with water, and the resulting mixture was extracted with ethyl acetate. The combined organic layers were evaporated under reduced pressure. The residue was purified by flash chromatography to give 0.100g of the title compound (yield: 32%). MS m/z (ES) [M+H]⁺: 468.1. ¹H NMR (300 MHz, DMSO-*d₆*) δ 10.51 (s, 1H), 10.11 (s, 1H), 8.44 (s, 1H), 7.99 (d, J = 5.7 Hz, 1H), 7.85 (d, J = 8.8 Hz, 1H), 7.17 (d, J = 2.8 Hz, 1H), 6.98 (dd, J = 8.8, 2.7 Hz, 1H), 6.44 (s, 1H) 6.34 (d, J = 5.7 Hz, 1H), 2.94 (t, J = 7.7 Hz, 2H), 2.56 - 2.54 (m, 2H), 2.46 (s, 3H), 1.30 (s, 9H).

### Example 027

### 1-[5-tert-butyl-2-(5-fluoro-3-pyridyl)pyrazol-3-yl]-3-[2-methylsulfanyl-4-[(7-oxo-6,8-dihydro-5H-1,8-naphthyridin-4-yl)oxy]phenyl]urea

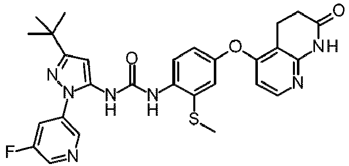

According to method A step 2 : 2,2,2-trichloroethyl N-[2-methylsulfanyl-4-[(7-oxo-6,8-dihydro-5H-1,8-naphthyridin-4-yl)oxy]phenyl]carbamate (69) (0.24g, 0.51mmol) and 5-tert-butyl-2-(5-fluoro-3-pyridyl)pyrazol-3-amine (15) (0.08g, 0.34mmol) were dissolved in a mixture of solvents ACN/DMSO (2mL:2mL). To the solution was added DIPEA (0.06mL, 0.34mmol) and the reaction was stirred overnight at 70 °C. The reaction was quenched with water, and the resulting mixture was extracted with ethyl acetate. The combined organic layers were evaporated under reduced pressure. The residue was purified by Flash chromatography to give 0.040g of the title compound (yield: 21%). MS m/z (ES) [M+H]⁺: 562.2. ¹H NMR (400 MHz, DMSO-*d₆*) δ 10.50 (s, 1H), 9.09 (s, 1H), 8.73 (t, J = 1.6 Hz, 1H), 8.64 (d, J = 2.6 Hz, 1H), 8.39 (s, 1H), 8.02 - 7.97 (m, 2H), 7.66 (d, J = 8.8 Hz, 1H), 7.12 (d, J = 2.7 Hz, 1H), 6.93 (dd, J = 8.8, 2.7 Hz, 1H), 6.43 (s, 1H) 6.33 (d, J = 5.8 Hz, 1H), 2.93 (t, J = 7.7 Hz, 2H), 2.56 (s, 2H), 2.44 (s, 3H), 1.30 (s, 9H).

### Example 028

### 1-[5-tert-butyl-2-[3-[(dimethylamino)methyl]phenyl]pyrazol-3-yl]-3-[4-[(7-oxo-6,8-dihydro-5H-1,8-naphthyridin-4-yl)oxy]-2-(trifluoromethyl)phenyl]urea

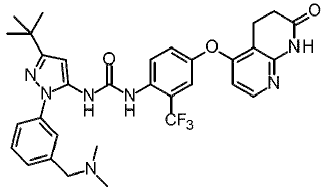

According to method A step 2 : 2,2,2-trichloroethyl N-[4-[(7-oxo-6,8-dihydro-5H-1,8-naphthyridin-4-yl)oxy]-2-(trifluoromethyl)phenyl]carbamate (68) (0.22g, 0.44mmol) and 5-tert-butyl-2-[3-[(dimethylamino)methyl]phenyl]pyrazol-3-amine (35) (0.08g, 0.29mmol) were dissolved in a mixture of solvents ACN/DMSO (1.5mL:1.5mL). To the solution was added DIPEA (0.051mL, 0.29mmol) and the reaction was stirred overnight at 70 °C. The reaction was quenched with water, and the resulting mixture was extracted with ethyl acetate. The combined organic layers were evaporated under reduced pressure. The residue was purified by Flash chromatography to give 0.021g of the title compound (yield: 12%). MS m/z (ES) [M+H]⁺: 622.2. ¹H NMR (400 MHz, DMSO-*d₆*) δ 10.55 (s, 1H), 9.02 (s, 1H), 8.39 (s, 1H), 8.02 (d, *J* = 5.7 Hz, 1H), 7.85 (d, J = 8.9 Hz, 1H), 7.61 - 7.28 (m, 6H), 6.39 (d, J = 5.7 Hz, 1H), 6.35 (s, 1H), 3.46 (s, 2H), 2.92 (d, J = 7.7 Hz, 2H), 2.56 (s, 2H), 2.17 (s, 6H), 1.29 (s, 9H).

### Example 029

### 1-[5-tert-butyl-2-(2,4-difluorophenyl)pyrazol-3-yl]-3-[4-[(7-oxo-6,8-dihydro-5H-1,8-naphthyridin-4-yl)oxy]-2-(trifluoromethyl)phenyl]urea

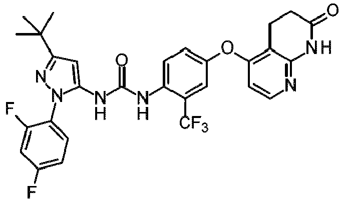

According to method A step 2 : 2,2,2-trichloroethyl N-[4-[(7-oxo-6,8-dihydro-5H-1,8-naphthyridin-4-yl)oxy]-2-(trifluoromethyl)phenyl]carbamate (68) (0.238g, 0.48mmol) and 5-tert-butyl-2-(2,4-difluorophenyl)pyrazol-3-amine (17) (0.08g, 0.32mmol) were dissolved in a mixture of solvents ACN/DMSO (2mL:2mL). To the solution was added DIPEA (0.055mL, 0.32mmol) and the reaction was stirred overnight at 70 °C. The reaction was quenched with water, and the resulting mixture was extracted with ethyl acetate. The combined organic layers were evaporated under reduced pressure. The residue was purified by Flash chromatography then preparative HPLC to give 0.042g of the title compound (yield: 22%). MS m/z (ES) [M+H]⁺: 601.1. ¹H NMR (400 MHz, DMSO-*d₆*) δ 10.55 (s, 1H), 8.99 (s, 1H), 8.32 (s, 1H), 8.02 (d, J = 5.8 Hz, 1H), 7.78 (d, J = 8.9 Hz, 1H), 7.70 - 7.54 (m, 2H), 7.50 - 7.38 (m, 2H), 7.37 - 7.24 (m, 1H), 6.45 - 6.31 (m, 2H), 2.92 (t, J = 7.7 Hz, 2H), 2.56 - 2.54 (m 2H), 1.26 (s, 9H).

### Example 030

### 1-[5-tert-butyl-2-(3-pyridyl)pyrazol-3-yl]-3-[4-[(7-oxo-6,8-dihydro-5H-1,8-naphthyridin-4-yl)oxy]-2-(trifluoromethyl)phenyl]urea

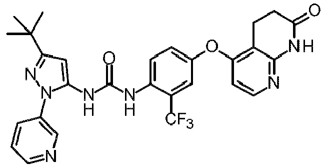

According to method B step 2: 2,2,2-trichloroethyl N-[5-tert-butyl-2-(3-pyridyl)pyrazol-3-yl]carbamate (62) (0.455g, 1.16mmol) and 5-[4-amino-3-(trifluoromethyl)phenoxy]-3,4-dihydro-1H-1,8-naphthyridin-2-one (9) (0.25g, 0.77mmol) were dissolved in a mixture of solvents DMSO/ACN (3mL/9mL). To the solution was added DIPEA (0.229mL, 1.32mmol) and the mixture was stirred at 70 °C for 2 days. The reaction was quenched with water, and the resulting mixture was extracted with ethyl acetate. The combined organic layers were evaporated under reduced pressure. The residue was purified by flash chromatography then preparative HPLC to give 0.018g of the title compound (yield: 4%). MS m/z (ES) [M+H]⁺: 566.2. ¹H NMR (400 MHz, DMSO-*d₆*) δ 10.57 (s, 1H), 9.11 (s, 1H), 8.80 (d, J = 2.6 Hz, 1H), 8.62 (dd, J = 4.8,1.5 Hz, 1H), 8.44 (s, 1H), 8.04 - 7.99 (m, 2H), 7.78 (d, J = 8.9 Hz, 1H), 7.61 (dd, J = 8.2, 4.8 Hz, 1H),7.49 - 7.39 (m, 2H), 6.42 - 6.38 (m, 2H), 2.92 (t, J = 7.7 Hz, 2H), 2.58 - 2.52 (m, 2H), 1.29 (s, 9H).

### Example 031

### 1-[5-tert-butyl-2-(2,4-difluorophenyl)pyrazol-3-yl]-3-[2-methylsulfanyl-4-[(3-oxo-4H-pyrido[3,2-b][1,4]oxazin-8-yl)oxy]phenyl]urea

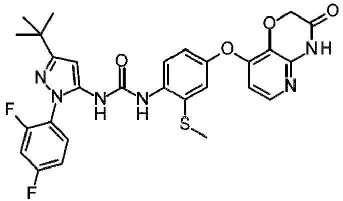

According to method B step 2: 2,2,2-trichloroethyl N-[5-tert-butyl-2-(2,4-difluorophenyl)pyrazol-3-yl]carbamate (61A) (0.298g, 0.49mmol) and 8-(4-amino-3-methylsulfanyl-phenoxy)-4H-pyrido[3,2-b][1,4]oxazin-3-one (13) (0.10g, 0.33mmol) were dissolved in a mixture of solvents DMSO/ACN (1mL/3mL). To the solution was added DIPEA (0.057mL, 0.33mmol) and the mixture was stirred at 70 °C overnight. The reaction was quenched with water, and the resulting mixture was extracted with ethyl acetate. The combined organic layers were evaporated under reduced pressure. The solid residue was purified by flash chromatography then preparative HPLC to give 0.015g of the title compound (yield: 8%). MS m/z (ES) [M+H]⁺: 581.2. ¹H NMR (300 MHz, DMSO-*d₆*) δ 11.31 (s, 1H), 8.96 (s, 1H), 8.25 (s, 1H), 7.78 (d, J = 5.6 Hz, 1H), 7.65 - 7.50 (m, 3H), 7.25 (t, J = 5.6 Hz, 1H), 7.10 (s, 1H), 6.89 (dd, J = 8.8, 2.8 Hz, 1H) 6.51 (d, J = 5.6 Hz, 1H), 6.41 (s, 1H), 4.65 (s, 2H), 2.42 (s, 3H), 1.24 (s, 9H).

### Example 032

### 1-[5-tert-butyl-2-(6-methyl-3-pyridyl)pyrazol-3-yl]-3-[2-methylsulfanyl-4-[(7-oxo-6,8-dihydro-5H-1,8-naphthyridin-4-yl)oxy]phenyl]urea

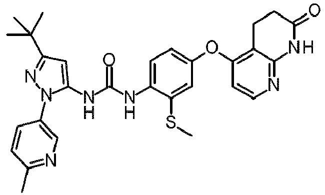

According to method A step 2 : 2,2,2-trichloroethyl N-[2-methylsulfanyl-4-[(7-oxo-6,8-dihydro-5H-1,8-naphthyridin-4-yl)oxy]phenyl]carbamate (69) (0.32g, 0.68mmol) and 5-tert-butyl-2-(6-methyl-3-pyridyl)pyrazol-3-amine (16) (0.10g, 0.46mmol) were dissolved in a mixture of solvents ACN/DMSO (3mL:3mL). To the solution was added DIPEA (0.08mL, 0.46mmol) and the reaction was stirred overnight at 70 °C. The reaction was quenched with water, and the resulting mixture was extracted with ethyl acetate. The combined organic layers were evaporated under reduced pressure. The residue was purified by Flash chromatography then preparative HPLC to give 0.035g of the title compound (yield: 14%). MS m/z (ES) [M+H]⁺: 558.2. ¹H NMR (400 MHz, DMSO-*d₆*) δ 10.50 (s, 1H), 9.02 (s, 1H), 8.63 (s, 1H), 8.35 (s, 1H), 7.98 (d, J = 5.8 Hz, 1H), 7.85 (dd, J = 8.3, 2.7 Hz, 1H), 7.72 (d, J = 8.8 Hz, 1H), 7.44 (d, J = 8.3 Hz, 1H), 7.12 (d, J = 2.7 Hz, 1H), 6.94 (dd, J = 8.8, 2.7 Hz, 1H), 6.39 (s, 1H), 6.33 (d, J = 5.8 Hz, 1H), 2.93 (t, J = 7.7 Hz, 2H), 2.55 (s, 3H), 2.54 (d, *J* = 1.3 Hz, 2H), 2.43 (s, 3H), 1.29 (s, 9H).

### Example 033

### 1-[5-tert-butyl-2-[4-[(dimethylamino)methyl]phenyl]pyrazol-3-yl]-3-[2-methylsulfanyl-4-[(3-oxo-4H-pyrido[3,2-b][1,4]oxazin-8-yl)oxy]phenyl]urea

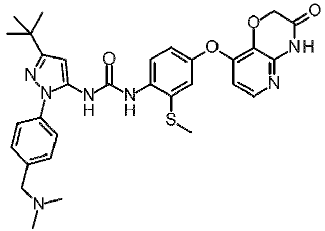

According to method B step 2: 2,2,2-trichloroethyl N-[5-tert-butyl-2-[4-[(dimethylamino)methyl]phenyl]pyrazol-3-yl]carbamate (63) (0.177g, 0.40mmol) and 8-(4-amino-3-methylsulfanyi-phenoxy)-4H-pyrido[3,2-b][1,4]oxazin-3-one (13) (0.08g, 0.26mmol) were dissolved in a mixture of solvents DMSO/ACN (2mL/0.5mL). To the solution was added DIPEA (0.046mL, 0.26mmol) and the mixture was stirred at 70 °C overnight. The reaction was quenched with water, and the resulting mixture was extracted with ethyl acetate. The combined organic layers were evaporated under reduced pressure. The solid residue was purified by flash chromatography to give 0.041g of the title compound (yield: 26%). MS m/z (ES) [M+H]⁺: 602.1. ¹H NMR (400 MHz, DMSO-*d₆*) δ 11.31 (s, 1H), 8.94 (s, 1H), 8.33 (s, 1H), 7.81 (d, *J* = 5.7 Hz, 1H), 7.67 (d, *J* = 8.8 Hz, 1H), 7.48 (d, J = 8.5 Hz, 2H), 7.43 (d, *J* = 8.6 Hz, 2H), 7.09 (d, J = 2.8 Hz, 1H), 6.90 (dd, J = 8.8, 2.8 Hz, 1H) 6.51 (d, J = 5.6 Hz, 1H), 6.35 (s, 1H), 4.66 (s, 2H), 3.44 (s, 2H), 2.42 (s, 3H), 2.19 (s, 6H), 1.28 (s, 9H).

### Example 034

### 1-[5-tert-butyl-2-[4-[(dimethylamino)methyl]phenyl]pyrazol-3-yl]-3-[4-[(7-oxo-6,8-dihydro-5H-1,8-naphthyridin-4-yl)oxy]-2-(trifluoromethyl)phenyl]urea

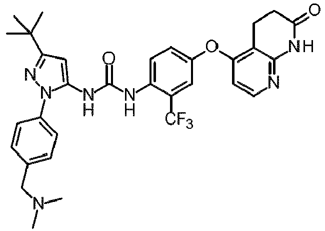

According to method A step 2 : 2,2,2-trichloroethyl N-[4-[(7-oxo-6,8-dihydro-5H-1,8-naphthyridin-4-yl)oxy]-2-(trifluoromethyl)phenyl]carbamate (68) (0.22g, 0.44mmol) and 5-tert-butyl-2-[4-[(dimethylamino)methyl]phenyl]pyrazol-3-amine (23) (0.08g, 0.29mmol) were dissolved in a mixture of solvents ACN/DMSO (1.5mL:1.5mL). To the solution was added DIPEA (0.08mL, 0.46mmol) and the reaction was stirred overnight at 70 °C. The reaction was quenched with water, and the resulting mixture was extracted with ethyl acetate. The combined organic layers were evaporated under reduced pressure. The residue was purified by Flash chromatography then preparative HPLC to give 0.018g of the title compound (yield: 10%). MS m/z (ES) [M+H]⁺: 622.2. ¹H NMR (400 MHz, DMSO-*d₆*) δ 10.55 (s, 1H), 9.02 (s, 1H), 8.43 (s, 1H), 8.02 (d, J = 5.8 Hz, 1H), 7.81 (d, J = 8.9 Hz, 1H), 7.52 - 7.40 (m, 6H), 6.40 - 6.33 (m, 2H), 3.44 (s, 2H), 2.92 (t, J = 7.7 Hz, 2H), 2.56 (s, 2H), 2.19 (s, 6H), 1.28 (s, 9H).

### Example 035

### 1-[5-tert-butyl-2-[3-(morpholinomethyl)phenyl]pyrazol-3-yl]-3-[2-methylsulfanyl-4-[(3-oxo-4H-pyrido[3,2-b][1,4]oxazin-8-yl)oxy]phenyl]urea

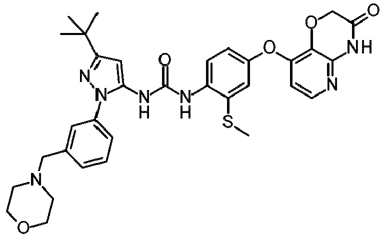

According to method A step 2 : 2,2,2-trichloroethyl N-[2-methylsulfanyl-4-[(3-oxo-4H-pyrido[3,2-b][1,4]oxazin-8-yl)oxy]phenyl]carbamate (70) (0.228g, 0.48mmol) and 5-tert-butyl-2-[3-(morpholinomethyl)methyl]phenyl]pyrazol-3-amine (31) (0.10g, 032mmol) were dissolved in a mixture of solvents ACN/DMSO (0.5mL:2mL). To the solution was added DIPEA (0.055mL, 0.32mmol) and the reaction was stirred overnight at 70 °C. The reaction was quenched with water, and the resulting mixture was extracted with ethyl acetate. The combined organic layers were evaporated under reduced pressure. The residue was purified by Flash chromatography to give 0.051g of the title compound (yield: 25%). MS m/z (ES) [M+H]⁺: 644.2. ¹H NMR (400 MHz, DMSO-*d₆*) δ 11.31 (s, 1H), 8.96 (s, 1H), 8.28 (s, 1H), 7.80 (d, *J* = 5.6 Hz, 1H), 7.71 (d, J = 8.8 Hz, 1H), 7.51 - 7.41 (m, 3H), 7.34 (dt, J = 7.4, 1.5 Hz, 1H ), 7.10 (d, J = 2.8 Hz, 1H) 6.91 (dd, J = 8.9, 2.7 Hz, 1H), 6.50 (d, J = 5.6 Hz, 1H), 6.35 (s, 1H), 4.66 (s, 2H), 3.54 (m, 6H), 2.43 (s, 3H), 2.38 (m, 4H), 1.29 (s, 9H).

### Example 037

### 1-(3-tert-butylisoxazol-5-yl)-3-[4-[(3-oxo-4H-pyrido[3,2-b][1,4]oxazin-8-yl)oxy]-2-(trifluoromethyl)phenyl]urea

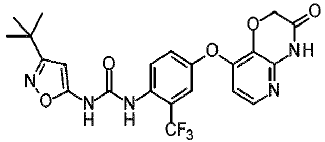

According to method B step 2: 2,2,2-trichloroethyl (3-(tert-butyl)isoxazol-5-yl)carbamate (53) (0.27g, 0.89mmol) and 8-[4-amino-3-(trifluoromethyl)phenoxy]-4H-pyrido[3,2-b][1,4]oxazin-3-one (11) (0.16g, 0.49mmol) were dissolved in a mixture of solvents DMSO/ACN (3mL/3mL). To the solution was added DIPEA (0.086mL, 0.49mmol) and the mixture was stirred at 70 °C overnight. The reaction was quenched with water, and the resulting mixture was extracted with ethyl acetate. The combined organic layers were evaporated under reduced pressure. The residue was purified by flash chromatography then flash reverse phase chromatography to give 0.017g of the title compound (yield: 7%). MS m/z (ES) [M+H]⁺: 492.1. ¹H NMR (300 MHz, DMSO-*d₆*) δ 11.38 (s, 1H), 10.65 (s, 1H), 8.29 (s, 1H), 7.92 - 7.81 (m, 2H), 7.46 (d, J = 2.9 Hz, 1H), 7.41 (dd, J = 8.9, 2.9 Hz, 1H), 6.67 (d, J = 5.6 Hz, 1H), 6.04 (s, 1H), 4.65 (s, 2H), 1.25 (s, 9H).

### Example 038

### 1-[5-tert-butyl-2-[4-(morpholinomethyl)phenyl]pyrazol-3-yl]-3-[2-methylsulfanyl-4-[(3-oxo-4H-pyrido[3,2-b][1,4]oxazin-8-yl)oxy]phenyl]urea

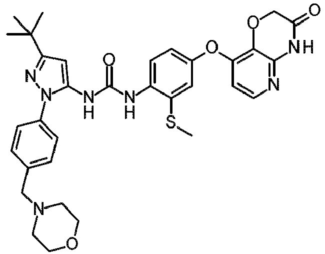

According to method B step 2: 2,2,2-trichloroethyl N-[5-tert-butyl-2-[4-( morpholinomethyl)phenyl]pyrazol-3-yl]carbamate (63) (0.21g, 0.45mmol) and 8-(4-amino-3-methylsulfanyl-phenoxy)-4H-pyrido[3,2-b][1,4]oxazin-3-one (13) (0.09g, 0.30mmol) were dissolved in a mixture of solvents DMSO/ACN (2mL/2mL). To the solution was added DIPEA (0.052mL, 0.30mmol) and the mixture was stirred at 70 °C overnight. The reaction was quenched with water, and the resulting mixture was extracted with ethyl acetate. The combined organic layers were evaporated under reduced pressure. The solid residue was purified by flash chromatography to give 0.035g of the title compound (yield: 18%). MS m/z (ES) [M+H]⁺: 644.2. ¹H NMR (400 MHz, DMSO-*d₆*) δ 11.31 (s, 1H), 8.94 (s, 1H), 8.32 (s, 1H), 7.80 (d, J = 5.7 Hz, 1H), 7.67 (d, J = 8.8 Hz, 1H), 7.54 - 7.41 (m, 4H), 7.09 (d, J = 2.8 Hz, 1H), 6.90 (dd, J = 8.8, 2.8 Hz, 1H) 6.51 (d, J = 5.6 Hz, 1H), 6.35 (s, 1H), 4.66 (s, 2H), 3.59 (t, J = 4.6 Hz, 4H), 3.52 (s, 2H), 2.42 (s, 3H), 2.40 (d, J = 4.7 Hz, 4H), 1.28 (s, 9H).

### Example 039

### 1-[5-tert-butyl-2-(5-fluoro-3-pyridyl)pyrazol-3-yl]-3-[4-[(3-oxo-4H-pyrido[3,2-b][1,4]oxazin-8-yl)oxy]-2-(trifluoromethyl)phenyl]urea

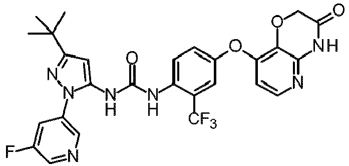

According to method A step 2 : 2,2,2-trichloroethyl N-[4-[(3-oxo-4H-pyrido[3,2-b][1,4]oxazin-8-yl)oxy]-2-(trifluoromethyl)phenyl]carbamate (67) (0.256g, 0.51mmol) and 5-tert-butyl-2-(5-fluoro-3-pyridyl)pyrazol-3-amine (15) (0.08g, 0.34mmol) were dissolved in a mixture of solvents ACN/DMSO (2mL:2mL). To the solution was added DIPEA (0.06mL, 0.34mmol) and the reaction was stirred overnight at 70 °C. The reaction was quenched with water, and the resulting mixture was extracted with ethyl acetate. The combined organic layers were evaporated under reduced pressure. The residue was purified by Flash chromatography to give 0.039g of the title compound (yield: 20%). MS m/z (ES) [M+H]⁺: 586.2. ¹H NMR (400 MHz, DMSO-*d₆*) δ 11.37 (s, 1H), 9.07 (s, 1H), 8.71 (t, J = 1.7 Hz, 1H), 8.62 (d, J = 2.6 Hz, 1H), 8.44 (s, 1H), 7.96 (dt, J = 10.0, 2.3 Hz, 1H), 7.85 (d, J = 5.6 Hz, 1H), 7.67 (d, J = 8.9 Hz, 1H), 7.42 - 7.33 (m, 2H), 6.64 (d, *J* = 5.6 Hz, 1H), 6.41 (s, 1H), 4.64 (s, 2H), 1.29 (s, 9H).

### Example 040

### 1-[5-tert-butyl-2-(6-methyl-3-pyridyl)pyrazol-3-yl]-3-[4-[(3-oxo-4H-pyrido[3,2-b][1,4]oxazin-8-yl)oxy]-2-(trifluoromethyl)phenyl]urea

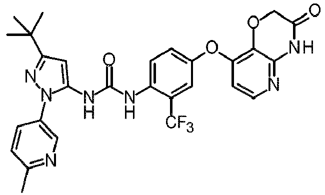

According to method A step 2 : 2,2,2-trichloroethyl N-[4-[(3-oxo-4H-pyrido[3,2-b][1,4]oxazin-8-yl)oxy]-2-(trifluoromethyl)phenyl]carbamate (67) (0.261g, 0.52mmol) and 5-tert-butyl-2-(6-methyl-3-pyridyl)pyrazol-3-amine (16) (0.08g, 0.35mmol) were dissolved in a mixture of solvents ACN/DMSO (2mL:2mL). To the solution was added DIPEA (0.06mL, 0.35mmol) and the reaction was stirred overnight at 70 °C. The reaction was quenched with water, and the resulting mixture was extracted with ethyl acetate. The combined organic layers were evaporated under reduced pressure. The residue was purified by Flash chromatography to give 0.034g of the title compound (yield: 17%). MS m/z (ES) [M+H]⁺: 582.1. ¹H NMR (400 MHz, DMSO-*d₆*) δ 11.37 (s, 1H), 9.03 (s, 1H), 8.62 (d, J = 2.6 Hz, 1H), 8.39 (s, 1H), 7.89 - 7.81 (m, 2H), 7.74 (d, *J* = 8.9 Hz, 1H), 7.46 - 7.35 (m, 3H), 6.64 (d, *J* = 5.7 Hz, 1H), 6.38 (s, 1H), 4.65 (s, 2H), 2.55 (s, 3H), 1.28 (s, 9H).

### Example 041

### 1-[5-tert-butyl-2-[3-(dimethylaminomethyl)phenyl]pyrazol-3-yl]-3-[2-methylsulfanyl-4-[(3-oxo-4H-pyrido[3,2-b][1,4]oxazin-8-yl)oxy]phenyl]urea

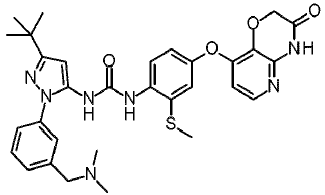

According to method B step 2: 2,2,2-trichloroethyl N-[5-tert-butyl-2-[3-[(dimethylamino)methyl]phenyl]pyrazol-3-yl]carbamate (60) (0.31g, 0.69mmol) and 8-(4-amino-3-methylsulfanyl-phenoxy)-4H-pyrido[3,2-b][1,4]oxazin-3-one (13) (0.14g, 0.46mmol) were dissolved in a mixture of solvents DMSO/ACN (2mL/2mL). To the solution was added DIPEA (0.088mL, 0.51mmol) and the mixture was stirred at 70 °C overnight. The reaction was quenched with water, and the resulting mixture was extracted with ethyl acetate. The combined organic layers were evaporated under reduced pressure. The solid residue was purified by flash chromatography to give 0.039g of the title compound (yield: 14%). MS m/z (ES) [M+H]⁺: 602.2. ¹H NMR (400 MHz, DMSO-*d₆*) δ 11.30 (s, 1H), 8.94 (s, 1H), 8.29 (s, 1H), 7.80 (d, J = 5.7 Hz, 1H), 7.69 (d, J = 8.8 Hz, 1H), 7.50 - 7.40 (m, 3H), 7.32 (dd, J = 7.3, 1.5 Hz, 1H), 7.09 (d, J = 2.8 Hz, 1H), 6.91 (dd, J = 8.8, 2.8 Hz, 1H), 6.51 (d, J = 5.6 Hz, 1H), 6.34 (s, 1H), 4.66 (s, 2H), 3.45 (s, 2H), 2.42 (s, 3H), 2.17 (s, 6H), 1.28 (s, 9H).

### Example 042

### 1-[5-tert-butyl-2-[4-(morpholinomethyl)phenyl]pyrazol-3-yl]-3-[2-methylsulfanyl-4-[(7-oxo-6,8-dihydro-5H-1,8-naphthyridin-4-yl)oxy]phenyl]urea

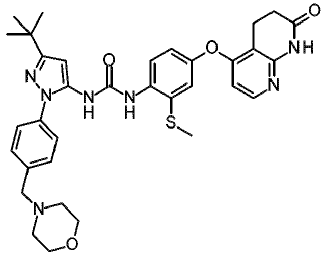

According to method B step 2: 2,2,2-trichloroethyl N-[5-tert-butyl-2-[4-(morpholinomethyl)phenyl]pyrazol-3-yl]carbamate (65) (0.232g, 0.47mmol) and 5-(4-amino-3-methylsulfanyl-phenoxy)-3,4-dihydro-1H-1,8-naphthyridin-2-one (7) (0.095g, 0.32mmol) were dissolved in a mixture of solvents DMSO/ACN (2mL/2mL). To the solution was added DIPEA (0.06mL, 0.35mmol) and the mixture was stirred at 70 °C overnight. The reaction was quenched with water, and the resulting mixture was extracted with ethyl acetate. The combined organic layers were evaporated under reduced pressure. The solid residue was purified by flash chromatography and triturated in ACN to give 0.017g of the title compound (yield: 8%). MS m/z (ES) [M+H]⁺: 642.5. ¹H NMR (400 MHz, DMSO-*d₆*) δ 10.50 (s, 1H), 8.97 (s, 1H), 8.35 (s, 1H), 7.98 (d, J = 5.7 Hz, 1H), 7.71 (d, J = 8.8 Hz, 1H), 7.52 - 7.43 (m, 4H), 7.12 (d, J = 2.7 Hz, 1H), 6.93 (dd, J = 8.8, 2.7 Hz, 1H), 6.37 - 6.30 (m, 2H), 3.59 (m, 4H), 3.53 (s, 2H), 2.93 (t, J = 7.7 Hz, 2H), 2.56 (s, 2H), 2.42 (m, 4H), 2.41 (s, 3H), 1.28 (s, 9H).

### Example 043

### 1-[5-tert-butyl-2-[4-(morpholinomethyl)phenyl]pyrazol-3-yl]-3-[4-[(3-oxo-4H-pyrido[3,2-b][1,4]oxazin-8-yl)oxy]-2-(trifluoromethyl)phenyl]urea

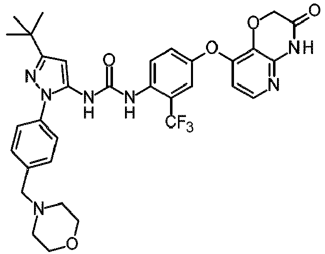

According to method A step 2 : 2,2,2-trichloroethyl N-[4-[(3-oxo-4H-pyrido[3,2-b][1,4]oxazin-8-yl)oxy]-2-(trifluoromethyl)phenyl]carbamate (67) (0.215g, 0.43mmol) and 5-tert-butyl-2-[4-(morpholinomethyl)phenyl]pyrazol-3-amine (27) (0.09g, 0.29mmol) were dissolved in a mixture of solvents ACN/DMSO (1.5mL:1.5mL). To the solution was added DIPEA (0.05mL, 0.29mmol) and the reaction was stirred overnight at 70 °C. The reaction was quenched with water, and the resulting mixture was extracted with ethyl acetate. The combined organic layers were evaporated under reduced pressure. The residue was purified by Flash chromatography to give 0.018g of the title compound (yield: 9%). MS m/z (ES) [M+H]⁺: 666.2. ¹H NMR (400 MHz, DMSO-*d₆*) δ 11.37 (s, 1H), 8.97 (s, 1H), 8.39 (s, 1H), 7.86 (dd, J = 5.8, 1.6 Hz, 1H), 7.74 (d, J = 8.9 Hz, 1H), 7.51 - 7.34 (m, 6H), 6.63 (d, J = 5.7 Hz, 1H), 6.34 (d, J = 1.7 Hz, 1H), 4.65 (d, J = 1.5 Hz, 2H), 3.59 (t, J = 4.6 Hz, 4H ), 3.52 (s, 2H), 2.40 (m, 4H), 1.27 (s, 9H).

### Example 044

### 1-[5-tert-butyl-2-[3-(morpholinomethyl)phenyl]pyrazol-3-yl]-3-[4-[(3-oxo-4H-pyrido[3,2-b][1,4]oxazin-8-yl)oxy]-2-(trifluoromethyl)phenyl]urea

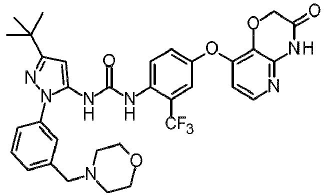

According to method A step 2 : 2,2,2-trichloroethyl N-[4-[(3-oxo-4H-pyrido[3,2-b][1,4]oxazin-8-yl)oxy]-2-(trifluoromethyl)phenyl]carbamate (67) (0.215g, 0.43mmol) and 5-tert-butyl-2-[3-(morpholinomethyl)phenyl]pyrazol-3-amine (31) (0.09g, 0.29mmol) were dissolved in a mixture of solvents ACN/DMSO (1.5mL:1.5mL). To the solution was added DIPEA (0.05mL, 0.29mmol) and the reaction was stirred overnight at 70 °C. The reaction was quenched with water, and the resulting mixture was extracted with ethyl acetate. The combined organic layers were evaporated under reduced pressure. The residue was purified by Flash chromatography to give 0.035g of the title compound (yield: 18%). MS m/z (ES) [M+H]⁺: 666.2. ¹H NMR (400 MHz, DMSO-*d₆*) δ 11.37 (s, 1H), 9.00 (s, 1H), 8.35 (s, 1H), 7.86 (d, *J* = 5.6 Hz, 1H), 7.78 (d, J = 8.9 Hz, 1H), 7.51 - 7.34 (m, 6H), 6.63 (d, J = 5.6 Hz, 1H), 6.35 (s, 1H), 4.65 (s, 2H), 3.55 - 3.51 (m, 6H), 2.39 (m, 4H), 1.28 (s, 9H).

### Example 045

### 1-[5-tert-butyl-2-[3-(morpholinomethyl)phenyl]pyrazol-3-yl]-3-[4-[(7-oxo-6,8-dihydro-5H-1,8-naphthyridin-4-yl)oxy]-2-(trifluoromethyl)phenyl]urea

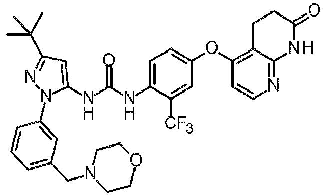

According to method A step 2 : 2,2,2-trichloroethyl N-[4-[(7-oxo-6,8-dihydro-5H-1,8-naphthyridin-4-yl)oxy]-2-(trifluoromethyl)phenyl]carbamate (68) (0.238g, 0.49mmol) and 5-tert-butyl-2-[3-(morpholinomethyl)phenyl]pyrazol-3-amine (31) (0.10g, 0.32mmol) were dissolved in a mixture of solvents ACN/DMSO (2mL:2mL). To the solution was added DIPEA (0.056mL, 0.32mmol) and the reaction was stirred overnight at 70 °C. The reaction was quenched with water, and the resulting mixture was extracted with ethyl acetate. The combined organic layers were evaporated under reduced pressure. The residue was purified by Flash chromatography to give 0.034g of the title compound (yield: 16%). MS m/z (ES) [M+H]⁺: 664.1. ¹H NMR (400 MHz, DMSO-*d₆*) δ 10.55 (s, 1H), 9.03 (s, 1H), 8.37 (s, 1H), 8.02 (d, J = 5.8 Hz, 1H), 7.85 (d, *J* = 9.0 Hz, 1H), 7.52 - 7.40 (m, 5H),7.35 (d, J = 7.5 Hz, 1H), 6.38 (d, J = 5.8 Hz, 1H), 6.35 (s, 1H), 3.60 - 3.51 (m, 6H), 2.92 (t, J = 7.7 Hz, 2H), 2.56 (s, 2H), 2.39 (d, J = 5.0 Hz, 4H), 1.28 (s, 9H).

### Example 046

### 1-[5-tert-butyl-2-[4-(dimethylaminomethyl)phenyl]pyrazol-3-yl]-3-[2-methylsulfanyl-4-[(7-oxo-6,8-dihydro-5H-1,8-naphthyridin-4-yl)oxy]phenyl]urea

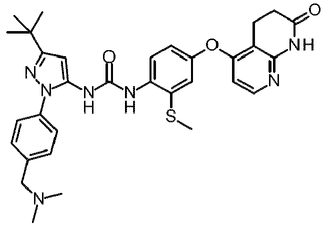

According to method B step 2: 2,2,2-trichloroethyl N-[5-tert-butyl-2-[4-[(dimethylamino)methyl]phenyl]pyrazol-3-yl]carbamate (63) (0.178g, 0.40mmol) and 5-(4-amino-3-methylsulfanyl-phenoxy)-3,4-dihydro-1H-1,8-naphthyridin-2-one (7) (0.08g, 0.27mmol) were dissolved in a mixture of solvents DMSO/ACN (1.5mL/1.5mL). To the solution was added DIPEA (0.11mL, 0.51mmol) and the mixture was stirred at 70 °C overnight. The reaction was quenched with water, and the resulting mixture was extracted with ethyl acetate. The combined organic layers were evaporated under reduced pressure. The solid residue was purified by flash chromatography then preparative HPLC to give 0.020g of the title compound (yield: 12%). MS m/z (ES) [M+H]⁺: 600.2. ¹H NMR (400 MHz, DMSO-*d₆*) δ 10.50 (s, 1H), 8.97 (s, 1H), 8.35 (s, 1H), 7.98 (d, J = 5.8 Hz, 1H), 7.72 (d, J = 8.8 Hz, 1H), 7.49 (d, J = 8.5 Hz, 2H), 7.43 (d, J = 8.6 Hz, 2H), 7.12 (d, J = 2.7 Hz, 1H), 6.93 (dd, J = 8.8, 2.8 Hz, 1H), 6.35 (s, 1H), 6.32 (d, *J* = 5.7 Hz, 1H), 3.44 (s, 2H), 2.93 (t, *J* = 7.7 Hz, 2H), 2.56 (m, 2H), 2.43 (s, 3H), 2.19 (s, 6H), 1.28 (s, 9H).

### Example 047

### 1-[5-tert-butyl-2-[3-(morpholinomethyl)phenyl]pyrazol-3-yl]-3-[2-methylsulfanyl-4-[(7-oxo-6,8-dihydro-5H-1,8-naphthyridin-4-yl)oxy]phenyl]urea

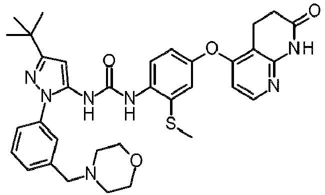

According to method A step 2 : 2,2,2-trichloroethyl N-[2-methylsulfanyl-4-[(7-oxo-6,8-dihydro-5H-1,8-naphthyridin-4-yl)oxy]phenyl]carbamate (69) (0.228g, 0.48mmol) and 5-tert-butyl-2-[3-(morpholinomethyl)phenyl]pyrazol-3-amine (31) (0.10g, 0.32mmol) were dissolved in a mixture of solvents ACN/DMSO (0.5mL:2mL). To the solution was added DIPEA (0.055mL, 0.32mmol) and the reaction was stirred overnight at 70 °C. The reaction was quenched with water, and the resulting mixture was extracted with ethyl acetate. The combined organic layers were evaporated under reduced pressure. The residue was purified by Flash chromatography to give 0.048g of the title compound (yield: 23%). MS m/z (ES) [M+H]⁺: 642.2. ¹H NMR (400 MHz, DMSO-*d₆*) δ 10.50 (s, 1H), 8.98 (s, 1H), 8.30 (s, 1H), 7.98 (d, *J* = 5.8 Hz, 1H), 7.76 (d, J = 8.9 Hz, 1H), 7.46 (dt, J = 9.9, 8.0 Hz, 3H), 7.34 (dt, J = 7.4, 1.5 Hz, 1H), 7.13 (d, J = 2.7 Hz, 1H), 6.94 (dd, J = 8.8, 2.8 Hz, 1H), 6.35 (s, 1H), 6.31 (d, J = 5.8 Hz, 1H), 3.54 (m, 6H), 2.93 (t, J = 7.7 Hz, 2H), 2.56 (m, 2H), 2.43 (s, 3H), 2.39 (m, 4H), 1.29 (s, 9H).

### Example 048

### 1-[5-tert-butyl-2-[3-(dimethylaminomethyl)phenyl]pyrazol-3-yl]-3-[4-[(3-oxo-4H-pyrido[3,2-b][1,4]oxazin-8-yl)oxy]-2-(trifluoromethyl)phenyl]urea

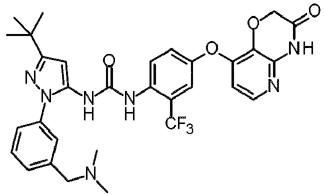

According to method A step 2 : 2,2,2-trichloroethyl N-[4-[(3-oxo-4H-pyrido[3,2-b][1,4]oxazin-8-yl)oxy]-2-(trifluoromethyl)phenyl]carbamate (67) (0.275g, 0.55mmol) and 5-tert-butyl-2-[3-[(dimethylamino)methyl]phenyl]pyrazol-3-amine (35) (0.10g, 0.37mmol) were dissolved in a mixture of solvents ACN/DMSO (2mL:2mL). To the solution was added DIPEA (0.064mL, 0.37mmol) and the reaction was stirred overnight at 70 °C. The reaction was quenched with water, and the resulting mixture was extracted with ethyl acetate. The combined organic layers were evaporated under reduced pressure. The residue was purified by Flash chromatography to give 0.030g of the title compound (yield: 13%). MS m/z (ES) [M+H]⁺: 624.2. ¹H NMR (400 MHz, DMSO-*d₆*) δ 11.37 (s, 1H), 8.98 (s, 1H), 8.36 (s, 1H), 7.86 (d, J = 5.6 Hz, 1H), 7.78 (d, J = 8.9 Hz, 1H), 7.53 - 7.29 (m, 6H), 6.64 (d, J = 5.6 Hz, 1H), 6.34 (s, 1H), 4.65 (s, 2H), 3.45 (s, 2H), 2.17 (s, 6H), 1.28 (s, 9H).

### Example 049

### 1-[5-tert-butyl-2-[4-(dimethylaminomethyl)phenyl]pyrazol-3-yl]-3-[4-[(3-oxo-4H-pyrido[3,2-b][1,4]oxazin-8-yl)oxy]-2-(trifluoromethyl)phenyl]urea

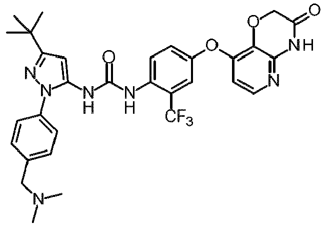

According to method A step 2 : 2,2,2-trichloroethyl N-[4-[(3-oxo-4H-pyrido[3,2-b][1,4]oxazin-8-yl)oxy]-2-(trifluoromethyl)phenyl]carbamate (67) (0.358g, 0.72mmol) and 5-tert-butyl-2-[4-[(dimethylamino)methyl]phenyl]pyrazol-3-amine (23) (0.13g, 0.48mmol) were dissolved in a mixture of solvents ACN/DMSO (3mL:3mL). To the solution was added DIPEA (0.083mL, 0.48mmol) and the reaction was stirred overnight at 70 °C. The reaction was quenched with water, and the resulting mixture was extracted with ethyl acetate. The combined organic layers were evaporated under reduced pressure. The residue was purified by Flash chromatography then preparative TLC to give 0.024g of the title compound (yield: 8%). MS m/z (ES) [M+H]⁺: 624.2. ¹H NMR (400 MHz, DMSO-*d₆*) δ 11.36 (s, 1H), 8.97 (s, 1H), 8.39 (s, 1H), 7.85 (d, J = 5.6 Hz, 1H), 7.74 (d, J = 8.9 Hz, 1H), 7.49 - 7.99 (m, 6H), 6.63 (d, J = 5.6 Hz, 1H), 6.34 (s, 1H), 4.65 (s, 2H), 3.44 (s, 2H), 2.18 (s, 6H), 1.27 (s, 9H).

### Example 050

### 1-[5-tert-butyl-2-[3-(dimethylaminomethyl)phenyl]pyrazol-3-yl]-3-[2-methylsulfanyl-4-[(7-oxo-6,8-dihydro-5H-1,8-naphthyridin-4-yl)oxy]phenyl]urea

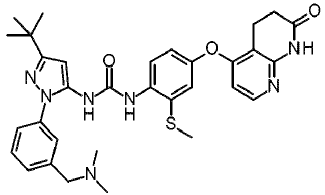

According to method B step 2: 2,2,2-trichloroethyl N-[5-tert-butyl-2-[3-[(dimethylamino)methyl]phenyl]pyrazol-3-yl]carbamate (60) (0.223g, 0.40mmol) and 5-(4-amino-3-methylsulfanyl-phenoxy)-3,4-dihydro-1H-1,8-naphthyridin-2-one (7) (0.10g, 0.33mmol) were dissolved in a mixture of solvents DMSO/ACN (2mL/2mL). To the solution was added DIPEA (0.064mL, 0.37mmol) and the mixture was stirred at 70 °C overnight. The reaction was quenched with water, and the resulting mixture was extracted with ethyl acetate. The combined organic layers were evaporated under reduced pressure. The solid residue was purified by flash chromatography then preparative HPLC to give 0.012g of the title compound (yield: 6%). MS m/z (ES) [M+H]⁺: 600.2. ¹H NMR (400 MHz, DMSO-*d₆*) δ 10.49 (s, 1H), 8.97 (s, 1H), 8.33 (s, 1H), 7.98 (d, J = 5.8 Hz, 1H), 7.74 (d, J = 8.8 Hz, 1H), 7.51 - 7.40 (m, 3H), 7.32 (dd, J = 7.4, 1.6 Hz, 1H), 7.12 (d, *J* = 2.7 Hz, 1H), 6.94 (dd, *J* = 8.8, 2.8 Hz, 1H), 6.38 - 6.29 (m, 2H), 3.46 (s, 2H), 2.93 (t, J = 7.7 Hz, 2H), 2.55 (d, J = 3.8 Hz, 2H), 2.17 (s, 3H), 1.91 (s, 6H), 1.29 (s, 9H).

### Example 051

### 1-[5-tert-butyl-2-[4-[2-(dimethylamino)ethyl]phenyl]pyrazol-3-yl]-3-[2-methylsulfanyl-4-[(3-oxo-4H-pyrido[3,2-b][1,4]oxazin-8-yl)oxy]phenyl]urea

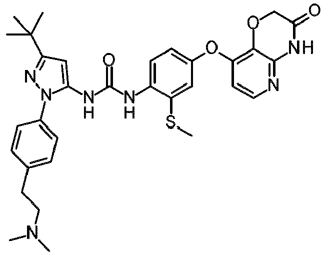

According to method A step 2 : 2,2,2-trichloroethyl N-[2-methylsulfanyl-4-[(3-oxo-4H-pyrido[3,2-b][1,4]oxazin-8-yl)oxy]phenyl]carbamate (70) (0.280g, 0.60mmol) and 5-tert-butyl-2-[4-[2-(dimethylamino)ethyl]phenyl]pyrazol-3-amine (39) (0.115g, 0.40mmol) were dissolved in a mixture of solvents ACN/DMSO (2mL:3mL). To the solution was added DIPEA (0.07mL, 0.40mmol) and the reaction was stirred overnight at 70 °C. The reaction was quenched with water, and the resulting mixture was extracted with ethyl acetate. The combined organic layers were evaporated under reduced pressure. The residue was purified by Flash chromatography then preparative HPLC to give 0.023g of the title compound (yield: 9%). MS m/z (ES) [M+H]⁺: 616.2. ¹H NMR (400 MHz, DMSO-*d₆*) δ 11.31 (s, 1H), 8.94 (s, 1H), 8.34 (s, 1H), 7.80 (d, J = 5.6 Hz, 1H), 7.68 (d, *J* = 8.8 Hz, 1H), 7.46 - 7.34 (m, 4H), 7.08 (d, J = 2.7 Hz, 1H) 6.94 - 6.87 (m, 1H), 6.51 (d, J = 5.6 Hz, 1H), 6.33 (s, 1H), 4.65 (s, 2H), 2.77 (t, J = 7.6 Hz, 2H), 2.52 (s, 2H), 2.42 (s, 3H), 2.21 (s, 6H), 1.27 (s, 9H).

### Example 052

### 1-[5-tert-butyl-2-[4-[2-(morpholino)ethyl]phenyl]pyrazol-3-yl]-3-[2-methylsulfanyl-4-[(3-oxo-4H-pyrido[3,2-b][1,4]oxazin-8-yl)oxy]phenyl]urea

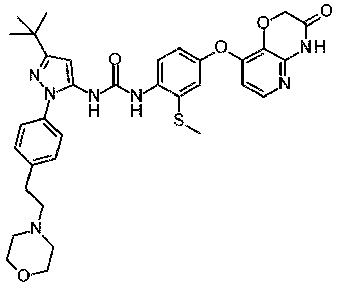

According to method A step 2 : 2,2,2-trichloroethyl N-[2-methylsulfanyl-4-[(3-oxo-4H-pyrido[3,2-b][1,4]oxazin-8-yl)oxy]phenyl]carbamate (70) (0.219g, 0.46mmol) and 5-tert-butyl-2-[4-(2-morpholinoethyl)phenyl]pyrazol-3-amine (43) (0.10g, 0.30mmol) were dissolved in a mixture of solvents ACN/DMSO (0.5mL:2mL). To the solution was added DIPEA (0.053mL, 0.30mmol) and the reaction was stirred for 2.5 days at 70 °C. The reaction was quenched with water, and the resulting mixture was extracted with ethyl acetate. The combined organic layers were evaporated under reduced pressure. The residue was purified by Flash chromatography to give 0.020g of the title compound (yield: 10%). MS m/z (ES) [M+H]⁺: 658.1. ¹H NMR (400 MHz, DMSO-*d₆*) δ 11.31 (s, 1H), 8.93 (s, 1H), 8.34 (s, 1H), 7.81 (d, J = 5.6 Hz, 1H), 7.68 (d, J = 8.9 Hz, 1H), 7.41 (m, 4H), 7.09 (d, J = 2.8 Hz, 1H) 6.91 (dd, J = 8.8, 2.8 Hz, 1H), 6.51 (d, J = 5.6 Hz, 1H), 6.34 (s, 1H), 4.66 (s, 2H), 3.59 (m, 4H), 2.81 (dd, J = 9.0, 6.4 Hz, 2H), 2.59 - 2.54 (m, 2H), 2.45 (m, 4H), 2.43 (s, 3H), 1.28 (s, 9H).

### Example 053

### 1-[5-tert-butyl-2-[4-(2-morpholinoethyl)phenyl]pyrazol-3-yl]-3-[4-[(3-oxo-4H-pyrido[3,2-b][1,4]oxazin-8-yl)oxy]-2-(trifluoromethyl)phenyl]urea

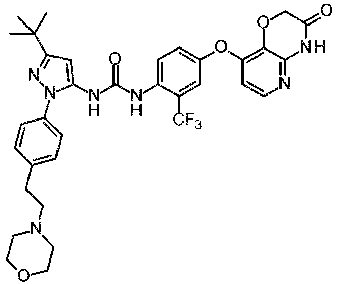

According to method A step 2 : 2,2,2-trichloroethyl N-[4-[(3-oxo-4H-pyrido[3,2-b][1,4]oxazin-8-yl)oxy]-2-(trifluoromethyl)phenyl]carbamate (67) (0.230g, 0.46mmol) and 5-tert-butyl-2-[4-(2-morpholinoethyl)phenyl]pyrazol-3-amine (43) (0.10g, 0.30mmol) were dissolved in a mixture of solvents ACN/DMSO (2mL:3mL). To the solution was added DIPEA (0.053mL, 0.30mmol) and the reaction was stirred overnight at 70 °C. The reaction was quenched with water, and the resulting mixture was extracted with ethyl acetate. The combined organic layers were evaporated under reduced pressure. The residue was purified by Flash chromatography then preparative HPLC to give 0.013g of the title compound (yield: 6%). MS m/z (ES) [M+H]⁺: 680.2. ¹H NMR (400 MHz, DMSO-*d₆*) δ 11.37 (s, 1H), 8.98 (s, 1H), 8.41 (s, 1H), 7.86 (d, J = 5.6 Hz, 1H), 7.76 (d, J = 8.9 Hz, 1H), 7.47 - 7.34 (m, 6H), 6.64 (d, J = 5.6 Hz, 1H), 6.34 (s, 1H), 4.65 (s, 2H), 3.59 (t, J = 4.6 Hz, 4H), 2.81 (t, J = 7.7 Hz, 2H), 2.62 - 2.54 (m, 2H), 2.45 (d, J = 4.8 Hz, 4H), 1.27 (s, 9H).

### Example 054

### 1-[5-tert-butyl-2-[4-[(4-methylpiperazin-1-yl)methyl]phenyl]pyrazol-3-yl]-3-[2-methylsulfanyl-4-[(3-oxo-4H-pyrido[3,2-b][1,4]oxazin-8-yl)oxy]phenyl]urea

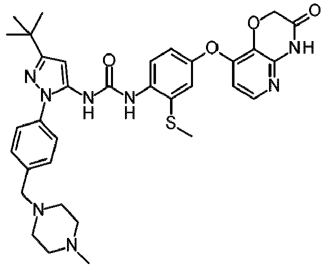

According to method A step 2 : 2,2,2-trichloroethyl N-[2-methylsulfanyl-4-[(3-oxo-4H-pyrido[3,2-b][1,4]oxazin-8-yl)oxy]phenyl]carbamate (70) (0.241g, 0.48mmol) and 5-tert-butyl-2-[4-[(4-methylpiperazin-1-yl)methyl]phenyl]pyrazol-3-amine (47) (0.11g, 0.31mmol) were dissolved in a mixture of solvents ACN/DMSO (0.5mL:2mL). To the solution was added DIPEA (0.055mL, 0.31mmol) and the reaction was stirred overnight at 70 °C. The reaction was quenched with water, and the resulting mixture was extracted with ethyl acetate. The combined organic layers were evaporated under reduced pressure. The residue was purified by preparative TLC to give 0.016g of the title compound (yield: 8%). MS m/z (ES) [M+H]+: 657.6. ¹H NMR (400 MHz, DMSO-*d₆*) δ 11.31 (s, 1H), 8.94 (s, 1H), 8.32 (s, 1H), 7.80 (d, J = 5.6 Hz, 1H), 7.66 (d, J = 8.8 Hz, 1H), 7.51 - 7.41 (m, 4H), 7.09 (d, J = 2.8 Hz, 1H) 6.9 (dd, J = 8.8, 2.8 Hz, 1H), 6.51 (d, J = 5.7 Hz, 1H), 6.34 (s, 1H), 4.66 (s, 2H), 3.51 (s, 2H), 2.42 (m, 11H), 2.16 (s, 3H), 1.28 (s, 9H).

### EXEMPLE 2: In silico binding affinity determination

Structure based drug design (SBDD) protocol was employed for the purpose of designing new candidates as kinase inhibitor. In this approach, firstly the core structures are derived from bioactive molecules. In the present case, earlier developed kinase inhibitors from Formula (I) served as the parent bioactive molecules, which was deconstructed and redesigned to obtain new kinase inhibitor candidates based on features known in the literature to produce Type II class of inhibitors (J. Med. Chem. 2015, 58, 466-479). Docking experiments using LigandScout^{®} software (www.inteligand.com) were performed to validate the predictive interaction of these new inhibitors in the isolated binding ATP pocket and to calculate the corresponding binding affinity score. As reference model and kinase of interest, the published crystal structure of c-SRC (PDB: 3F3V) (J. Med. Chem. 2009, 52, 13, 3915-3926) was used to perform the docking process. Theses binding affinity scores represent the binding affinity between the molecule and the macromolecule. They were compared to those obtained with the parent bioactive molecules to assess their predictive kinase inhibitors properties (Table 5).

**Table 5**

| | | | | | | | | | | |
|---|---|---|---|---|---|---|---|---|---|---|
| **Examples** | **001** | **002** | **003** | **004** | **005** | **006** | **007** | **008** | **009** | **010** |
| **Binding Affinity Score** | -37,55 | -38,02 | -38,27 | -30,37 | -25,18 | -36,10 | -31,97 | -28,98 | -31,44 | -40,02 |
| **Examples** | **011** | **012** | **013** | **014** | **015** | **016** | **017** | **018** | **019** | **020** |
| **Binding Affinity Score** | -37,59 | -32,86 | -37,19 | -40,93 | -41,01 | -37,66 | -38,87 | -34,65 | -33,69 | -25,75 |
| **Examples** | **021** | **022** | **023** | **024** | **025** | **026** | **027** | **028** | **029** | **030** |
| **Binding Affinity Score** | -26,11 | -31,71 | -32,08 | -29,68 | -26,71 | -24,82 | -34,59 | -44,07 | -42,68 | -37,40 |
| **Examples** | **031** | **032** | **033** | **034** | **035** | **036** | **037** | **038** | **039** | **040** |
| **Binding Affinity Score** | -37,27 | -33,23 | -39,34 | -39,37 | -40,62 | -32,33 | -28,03 | -35,66 | -35,09 | -38,81 |
| **Examples** | **041** | **042** | **043** | **044** | **045** | **046** | **047** | **048** | **049** | **050** |
| **Binding Affinity Score** | -40,11 | -35,79 | -38,86 | -43,43 | -46,86 | -39,59 | -40,24 | -44,02 | -42,36 | -39,76 |
| **Examples** | **051** | **052** | **053** | **054** | **055** | **056** | **057** | **058** | **059** | **060** |
| **Binding Affinity Score** | -32,26 | -34,83 | -43,32 | -32,70 | -22,75 | -38,94 | -32,74 | -32,35 | -29,29 | -29,65 |
| **Examples** | **061** | **062** | **063** | **064** | **065** | **066** | **067** | **068** | **069** | **070** |
| **Binding Affinity Score** | -25,69 | -30,84 | -35,51 | -31,61 | -32,30 | -33,93 | -31,21 | -28,48 | -36,58 | -30,92 |
| **Examples** | **071** | **072** | **073** | **074** | **075** | **076** | **077** | **078** | **079** | **080** |
| **Binding Affinity Score** | -33,95 | -37,19 | -31,07 | -29,73 | -23,62 | -22,23 | -31,56 | -31,97 | -43,30 | -30,42 |
| **Examples** | **081** | **082** | **083** | **084** | **085** | **086** | **087** | | | |
| **Binding Affinity Score** | -32,56 | -31,70 | -36,64 | -29,16 | -25,23 | -31,58 | -34,86 | | | |

### EXAMPLE 3: In vitro biological activities

### Example 3.1: in vitro cytotoxic activity on cancer cell lines

Cytotoxicity assays consist in treating cells with compounds and assessing remaining viability after a defined incubation time, depending on the cell line. CellTiter-Glo^{®} 2.0 reagent allows viability measurement by quantification of ATP present, which is an indicator of metabolically active cells.

The cancer cell lines were cultivated in their respective growth medium (see tables 6a and 6b). On day 0 of the cytotoxicity assay, the cells were harvested and seeded into a 384-well plate at a defined density in their respective growth medium (see tables 6a and 6b). Compounds to be tested were then added onto the cells and incubated for a defined duration (see tables 6a and 6b). Following incubation, CellTiter-Glo^{®} 2.0 reagent (cat.no. G9241 from Promega) was added into the wells (vol/vol, according to the manufacturer's recommendations), plates were gently shaken and incubated for 10 minutes in the dark at room temperature. Bioluminescence was then measured with a multimode plate reader (Victor Nivo from Perkin Elmer), and cell viability and relative IC50 were determined.

**Table 6a: Cancer cell characteristics, culture and cytotoxic assay conditions**

| **Cell line** | **RD** | **EoL-1** |
|---|---|---|
| **Species** | Human | Human |
| **Type of cancer** | Rhabdomyosarcoma | Leukemia |
| **Growth medium composition** | DMEM (4,5g/L glucose) with L-Gln and Pyruvate (cat.no. 41966-029 from Gibco), supplemented with 10% of fetal calf serum and 1% PenStrep | RPMI-1640 with of L-Gln (cat.no. 21875-034 from Gibco), supplemented with 10% of fetal calf serum and 1% PenStrep |
| **Cell density in the cytotoxicity assay** | 2500 cells/well | 5000 cells/well |
| **Compound incubation duration** | 2 days and 4 days | 1 day |
| | | |

| **Cell line** | **4T1** | **MDA-MB-231** |
|---|---|---|
| **Species** | Mouse | Human |
| **Type of cancer** | Triple negative breast cancer | Triple negative breast cancer |
| **Growth medium composition** | RPMI-1640 with L-Gln (cat.no. 21875-034 from Gibco), supplemented with 10% of fetal calf serum and 1% PenStrep | DMEM (4,5g/L glucose) with L-Gln (cat.no. 41965-039 from Gibco), supplemented with 10% of fetal calf serum and 1% PenStrep |
| **Cell density in the cytotoxicity assay** | 2500 cells/well | 2500 cells/well |
| **Compound incubation duration** | 2 days | 3 days |

**Table 6b: Human KRAS mutated cancer cell characteristics, culture and cytotoxic assay conditions**

| **Cell line** | **Type of cancer** | **Growth medium composition** | **Compound incubation duration** |
|---|---|---|---|
| **A549** | Lung adenocarcinoma | DMEM + 10% FCS | 2 days |
| **HCT 116** | Colon adenocarcinoma | DMEM + 10% FCS | 2 days |
| **Mia PaCa-2** | Pancreatic adenocarcinoma | DMEM + 10% FCS | 2 days |
| **SW 480** | Colon adenocarcinoma | DMEM + 10% FCS | 2 days |
| **HEC-1-A** | Endometrial adenocarcinoma | McCoys 5a + 10% FCS | 2 days |
| **PSN1** | Pancreatic adenocarcinoma | RPMI-1640 + 10% FCS | 2 days |
| **RPMI 8226** | Plasma cell myeloma | RPMI-1640 + 10% FCS | 2 days |

As shown in Tables 7, 9 and 11, compounds of the present invention demonstrated cytotoxic activity on RD, 4T1, MDA-MB-231 and EoL-1 cancer cell lines, respectively. This cytotoxic activity was similar or better than the one of structurally related commercial kinase inhibitors tested in the same conditions, as shown in Tables 8, 10 and 12. Compound #020 has been tested on a panel of KRAS mutated cell lines and also showed cytotoxic activity on them, as shown in Table 13.

**Table 7 shows cytotoxicity of compounds of the present invention on Rhabdomyosarcoma RD cells.**

| | | | | |
|---|---|---|---|---|
| Qualitative scale: +++: IC50 < 1 µM; ++: 1 µM ≤ IC50 < 5 µM; +: 5 µM ≤ IC50 < 10 µM; -: IC50 ≥ 10 µM; +++: cytotoxicity ≥ 85%; ++: 50% ≤ cytotoxicity < 85%; +: 20 % ≤ cytotoxicity < 50%; -: cytotoxicity < 20%. | | | | |

| **Compound ID** | **RD cells** | | | |
|---|---|---|---|---|
| | **2 day-treatment** | | **4 day-treatment** | |
| | **IC50** | **Cytotoxicity** | **IC50** | **Cytotoxicity** |
| **001** | ++ | + | ++ | ++ |
| **002** | ++ | ++ | ++ | ++ |
| **003** | ++ | + | + | + |
| **004** | ++ | + | ++ | ++ |
| **005** | ++ | + | ++ | ++ |
| **006** | ++ | ++ | ++ | ++ |
| **007** | + | + | ++ | ++ |
| **008** | +++ | ++ | +++ | +++ |
| **009** | ++ | ++ | ++ | ++ |
| **010** | + | ++ | + | ++ |
| **011** | + | + | - | + |
| **012** | ++ | ++ | + | ++ |
| **013** | ++ | ++ | + | ++ |
| **014** | + | + | - | + |
| **015** | + | ++ | ++ | ++ |
| **016** | ++ | ++ | + | ++ |
| **017** | ++ | ++ | ++ | +++ |
| **018** | ++ | + | ++ | ++ |
| **019** | ++ | ++ | ++ | ++ |
| **020** | ++ | ++ | +++ | ++ |
| **021** | ++ | ++ | ++ | ++ |
| **022** | ++ | ++ | ++ | ++ |
| **023** | ++ | ++ | ++ | ++ |
| **024** | ++ | ++ | ++ | ++ |
| **026** | ++ | ++ | ++ | ++ |
| **027** | ++ | ++ | ++ | ++ |
| **028** | ++ | ++ | ++ | +++ |
| **030** | + | ++ | + | ++ |
| **031** | ++ | ++ | ++ | ++ |
| **032** | ++ | ++ | ++ | +++ |
| **033** | ++ | +++ | ++ | +++ |
| **034** | ++ | +++ | ++ | +++ |
| **035** | ++ | ++ | + | ++ |
| **036** | ++ | + | ++ | - |
| **037** | + | ++ | ++ | ++ |
| **038** | ++ | ++ | ++ | ++ |
| **039** | ++ | ++ | + | + |
| **040** | + | ++ | + | ++ |
| **041** | ++ | +++ | ++ | +++ |
| **042** | ++ | ++ | ++ | ++ |
| **043** | - | + | - | + |
| **044** | - | + | - | + |
| **045** | + | + | + | + |
| **046** | + | +++ | ++ | +++ |
| **047** | + | ++ | ++ | ++ |
| **048** | + | ++ | ++ | +++ |
| **049** | + | +++ | + | +++ |
| **050** | ++ | +++ | ++ | +++ |
| **051** | ++ | +++ | ++ | +++ |
| **052** | ++ | ++ | ++ | ++ |
| **054** | ++ | +++ | ++ | +++ |

**Table 8 shows cytotoxicity of structurally related commercial kinase inhibitors on Rhabdomyosarcoma RD cells in the same experimental conditions as the compounds of the present invention**

| | | | | |
|---|---|---|---|---|
| Qualitative scale: +++: IC50 < 1 µM; ++: 1 µM ≤ IC50 < 5 µM; +: 5 µM ≤ IC50 < 10 µM; -: IC50 ≥ 10 µM; +++: cytotoxicity ≥ 85 %; ++: 50 % ≤ cytotoxicity < 85 %; +: 20 % ≤ cytotoxicity < 50 %; -: cytotoxicity < 20 %. | | | | |

| **Compound name** | **RD cells** | | | |
|---|---|---|---|---|
| | **2 day-treatment** | | **4 day-treatment** | |
| | **IC50** | **Cytotoxicity** | **IC50** | **Cytotoxicity** |
| **Regorafenib** | + | + | ++ | + |
| **Sorafenib** | - | ++ | + | ++ |
| **Tivozanib** | - | - | - | - |

**Table 9 shows cytotoxicity of compounds of the present invention on 4T1 and MDA-MB-231 triple negative breast cancer cells.**

| | | | | |
|---|---|---|---|---|
| Qualitative scale: +++: IC50 < 1 µM; ++: 1 µM ≤ IC50 < 5 µM; +: 5 µM ≤ IC50 < 10 µM; -: IC50 ≥ 10 µM; +++: cytotoxicity ≥ 85%; ++: 50% ≤ cytotoxicity < 85%; +: 20 % ≤ cytotoxicity < 50%; -: cytotoxicity < 20%. | | | | |

| **Compound ID** | **4T1 cells** | | **MDA-MB-231 cells** | |
|---|---|---|---|---|
| | **IC50** | **Cytotoxicity** | **IC50** | **Cytotoxicity** |
| **001** | ++ | +++ | ++ | ++ |
| **002** | ++ | +++ | ++ | ++ |
| **003** | +++ | +++ | ++ | ++ |
| **004** | ++ | +++ | +++ | ++ |
| **005** | + | ++ | ++ | ++ |
| **006** | ++ | +++ | ++ | ++ |
| **007** | ++ | +++ | ++ | ++ |
| **008** | +++ | +++ | +++ | ++ |
| **009** | +++ | +++ | +++ | ++ |
| **010** | ++ | +++ | ++ | ++ |
| **011** | ++ | +++ | ++ | ++ |
| **012** | ++ | +++ | ++ | ++ |
| **013** | +++ | +++ | ++ | ++ |
| **014** | ++ | +++ | ++ | + |
| **015** | +++ | +++ | ++ | ++ |
| **016** | +++ | +++ | ++ | ++ |
| **017** | +++ | +++ | ++ | ++ |
| **018** | +++ | +++ | ++ | ++ |
| **019** | ++ | +++ | ++ | ++ |
| **020** | ++ | +++ | +++ | ++ |
| **021** | ++ | ++ | ++ | ++ |
| **022** | ++ | +++ | ++ | ++ |
| **023** | ++ | +++ | ++ | ++ |
| **024** | ++ | ++ | ++ | ++ |
| **026** | ++ | +++ | ++ | ++ |
| **027** | +++ | +++ | +++ | ++ |
| **028** | +++ | +++ | ++ | +++ |
| **029** | ++ | ++ | + | + |
| **030** | ++ | +++ | ++ | ++ |
| **031** | ++ | +++ | ++ | ++ |
| **032** | +++ | +++ | +++ | ++ |
| **033** | +++ | +++ | ++ | +++ |
| **034** | +++ | +++ | ++ | +++ |
| **035** | ++ | +++ | ++ | +++ |
| **036** | - | - | + | + |
| **037** | ++ | +++ | ++ | ++ |
| **038** | ++ | +++ | ++ | ++ |
| **039** | +++ | +++ | ++ | ++ |
| **040** | +++ | +++ | ++ | ++ |
| **041** | +++ | +++ | ++ | +++ |
| **042** | +++ | +++ | ++ | ++ |
| **043** | ++ | +++ | + | ++ |
| **044** | ++ | +++ | + | + |
| **045** | +++ | +++ | ++ | ++ |
| **046** | +++ | +++ | ++ | +++ |
| **047** | +++ | +++ | ++ | ++ |
| **048** | ++ | +++ | ++ | ++ |
| **049** | ++ | +++ | + | +++ |
| **050** | +++ | +++ | ++ | +++ |
| **051** | ++ | +++ | ++ | +++ |
| **052** | +++ | +++ | ++ | +++ |
| **053** | +++ | +++ | ++ | ++ |
| **054** | ++ | +++ | ++ | +++ |

**Table 10 shows cytotoxicity of structurally related commercial kinase inhibitors on 4T1 and MDA-MB-231 cells in the same experimental conditions as the compounds of the present invention**

| | | | | |
|---|---|---|---|---|
| Qualitative scale: +++: IC50 < 1 µM; ++: 1 µM ≤ IC50 < 5 µM; +: 5 µM ≤ IC50 < 10 µM; -: IC50 ≥ 10 µM; +++: cytotoxicity ≥ 85 %; ++: 50 % ≤ cytotoxicity < 85 %; +: 20 % ≤ cytotoxicity < 50 %; -: cytotoxicity < 20 %. | | | | |

| **Compound name** | **4T1 cells** | | **MDA-MB-231 cells** | |
|---|---|---|---|---|
| | **IC50** | **Cytotoxicity** | **IC50** | **Cytotoxicity** |
| **Lenvatinib** | ++ | + | - | - |
| **Regorafenib** | + | ++ | ++ | ++ |
| **Sorafenib** | + | + | + | +++ |
| **Tivozanib** | + | +++ | + | ++ |

**Table 11 shows cytotoxicity of compounds of the present invention on EoL-1 cells**

| | | |
|---|---|---|
| Qualitative scale: ++++: IC50 < 0.001 µM; +++: 0.001 µM ≤ IC50 < 0.01 µM; ++: 0.01 µM ≤ IC50 < 0.1 µM; +: IC50 ≥ 0.1 µM; +++: cytotoxicity ≥ 85%; ++: 50% ≤ cytotoxicity < 85%; +: 20 % ≤ cytotoxicity < 50%; -: cytotoxicity < 20%. | | |

| **Compound ID** | **EoL-1 cells** | |
|---|---|---|
| | **IC50** | **Cytotoxicity** |
| **001** | ++ | +++ |
| **002** | ++ | +++ |
| **004** | +++ | +++ |
| **005** | ++++ | +++ |
| **006** | ++ | +++ |
| **007** | ++ | +++ |
| **008** | +++ | +++ |
| **009** | +++ | +++ |
| **010** | ++ | ++ |
| **011** | ++ | ++ |
| **012** | +++ | ++ |
| **013** | +++ | +++ |
| **014** | ++ | ++ |
| **015** | ++ | ++ |
| **016** | ++ | +++ |
| **017** | +++ | ++ |
| **018** | +++ | +++ |
| **019** | +++ | +++ |
| **020** | ++++ | +++ |
| **021** | ++++ | +++ |
| **022** | ++++ | +++ |
| **023** | +++ | +++ |
| **024** | +++ | +++ |
| **025** | ++++ | +++ |
| **026** | ++++ | +++ |
| **027** | +++ | +++ |
| **028** | +++ | ++ |
| **029** | ++ | +++ |
| **030** | ++ | ++ |
| **031** | +++ | +++ |
| **032** | +++ | +++ |
| **033** | +++ | +++ |
| **034** | +++ | ++ |
| **035** | +++ | +++ |
| **036** | + | + |
| **037** | +++ | +++ |
| **038** | +++ | ++ |
| **039** | ++ | ++ |
| **040** | ++ | ++ |
| **041** | +++ | +++ |
| **042** | +++ | +++ |
| **043** | ++ | ++ |
| **044** | ++ | ++ |
| **045** | ++ | ++ |
| **046** | ++++ | +++ |
| **047** | +++ | +++ |
| **048** | +++ | ++ |
| **049** | ++ | ++ |
| **050** | +++ | +++ |
| **051** | +++ | +++ |
| **052** | +++ | +++ |
| **053** | ++ | +++ |
| **054** | +++ | +++ |

**Table 12 shows cytotoxicity of structurally related commercial kinase inhibitors on EoL-1 cells in the same experimental conditions as the compounds of the present invention**

| | | |
|---|---|---|
| Qualitative scale: ++++: IC50 < 0.001 µM; +++: 0.001 µM ≤ IC50 < 0.01 µM; ++: 0.01 µM ≤ IC50 < 0.1 µM; +: IC50 ≥ 0.1 µM; +++: cytotoxicity ≥ 85%; ++: 50% ≤ cytotoxicity < 85%; +: 20 % ≤ cytotoxicity < 50%; -: cytotoxicity < 20%. | | |

| **Compound name** | **EoL-1 cells** | |
|---|---|---|
| | **IC50** | **Cytotoxicity** |
| **Lenvatinib** | +++ | +++ |
| **Regorafenib** | +++ | +++ |
| **Sorafenib** | ++++ | +++ |

**Table 13 shows cytotoxicity of the compound #020 on human KRAS mutated cancer cell lines**

| | | |
|---|---|---|
| Qualitative scale: +++: IC50 < 1 µM; ++: 1 µM ≤ IC50 < 5 µM; +: 5 µM ≤ IC50 < 10 µM; -: IC50 ≥ 10 µM; +++: cytotoxicity ≥ 85%; ++: 50% ≤ cytotoxicity < 85%; +: 20 % ≤ cytotoxicity < 50%; -: cytotoxicity < 20%. | | |

| **Cell line** | **IC50** | **Cytotoxicity** |
|---|---|---|
| **A549** | +++ | ++ |
| **HCT116** | +++ | +++ |
| **Mia PaCa-2** | +++ | +++ |
| **SW480** | +++ | ++ |
| **HEC-1A** | +++ | ++ |
| **PSN1** | +++ | +++ |
| **RPMI 8226** | +++ | ++ |

### Example 3.2: in vitro anti-angiogenic activity

Angiogenesis is a phenomenon that includes different processes, such as endothelial cell proliferation, migration and differentiation that lead to the formation of new blood vessels. Tube formation assay is a standard *in vitro* test to assess angiogenesis: briefly, human Umbilical Vein Endothelial cells (hereafter "HUVECs") cultivated on a gelled basement matrix spontaneously proliferate, migrate and differentiate to form tube-like structures, that appear elongated and that connect to each other to form a network.

HUVECs were cultivated in Endothelial Cell Growth Medium (cat.no. C-22010 from Promocell) supplemented with 1% PenStrep (hereafter "HUVEC medium"). On day 0, HUVECs were harvested and transferred into a 384-well plate precoated with 10 µl/well of Cultrex UltiMatrix Reduced Growth factor basement membrane (cat.no. BME001-10 from BioTechne), according to the manufacturer's instructions, at a density of 5000 cells/well in HUVEC medium. Compounds to be tested were then added onto the cells and incubated for 16 to 18 hours at 37°C. After this incubation period, the cells were stained with calcein-AM (cat.no. 10462052 from Fisher Scientific) and pictures were acquired using a confocal fluorescent microscope (IXM from Molecular Devices). Tube formation was measured with MetaXpress^{®} Software.

As shown in Table 14, compounds of the present invention were efficient in inhibiting tube formation by HUVECs, sign of their anti-angiogenic activity. Their anti-angiogenic activity was overall better than the one of structurally related commercial kinase inhibitors, as shown in Table 15.

**Table 14 shows inhibition of HUVECs' tube formation by compounds of the present invention**

| | | |
|---|---|---|
| Qualitative scale: +++: IC50 < 0.1 µM; ++: 0.1 µM ≤ IC50 < 0.5 µM; +: 0.5 µM ≤ IC50 < 1 µM; -: IC50 ≥ 1 µM; +++: angiogenesis inhibition ≥ 80%; ++: 80% > angiogenesis inhibition ≥ 50%; +: 50% > angiogenesis inhibition ≥ 20%; -: angiogenesis inhibition < 20%. | | |

| **Compound ID** | **HUVECs** | |
|---|---|---|
| | **IC50** | **Angiogenesis inhibition** |
| **001** | **+++** | **+++** |
| **002** | **+++** | **+++** |
| **004** | **++** | **+++** |
| **005** | **++** | **++** |
| **006** | **++** | **+++** |
| **007** | **+++** | **+++** |
| **008** | **+++** | **+++** |
| **009** | **+++** | **+++** |
| **010** | **++** | **+++** |
| **011** | **++** | **+++** |
| **012** | **+++** | **+++** |
| **013** | **+++** | **+++** |
| **014** | **++** | **+++** |
| **015** | **+++** | **+++** |
| **016** | **+++** | **+++** |
| **017** | **+++** | **+++** |
| **018** | **+++** | **+++** |
| **019** | **+++** | **+++** |
| **020** | **+++** | **+++** |
| **021** | **++** | **+++** |
| **022** | **+++** | **++** |
| **023** | **++** | **+++** |
| **024** | **++** | **++** |
| **026** | **++** | **+++** |
| **027** | **+++** | **+++** |
| **028** | **+++** | **+++** |
| **029** | **++** | **++** |
| **030** | **+++** | **+++** |
| **031** | **++** | **+++** |
| **032** | **+++** | **+++** |
| **033** | **+++** | **+++** |
| **034** | **+++** | **+++** |
| **035** | **++** | **+++** |
| **037** | **++** | **++** |
| **038** | **++** | **+++** |
| **039** | **+++** | **+++** |
| **040** | **++** | **+++** |
| **041** | **+++** | **+++** |
| **042** | **+++** | **+++** |
| **043** | **++** | **+++** |
| **044** | **++** | **+++** |
| **045** | **++** | **+++** |
| **046** | **+++** | **+++** |
| **047** | **+++** | **+++** |
| **048** | **++** | **+++** |
| **049** | **++** | **+++** |
| **050** | **+++** | **+++** |
| **051** | **+++** | **+++** |
| **052** | **+++** | **+++** |
| **053** | **+++** | **+++** |
| **054** | **+++** | **+++** |

**Table 15 shows inhibition of HUVECs' tube formation of structurally related commercial kinase inhibitors on HUVECs in the same experimental conditions as the compounds of the present invention**

| | | |
|---|---|---|
| Qualitative scale: +++: IC50 < 0.1 µM; ++: 0.1 µM ≤ IC50 < 0.5 µM; +: 0.5 µM ≤ IC50 < 1 µM; -: IC50 ≥ 1 µM; +++: angiogenesis inhibition ≥ 80%; ++: 80% > angiogenesis inhibition ≥ 50%; +: 50% > angiogenesis inhibition ≥ 20%; -: angiogenesis inhibition < 20%. | | |

| **Compound name** | **HUVECs** | |
|---|---|---|
| | **IC50** | **Angiogenesis** inhibition |
| **Lenvatinib** | **+++** | **++** |
| **Regorafenib** | **+++** | **++** |
| **Sorafenib** | **+++** | **+** |
| **Tivozanib** | **++** | **+** |
| **Linifanib** | **+++** | **+** |

### Example 3.3: inhibition of in vitro cancer cell migration

Cell migration is involved in several pathological cancer processes such as tumor invasion, neoangiogenesis and metastasis. Scratch (or wound healing) assay is a method to study two-dimensional cell migration *in vitro.* It is based on the observation that cells growing in a monolayer migrate to reestablish cell contacts after a mechanical scratch wound.

The MDA-MB-231 cells were seeded into a 24-well plate and grown to confluent density. A scrape was made in each well using a pipette tip. Then, the cells monolayers were incubated for 2 hours with 5 µg/ml mitomycin C to stop cell proliferation. The cells were treated with the compounds to be tested for 24 hours. The cells were stained using CellMask Green actin tracking stain (cat.no. A57246 from Fisher Scientific) at time zero and at endpoint. Pictures were acquired using a fluorescence microscope (IXM from Molecular Device). The scratch area was measured with the ImageJ software. The recovery of the scratch area was calculated as a percentage of the scratch area at time zero.

As shown in Figure 1, treatment of MDA-MB-231 cells in the scratch assay by compounds #006, #016, #017, #032 and #040, resulted in inhibition of their migration.

### Example 3.4: in vitro prevention of epithelial-mesenchymal transition

The epithelial-mesenchymal transition (hereafter "EMT") is a process by which epithelial cells lose their cell polarity and cell-cell adhesion while gaining migratory and invasive properties to become mesenchymal stem cells. EMT is commonly measured by quantifying both epithelial and mesenchymal marker's expression by quantitative reverse-transcription polymerase chain reaction (hereafter "qRT-PCR"). EMT inhibition is observed when epithelial markers are stable or increased while mesenchymal markers are decreased.

4T1 cells were treated with compounds to be tested or DMSO for 24 hours. 4T1 cells were then harvested directly in NucleoZol lysis buffer (cat. no. 740404.200 from Macherey-Nagel), according to the manufacturer's instructions. Total RNA was extracted using the NucleoSpin RNA set for NucleoZol (cat. no. 740406.50 from Macherey-Nagel). The quantity and purity of RNA was determined using a Quickdrop (Molecular Devices). RT-PCR was performed on 500 ng total RNA using iScript gDNA clear cDNA synthesis Kit (cat.no. 1725035 from Biorad). The qPCR was run on a QuantStudio 5 real time thermocycler (Applied Biosystems), with PowerUp SYBR Green Master Mix (cat.no. A25742 from Fisher Scientific) and the primers listed in Table 16.

**Table 16 shows the sequences of the primers used in qPCR**

| **Primer ID** | **Gene** | **Forward primer sequence** | **Reverse primer sequence** |
|---|---|---|---|
| **1** | E-Cadherin | AACCCAAGCACGTATCAGGG (SEQ ID NO: 1) | GAGTGTTGGGGGCATCATCA (SEQ ID NO: 2) |
| **2** | EpCAM | CATTTGCTCCAAACTGGCGT (SEQ ID NO: 3) | TTGTTCTGGATCGCCCCTTC (SEQ ID NO: 4) |
| **3** | Vimentin | TCCAGAGAGAGGAAGCCGAA (SEQ ID NO: 5) | AAGGTCAAGACGTGCCAGAG (SEQ ID NO: 6) |
| **4** | Fibronectin | GGATGTTCCCTCCACAGTTCA (SEQ ID NO: 7) | TGTCCGCCTAAAGCCATGTT (SEQ ID NO: 8) |
| **5** | RPL13 | TCAAGACCAACGGACTCCTG (SEQ ID NO: 9) | GGTCCCCACTTCCCTAGTTG (SEQ ID NO: 10) |

Expression data of EMT markers have been normalized against expression data of the housekeeping gene RPL13.

As shown in Table 17, compounds of the present invention prevented 4T1 cells to undergo EMT, as shown by increased or stabilized expression of epithelial markers and decreased expression of mesenchymal markers.

**Table 17 shows prevention of EMT in 4T1 cells upon treatment with compounds of the present invention**

| | | | | |
|---|---|---|---|---|
| ↗: increased expression, ↘: decreased expression and = : unchanged expression levels. | | | | |

| **Compound ID** | **Epithelial markers** | | **Mesenchymal markers** | |
|---|---|---|---|---|
| | **E-cadherin** | **EpCAM** | **Vimentin** | **Fibronectin** |
| **006** | **↗** | **↗** | **↘** | **↘** |
| **016** | **↗** | **↗** | **↘** | **↘** |
| **017** | **↗** | **↗** | **↘** | **↘** |
| **018** | **↗** | **↗** | **↘** | **↘** |
| **020** | **↗** | = | **↘** | **↘** |
| **023** | **↗** | = | **↘** | **↘** |
| **027** | **↗** | = | **↘** | **↘** |
| **032** | **↗** | **↗** | **↘** | **=** |

### Example 3.5: inhibition of clonogenicity

Cancer stem cells are characterized by their ability to undergo virtually unlimited divisions. Colony forming assay (or clonogenic assay) is an *in vitro* assay designed to assess capability of single cells to grow into large colonies through clonal expansion.

4T1 cells were seeded at a density of 500 cells per well of six-well plates in 4T1 medium. Compounds to be tested were added 24 hours after seeding. After 6 days of treatment, the cells were fixed in 4% PFA and stained with 1% aqueous solution of crystal violet. Pictures of the entire wells were acquired, and the area of the positives colonies was evaluated by ImageJ software (Wayne Rasband; NIH).

As shown in Figure 2, treatment of 4T1 cells in the colony forming assay with compounds #006, #016, #017 and #032, resulted in inhibition of colony growth, meaning that these compounds were acting on cancer stem cells to prevent their proliferation *in vitro.*

### Example 3.6: differentiation of RD cells by the compounds of the present invention

RD cells are cancer cells of muscle origin that arose from myogenic precursors that fail to complete muscle differentiation. One strategy to stop their progression is to differentiate them into myotubes.

To allow differentiation, RD cells were seeded at a density of 3500 cells/well in a 384-well plate coated with collagen type I, in DMEM (4,5g/L glucose) with L-Gln (cat.no. 41965-039 from Gibco) supplemented with 5% of knockout serum replacement (cat.no. 10828-028 from Gibco), 1% PenStrep (Gibco), 1% MEM NEAA (Gibco) and 0.1 mM 2-mercaptoethanol (Gibco) (hereafter "K5 medium"). Compounds to be tested were then added onto the cells and incubated for 7 days at 37°C. On day 7, cells were fixed with 4% paraformaldehyde solution and immunostained for α-actinin (cat.no. EA-53 from Sigma, at 1/30000).

As shown in Figure 3, treatment of RD cells with compounds #006, #013, #018, #021 and #032, resulted in a dose-dependent differentiation into myotubes.

### Example 3.7: Inhibition of the Src family kinases and their downstream pathways

Inhibition of kinases can be observed by quantifying both their phosphorylation levels and the phosphorylation level of downstream proteins in their pathways.

RD cells were cultured *in vitro* and expanded for 4 days prior to the experiments (see example 3.1 and table 6a for details). The assay was performed using the human phospho-kinase array kit (cat.no. ARY003C from R&D Systems), according to the manufacturer's instructions. The RD cells were treated with compound #032 for 1 hour at its IC50 (previously defined) and lysed with lysis buffer supplemented with aprotinin, leupeptin and pepstatin. The total protein extract was titrated using a micro-BCA kit (cat. no. 23235 from Thermofisher Scientific). The array membranes were blocked and incubated overnight at 4 °C with 500 µg of total protein. Then, the detection antibodies were added for 2 hours followed by the HRP-conjugated Streptavidin solution for 30 minutes. The chemiluminescent signal was finally developed by incubating the membranes with the chemi-reagent mix and placed in the Fusion-FX acquisition system (Vilber Instruments). Data were analyzed using ImageJ software (Wayne Rasband; NIH).

As shown in Figure 4, treatment of rhabdomyosarcoma RD cells with compound #032 induced decreased phosphorylation of members of the Src kinase family and their downstream proteins.

In all the examples, the compounds of the invention are named with or without # before their reference number. For example, compound 006 can also be named #006.

## Claims

1. A compound of general formula (I), or a pharmaceutically acceptable salt and/or solvate thereof, for use for treating cancer and/or for reducing or preventing the appearance of metastases in a patient afflicted with a cancer, wherein formula (I) is 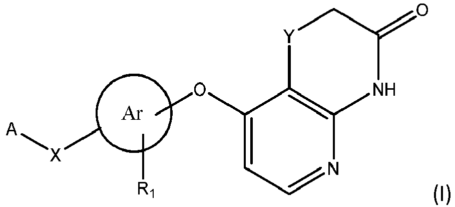 wherein,
X is
1) -X₁C(O)X₂- ; X₁ and X₂ being, independently of each other, absent, -CH₂- or -NH-; and Ar is a phenyl, naphthyl, or heteroaryl comprising 5 or 6 cyclic atoms;
or
2) -X₁C(O)-; X₁ being absent, -CH₂- or -NH-; and Ar is: 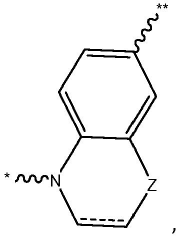 with Z being -O-, -S-, -CH₂-, -NR_{z}- or absent; Rz being H , (C₁-C₆)alkyl group, -CO-(C₁-C₆)alkyl group, -SO-(C₁-C₆)alkyl group or - SO₂-(C₁-C₆)alkyl group; and with 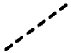 indicating an optional double bond; or
3) -NH-; and Ar is 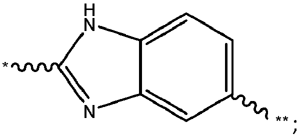 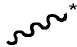 indicating the bound between Ar and X; 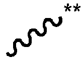 indicating the bound between Ar and the oxygen atom;
and wherein,
R₁ is selected in the group consisting of a (C₁-C₆)alkyl group, (C₁-C₆)heteroalkyl group, (C₁-C₆)haloalkyl group, or a halogen;
Y is an oxygen atom or -CH₂-; and
A is a group selected from (C₁-C₆)alkyl, (C₃-C₁₀)cycloalkyl, aryl or heterocycle, said group being optionally substituted with A₁, A₂, A₃ and/or A₄, wherein
A₁ is -CF₃; halo; -NHC(O)CH₂NR_{A}R_{B}; -NHSO₂R_{C}; -NHR_{E}; -OR_{F}; -C(O)R_{G}; -C(O)NHR_{H}; or A₁ is a group G1 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
said group G1 being optionally substituted with at least one, such as one or two, substituent S1 independently selected from a halo; -CN; -NR_{A}R_{B}; -R_{D}NR_{A}R_{B}; or a group G2 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
said group G2 being optionally substituted with at least one, such as one or two, substituent S2 independently selected from a halo, -CN, -NR_{A}R_{B}, -R_{D}NR_{A}R_{B}, (C₁-C₆)alkyl group, (C₃-C₁₀)cycloalkyl group, aryl group, heterocycle group, (C₁-C₆)alkylaryl, (C₁-C₆)alkyl-heterocycle, and (C₁-C₆)alkoxyl group;
R_{A} and R_{B} being independently an hydrogen atom or a (C₁-C₆)alkyl group;
R_{C}, R_{D}, R_{E}, R_{F}, R_{G} and R_{H} being independently a group G3 selected from a phenyl, (C₁-C₆)alkyl, heterocycle, (C₁-C₆)alkyl-phenyl, and (C₁-C₆)alkyl-heterocycle, said group G3 being optionally substituted with at least one, such as one or two, substituent S3 independently selected from -CN, halo, -CF₃ and a (C₁-C₆)alkyl ;
and
A₂, A₃ and A₄ are independently selected from -CF₃, halo, (C₁-C₆)alkyl, and (C₁-C₃)alkoxyl.

2. The compound for use according to claim 1, wherein the cancer is angiogenic and/or metastatic cancer such as bladder, brain, breast, colon, kidney, liver, lung, melanoma, ovary, muscle, pancreas, prostate, rectal, sarcoma, stomach, thyroid, or uterus cancers.

3. The compound for use according to claim 1 or 2, wherein X is -X₁C(O)X₂- ; X₁ and X₂ being, independently of each other, absent, -CH₂- or -NH-; and Ar is a phenyl, naphthyl, or heteroaryl comprising 5 or 6 cyclic atoms.

4. The compound for use according to any one of claims 1 to 3, wherein X is -X₁C(O)X₂- ; X₁ and X₂ being, independently of each other, absent, -CH₂- or -NH-; and a Ar is a phenyl, naphthyl, pyridinyl, pyridazinyl, pyrimidinyl or pyrazinyl, preferably Ar is a phenyl, naphthyl or pyridinyl, more preferably a phenyl.

5. The compound for use according to any one of claims 1 to 4, wherein X₁ and X₂ are -NH-.

6. The compound for use according to any one of claims 1 to 5, wherein A is a moiety selected from the group consisting of the moieties of Table 1.

7. The compound for use according to claim 1, wherein said compound is selected from the list consisting of the compounds of Table 2, or a pharmaceutically acceptable salt and/or solvate thereof.

8. A compound of general formula (II), or a pharmaceutically acceptable salt and/or solvate thereof: 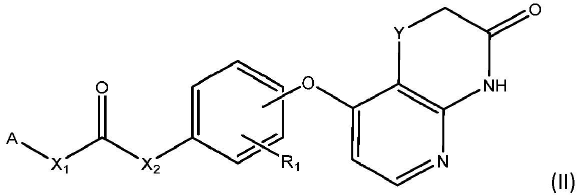 wherein,
X₁ and X₂ are, independently of each other, absent, -CH₂- or -NH-;
R₁ is selected in the group consisting of a (C₁-C₆)alkyl group, (C₁-C₆)heteroalkyl group, (C₁-C₆)haloalkyl group, or a halogen;
Y is an oxygen atom or -CH₂-; and
A is an aryl, preferably a phenyl, or a (C₃-C₁₀)cycloalkyl, preferably a cyclopropyl, and is optionally substituted with A₁, A₂, A₃ and/or A₄, wherein
A₁ is -CF₃; halo; -NHC(O)CH₂NR_{A}R_{B}; -NHSO₂R_{C}; -NHR_{E}; -OR_{F}; -C(O)R_{G}; -C(O)NHR_{H}; or A₁ is a group G1 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
said group G1 being optionally substituted with at least one, such as one or two, substituent S1 independently selected from a halo; -CN; -NR_{A}R_{B}; -R_{D}NR_{A}R_{B}; or a group G2 selected from a (C₁-C₆)alkyl, (C₃-C₁₀)cycloalkyl, aryl, heterocycle, (C₁-C₆)alkyl-aryl, (C₁-C₆)alkyl-heterocycle, and (C₁-C₆)alkoxyl,
said group G2 being optionally substituted with at least one, such as one or two, substituent S2 independently selected from a halo, -CN, -NR_{A}R_{B}, -R_{D}NR_{A}R_{B}, (C₁-C₆)alkyl group, (C₃-C₁₀)cycloalkyl group, aryl group, heterocycle group, (C₁-C₆)alkylaryl, (C₁-C₆)alkyl-heterocycle, and (C₁-C₆)alkoxyl group;
R_{A} and R_{B} being independently an hydrogen atom or a (C₁-C₆)alkyl group;
R_{C}, R_{D}, R_{E}, R_{F}, R_{G} and R_{H} being independently a group G3 selected from a phenyl, (C₁-C₆)alkyl, heterocycle, (C₁-C₆)alkyl-phenyl, and (C₁-C₆)alkyl-heterocycle, said group G3 being optionally substituted with at least one, such as one or two, substituent S3 independently selected from -CN, halo, -CF₃ and a (C₁-C₆)alkyl ;
and
A₂, A₃ and A₄ are independently selected from -CF₃, halo, (C₁-C₆)alkyl, and (C₁-C₃)alkoxyl.

9. The compound according to claim 8, wherein said compound is of the following general formula (II-1) or (II-2): 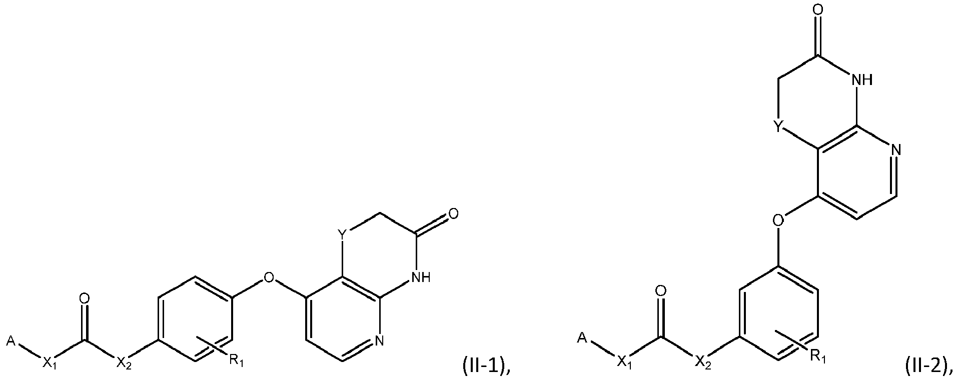 wherein A, X₁, X₂, R₁ and Y are as defined in claim 8.

10. The compound according to any one of claims 8 or 9, wherein X₁ and X₂ are -NH-.

11. The compound according to any one of claims 8 to 10, wherein A is a moiety selected from the group consisting of the moieties of Table 3.

12. The compound according to claim 8, wherein said compound is selected from the group consisting of the compounds of Table 4, or a pharmaceutically acceptable salt and/or solvate thereof.

13. A compound according to any one of claims 8 to 12, or a pharmaceutically acceptable salt and/or solvate thereof, for use as a drug.

14. A pharmaceutical composition comprising a compound as defined in any one of claims 8 to 12, or a pharmaceutically acceptable salt and/or solvate thereof, and at least one pharmaceutically acceptable excipient.

15. The compound for use according to claim 13 or pharmaceutical composition as defined in claim 14, for use for treating cancer and/or for reducing or preventing the appearance of metastases in a patient afflicted with a cancer, preferably said cancer being angiogenic and/or metastatic cancer such as bladder, brain, breast, colon, kidney, liver, lung, melanoma, ovary, muscle, pancreas, prostate, rectal, sarcoma, stomach, thyroid, or uterus cancers.
